# EUROPEAN PATENT APPLICATION

(11) **EP 4 332 111 A1**
(43) Date of publication of application: **06.03.2024**
(21) Application number: 22795867.5
(22) Date of filing: 27.04.2022
(51) Int. Cl.: C07F 9/6561

(54) **METHOD FOR PRODUCING OLIGONUCLEIC ACID COMPOUND**

(30) Priority: 28.04.2021 JP 2021076609
(71) Applicant: Nippon Shinyaku Co., Ltd., Kyoto-shi Kyoto 601-8550 (JP)
(72) Inventor: KATO, Koki, Tsukuba-shi, Ibaraki 305-0003 (JP); NOGATA, Masaki, Tsukuba-shi, Ibaraki 305-0003 (JP); TODA, Shunsuke, Kyoto-shi, Kyoto 601-8550 (JP)
(74) Representative: Vossius & Partner Patentanwälte Rechtsanwälte mbB
(86) International application number: PCT/JP2022/019139
(87) International publication number: WO 2022/230954

(57) **Abstract**

The present invention relates to a method for producing an oligonucleic acid compound, the method being characterized by (1) treating a compound represented by formula [A-1] and a compound represented by formula [B-1] with a condensation agent in the presence of a base and then (2) treating the compounds with an oxidizing agent and an organic amine to produce a compound represented by formula [C-1] (in the formulae, each symbol is as defined in the description).

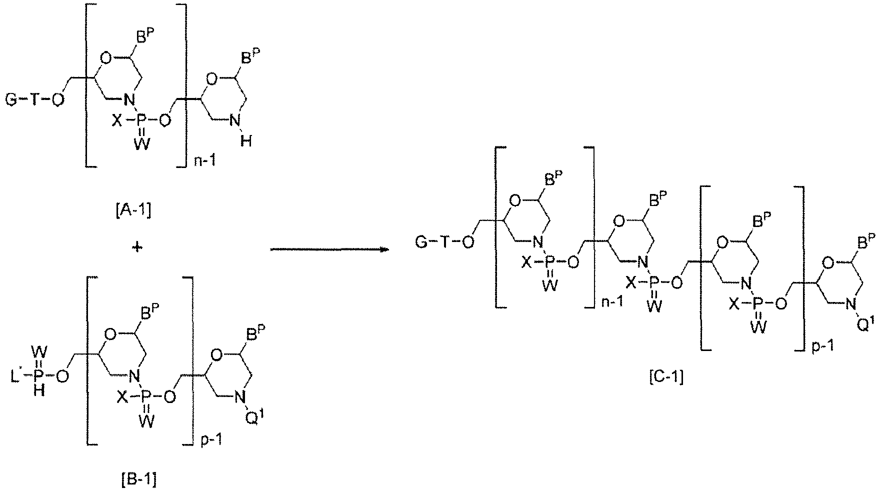

## Description

### TECHNICAL FIELD

The present invention relates to a novel method for producing an oligonucleic acid compound.

### BACKGROUND ART

A solid-phase method and a liquid-phase method are known as methods for preparing an oligonucleic acid compound. The solid-phase method is a heterogeneous reaction method in which a nucleic acid is extended while a substrate supported on a solid-phase support is brought into contact with a solution containing a reaction reagent. In the solid-phase method, a so-called batch method is used in which a reaction vessel with a filter is used and a reaction is carried out in the vessel (see, for example, Non-Patent Document 1 and Patent Document 1). In addition, a pseudo-flow synthesis method is also known in which, as in an automatic nucleic acid synthesizer (for example, DNA, RNA synthesizer), a solid-phase support is placed in a column and a solution containing a reaction reagent is passed through the column to cause a reaction.

On the other hand, the liquid-phase method is a homogeneous reaction method in which a nucleic acid is extended by causing a reaction in a solution containing both a substrate and a reaction reagent. In the liquid-phase method as well, a batch method in which a reaction is carried out in a vessel is used (see, for example, Patent Document 2 and Patent Document 3).

In any of the cases of the solid-phase method, the liquid-phase method, the batch method, and the pseudo-flow synthesis method, in a chemical synthesis method for an oligonucleic acid compound, a nucleic acid is extended by repeating many times a "deprotection" reaction for removing a protecting group for an oxygen atom or amino group on a nucleic acid compound, and a "condensation" reaction for forming a bond between a phosphorus atom and an oxygen atom or nitrogen atom deprotected to be enabled to react.

Among others, controlling the reaction efficiency or the reaction rate in the "condensation" reaction for forming a bond between a phosphorus atom and an oxygen atom or nitrogen atom is very important in the preparation of an oligonucleic acid compound, and the conditions of this condensation reaction are factors that have a great impact on the preparation period of the oligonucleic acid compound.

Since the solid-phase method is a heterogeneous reaction between a solid-phase support and a solution, it is known that the reactivity of the condensation reaction decreases due to steric hindrance caused by the solid-phase support. Polystyrene resin is generally used as the solid-phase support. During the reaction, the polystyrene resin swells due to the reaction solvent used, and its volume becomes larger than that in a dry state. The degree of swelling depends on the reaction solvent.

Therefore, the reaction efficiency and the reaction rate of the condensation reaction in the solid-phase method depend on the reaction solvent used. In particular, with a polar solvent such as acetonitrile, which is generally used for the synthesis of oligonucleic acid compounds, the degree of swelling of the polystyrene resin is not so high, so that the use of a polar solvent in the solid-phase method is not preferable from the viewpoint of improving the reaction efficiency and the reaction rate of the condensation reaction.

On the other hand, as a homogeneous reaction method, a liquid-phase method and a synthetic method using a hydrophobic group-binding nucleoside, a pseudo-solid phase-protected nucleoside, or the like are known.

The liquid-phase method is a homogeneous reaction method in which a reaction is carried out in a solution containing both a substrate and a reaction reagent, and the reaction efficiency is higher than that of the solid-phase method, and the reaction rate is faster than that of the solid-phase method. However, column purification is required to remove the reaction reagent and a reaction solvent that are to be impurities.

Similar to the liquid-phase method, in the synthetic method using a hydrophobic group-binding nucleoside, a pseudo-solid phase-protected nucleoside, or the like, a reaction can be carried out in a homogeneous system, and thus the reaction efficiency is higher than that of the solid-phase method, and the reaction rate is faster than that of the solid-phase method. Furthermore, after the reaction, unnecessary reaction reagent and reaction solvent can be removed by precipitating the target compound from the reaction mixture (see, for example, Patent Document 4).

In these homogeneous reaction methods, a non-polar solvent such as chloroform is used in a condensation reaction. However, for example, as reported in the synthesis of a morpholino nucleic acid (see, for example, Patent Document 5), the condensation reaction in the non-polar solvent requires a very long time, so that the use of a non-polar solvent in the homogeneous reaction is not preferable from the viewpoint of improving the reaction efficiency and the reaction rate of the condensation reaction.

### Prior Art Document

### [Patent Document]

[Patent Document 1] WO1991/09033A1
[Patent Document 2] WO2014/077292A1
[Patent Document 3] WO2013/122236A1
[Patent Document 4] Japanese Patent No. 5548852
[Patent Document 5] WO2016/060135A1

### [Non-Patent Document]

[Non-Patent Document 1] Acc. Chem. Res., Vol. 24, 278-284, 1991

### SUMMARY OF THE INVENTION

### PROBLEMS TO BE SOLVED BY THE INVENTION

An object of the present invention is to provide a novel preparation method that can shorten the time for the preparation of an oligonucleic acid compound.

### SOLUTION TO THE PROBLEMS

The inventors found that the condensation reaction of oligonucleic acid compounds proceeds efficiently by forming H-phosphonates and have achieved the present invention.

Thus, the present invention relates to the followings.
<1> A method for producing a compound of the formula [C-1] comprising that a compound of the formula [A-1]: 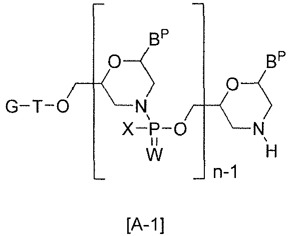 wherein
   B^{P} is each the same or different and is each a nucleobase optionally protected;
   n is an integer from 1 to 50, preferably from 1 to 30, more preferably from 1 to 25, and most preferably from 1 to 15;
   W is each the same or different and is each an oxygen atom or a sulfur atom;
   X is each the same or different and is each a hydroxyl group substituted with a group removable under neutral condition, 1,1,3,3-tetra(C₁₋₆ alkyl) guanidyl, C₁₋₆ alkoxy, di (C₁₋₆ alkyl) amino, mono (amino substituted with a group removable under basic condition-C₁₋₆ alkyl) amino, di (amino substituted with a group removable under basic condition-C₁₋₆ alkyl)amino, or a substituent group of the general formula [2] : 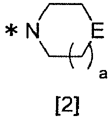
   wherein
      * is a binding position with P;
      a is an integer from 0 to 2;
      E is CH₂, CH-A¹ or N-A²;
      A¹ is C₁₋₆ alkyl, mono(C₁₋₆ alkyl) amino-C₁₋₆ alkyl substituted with a group removable under basic condition, di (C₁₋₆ alkyl) amino-C₁₋₆ alkyl, tri(C₁₋₆ alkyl) ammonio-C₁₋₆ alkyl, amino substituted with a group removable under basic condition, mono(C₁₋₆ alkyl)amino substituted with a group removable under basic condition, di(C₁₋₆ alkyl) amino, tri(C₁₋₆ alkyl)ammonio, amino substituted with amidino substituted with a group removable under basic condition, or a substituent group of the general formula [3] : 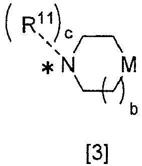
      wherein
         * is a binding position with E;
         b is an integer from 0 to 2;
         c is 0 or 1;
         R¹¹ is C₁₋₆ alkyl; and
         M is CH₂, oxygen atom, sulfur atom or N-(a group removable under basic condition); and
      A² is C₁₋₆ alkyl, mono(C₁₋₆ alkyl)amino-C₁₋₆ alkyl substituted with a group removable under basic condition, di(C₁₋₆ alkyl)amino-C₁₋₆ alkyl, tri(C₁₋₆ alkyl)ammonio-C₁₋₆ alkyl, a group removable under basic condition, aryl or heteroaryl;
   G is
      (1) a silicon substituent group,
      (2) C₁₋₁₈ alkyl-carbonyl optionally substituted, C₁₋₁₈ alkoxy-carbonyl optionally substituted, a long-chain alkyl-carbonyl, or a long-chain alkoxy-carbonyl,
      (3) benzoyl substituted with one to five long-chain alkyloxy and/or long-chain alkenyloxy, or
      (4) a substituent group represented by the general formula [7]: 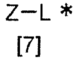
   wherein
      * is a binding position with T;
      Z is
         (1) (a soluble polymer soluble in an organic solvent)-oxy,
         (2) (a soluble polymer soluble in an organic solvent)-amino,
         (3) a long-chain alkyloxy, benzoyl substituted with one to five long-chain alkyloxy and/or long-chain alkenyloxy, or benzyl substituted with one to five long-chain alkyloxy and/or long-chain alkenyloxy,
         (4) a solid-phase support, or
         (5) a substituent group represented by the following general formula [8A] to [8N]: 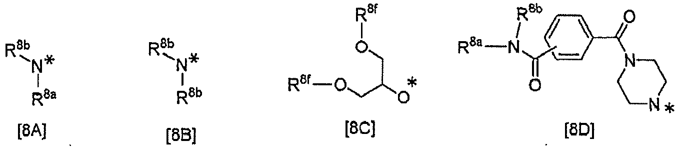 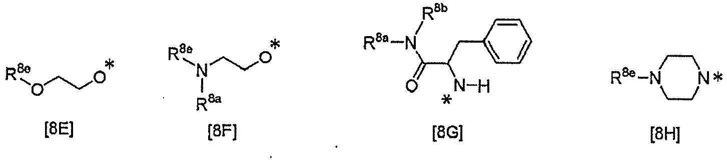 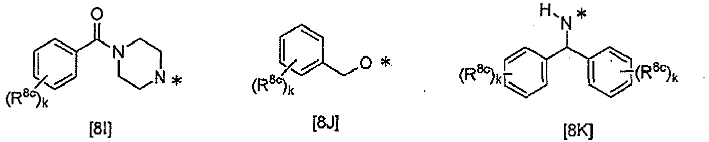 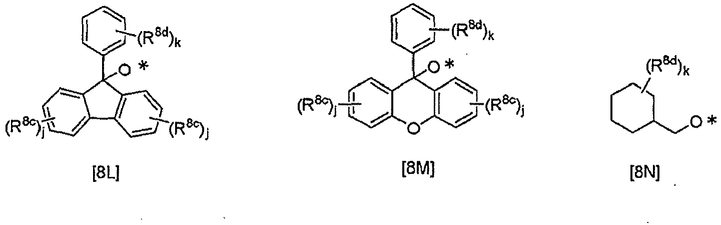
      wherein
         * is a binding position with L;
         j is an integer from 0 to 4;
         k is an integer from 0 to 5;
         R^{8a} is a hydrogen atom or C₁₋₆ alkyl;
         R^{8b} is each the same or different and is each a long-chain alkyl;
         R^{8c} is each the same or different and is each a substituent group of the general formulae selected from [9A] to [9E]: 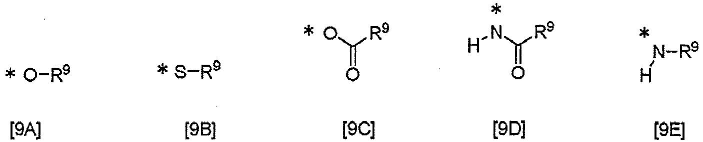
         wherein
            * is a binding position; and
            R⁹ is a long-chain alkyl and/or a long-chain alkenyl;
         R^{8d} is each the same or different and is each a hydrogen atom, a halogen, a long-chain alkyl optionally substituted with 1 to 13 halogen atoms or a long-chain alkyloxy optionally substituted with 1 to 13 halogen atoms;
         R^{8e} is
            (1) a long-chain alkyl,
            (2) a long-chain alkyl-carbonyl, or
            (3) benzoyl substituted with one to five long-chain alkyloxy and/or long-chain alkenyloxy; and
         R^{8f} is
            (1) a long-chain alkyl,
            (2) a long-chain alkyl-carbonyl, or
            (3) a long-chain alkenyl-carbonyl; and
      L is a group of the general formula [10]: 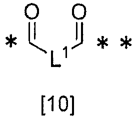
      wherein
         * is a binding position with Z;
         ** is a binding position with T; and
         L¹ is C₂₋₁₀ alkylene optionally substituted or C₆₋₁₀ arylene optionally substituted; or
   a group of the general formula [10-1]: 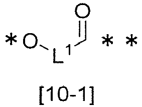
   wherein
      * is a binding position with Z;
      ** is a binding position with T; and
      L¹ is C₁₋₁₀ alkylene optionally substituted or C₆₋₁₀ arylene optionally substituted; and
   T is a single bond or a substituent group of the general formula [11], provided that T is a single bond when G is a silicon substituent group: 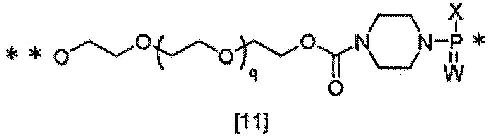
   wherein
      X and W are as defined above;
      * is a binding position with **O, *O or *N of the above formulae [4a] to [4d];
      ** is a binding position with G; and
      q is an integer from 0 to 10;

   and a compound of the formula [B-1]: 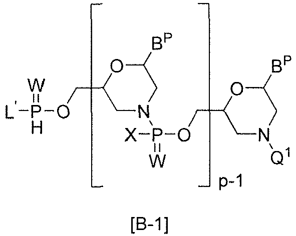 wherein
      B^{P}, W and X are as defined above; and
      p is an integer of 1 to 50, preferably from 1 to 30, more preferably from 1 to 25, and most preferably from 1 to 15;
      L' is OH, -O⁻N⁺H(aliphatic amine), -O⁻N⁺H(cyclic amine), or -O⁻N⁺H(aromatic amine), preferably -O⁻N⁺H(C₁₋₆ alkyl)₃ such as -O⁻N⁺H(CH₂CH₃)₃ or -O⁻N⁺H(cyclic amine) such as -O⁻ (HDBU)⁺; and
      Q¹ is a group removable under acidic condition;
   are subjected to
      (1) a treatment with at least one condensation agent selected from the group consisting of Phosphorus Reagent 1, Phosphorus Reagent 2 and onium reagent in the presence of a base, and then
      (2) a treatment with an oxidizing agent and an organic amine to obtain the compound of the formula [C-1]: 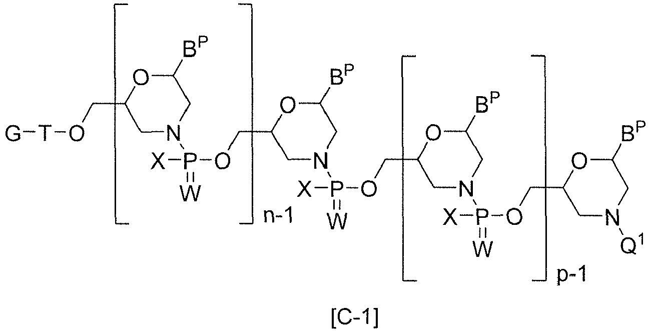 wherein
         B^{P}, Q¹, W, X, G, T, n and p are as defined above.
<2> The method according to <1> comprising further that the compound of the formula [C-1]: 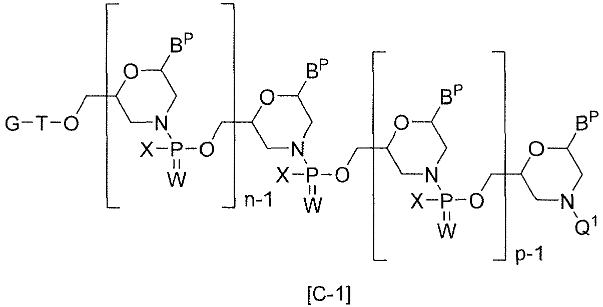 wherein
   B^{P}, Q¹, W, X, G, T, n and p are as defined above,
      is treated with an acid or with an acid and a scavenger to obtain a compound of the formula [C-0-1]: 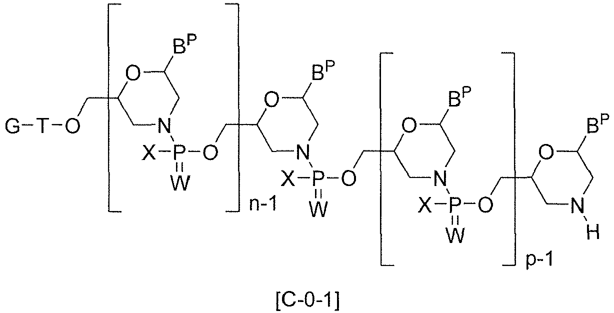 wherein
   B^{P}, Q¹, W, X, G, T, n and p are as defined above; and then,
      said compound of the formula [C-0-1] and a compound of the formula [B-0-1]: 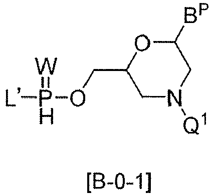
      wherein
      B^{P}, Q¹, W and L' are as defined above,
         are subjected to
         (1) a treatment with at least one condensation agent selected from the group consisting of Phosphorus Reagent 1, Phosphorus Reagent 2 and onium reagent in the presence of a base, and then
         (2) a treatment with an oxidizing agent and an organic amine to obtain a compound of the formula [C-1-1]: 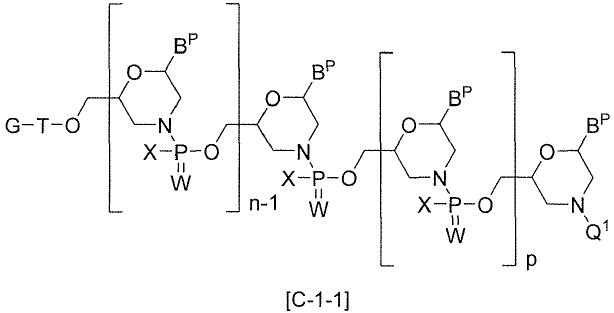
      wherein
         B^{P}, Q¹, W, X, G, T, n and p are as defined above.
<3> The method according to <1> comprising further that the compound of the formula [C-1]: 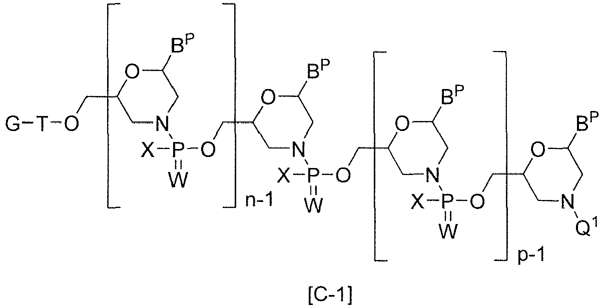 wherein
   B^{P}, Q¹, W, X, G, T, n and p are as defined above, is treated under a condition for removing the hydroxyl-protecting group to obtain a compound of the formula [C-0-2] : 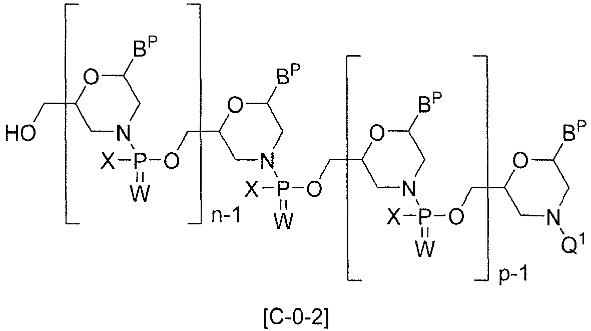 wherein
   B^{P}, Q¹, W, X, n and p are as defined above; and then,
      said compound of the formula [C-0-2] is subjected to
      (1) a treatment with a compound of the formula [P-1]: 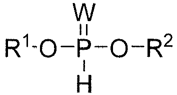 wherein
         W is an oxygen atom or a sulfur atom, and
         R¹ and R² are each the same or different and are each selected from the group consisting of H, C₁₋₆ alkyl optionally substituted, phenyl optionally substituted and PH(=O)-OH;
            and a base and then,
      (2) a treatment with a hydrolyzing solution to obtain a compound of the formula [C-0-2-1]: 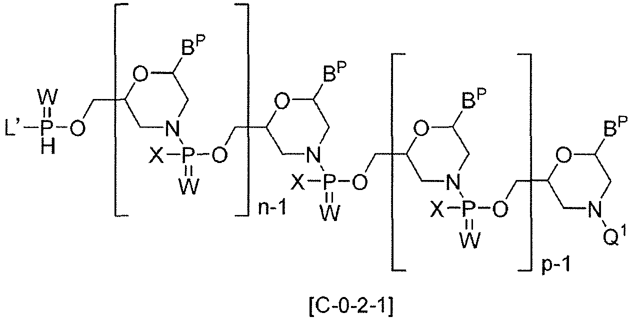 wherein
         B^{P}, Q¹, W, X, L', n and p are as defined above.
<4> The method according to <3> comprising further that the compound of the formula [C-0-2-1]: 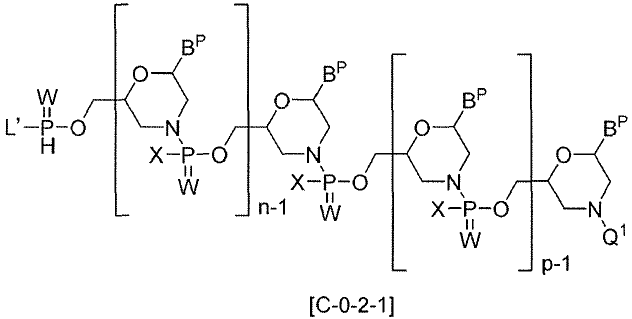 wherein
   B^{P}, Q¹, W, X, L', n and p are as defined above, and a compound of the formula [A-0-1]: 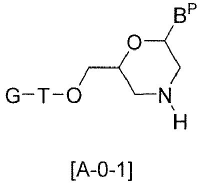 wherein
   B^{P}, G and T are as defined above,
      are subjected to
      (1) a treatment with at least one condensation agent selected from the group consisting of Phosphorus Reagent 1, Phosphorus Reagent 2 and onium reagent in the presence of a base, and then
      (2) a treatment with an oxidizing agent and an organic amine
         to obtain a compound of the formula [C-1-2]: 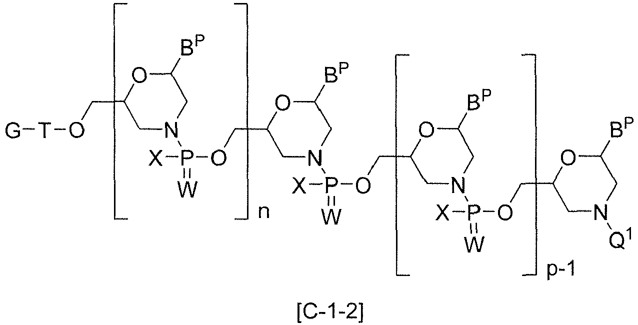 wherein
         B^{P}, Q¹, W, X, G, T, n and p are as defined above.
<5> The method according to <1> comprising further that a compound of the formula [B-1-0]: 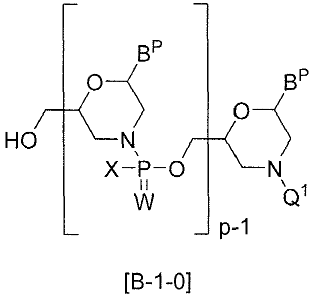 wherein
   B^{P}, Q¹, W, X and p are as defined above is subjected to
   (1) a treatment with a compound of the formula [P-1]: 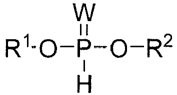 wherein
      W, R¹ and R² are as defined above;
      and a base, and then,
   (2) a treatment with a hydrolyzing solution to obtain a compound of the formula [B-1]: 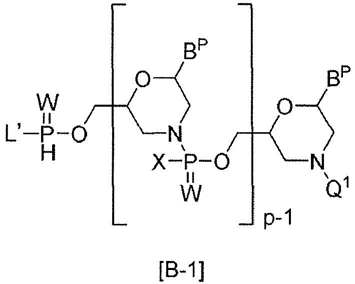 wherein
      B^{P}, Q¹, W, X, L' and p are as defined above.
<6> The method according to <1> comprising further that a compound of the formula [A-0]: 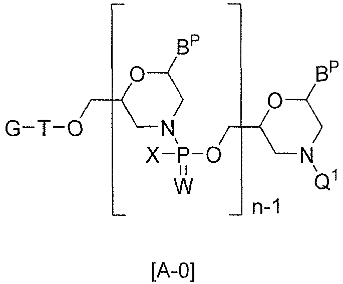 wherein
   B^{P}, Q¹, W, X, G, T and n are as defined above
      is treated with an acid or with an acid and a scavenger to obtain a compound of the formula [A-1]: 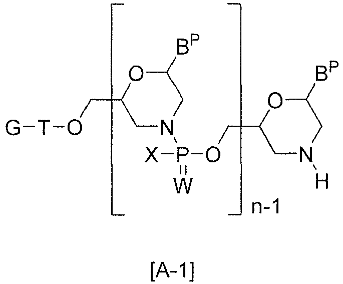 wherein
   B^{P}, W, X, G, T and n are as defined above.
<7> The method according to any one of <1> to <6>, wherein
   G is selected from the group consisting of: 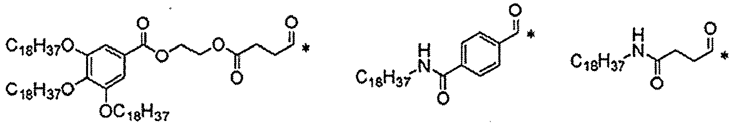 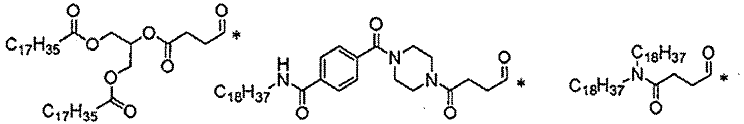 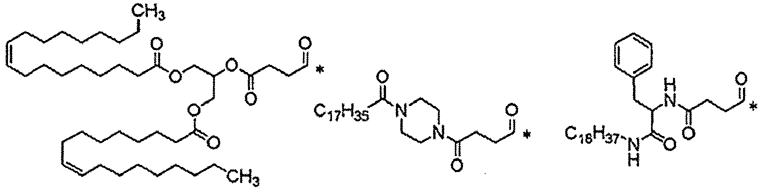 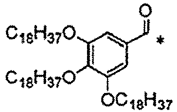
   wherein
      * is a binding position with T, and T is a single bond.
<8> The compound of the formula [B-1] as defined in <1>, wherein
   B^{P} is each the same or different and is each a nucleobase optionally protected;
   Q¹ is trityl or dimethoxytrityl;
   L' is -O⁻N⁺H(CH₂CH₃)₃ or -O⁻(HDBU)⁺;
   W is O; and
   p is an integer from 2 to 20, preferably from 2 to 15, more preferably from 2 to 10.
<9> The compound of the formula [B-1] according to <8> selected from the group consisting of:
   ((2S,6R)-6-(4-benzamido-2-oxopyrimidin-1(2H)-yl)-4-((((2S,6R)-6-(6-benzamido-9H-purin-9-yl)-4-tritylmorpholin-2-yl)methoxy) (dimethylamino)phosphoryl)morpholin-2-yl)methylphosphonate (i.e., H-phosphonate-PMO[C^{Bz}-A^{Bz}]-ON) ;
   Triethylammonium ((2S,6R)-6-(4-benzamido-2-oxopyrimidin-1(2H)-yl)-4-((((2S,6R)-6-(6-benzamido-9H-purin-9-yl)-4-tritylmorpholin-2-yl)methoxy)(dimethylamino)phosphoryl)morpholin-2-yl)methylphosphonate (i.e., H-phosphonate-PMO[C^{Bz}-A^{Bz}]-ON (triethylamine salt));
   ((2S,6R)-6-(4-benzamido-2-oxopyrimidin-1(2H)-yl)-4-((((2S,6R)-4-((((2S,6R)-6-(4-benzamido-2-oxopyrimidin-1(2H)-yl)-4-((dimethylamino)(((2S,6R)-6-(5-methyl-2,4-dioxo-3,4-dihydropyrimidin-1(2H)-yl)-4-trimethylmorpholin-2-yl)methoxy)phosphoryl)morpholin-2-yl) (dimethylamino)phosphoryl)-6-(5-methyl-2,4-dioxo-3,4-dihydropyrimidin-1(2H)-yl)morpholin-2-yl)methoxy)phosphoryl)-6-(6-benzamido-9H-purin-9-yl)morpholin-2-yl)methoxy)(dimethylamino)phosphoryl)morpholin-2-yl)methylphosphonate (i.e., H-phosphonate-PMO [C^{Bz}-A^{Bz}-T-C^{Bz}-T]-ON) ;
   Triethylammonium ((2S,6R)-6-(4-benzamido-2-oxopyrimidin-1(2H)-yl)-4-((((2S,6R)-4-((((2S,6R)-6-(4-benzamido-2-oxopyrimidin-1(2H)-yl)-4-((dimethylamino)(((2S,6R)-6-(5-methyl-2,4-dioxo-3,4-dihydropyrimidin-1(2H)-yl)-4-tritylmorpholin-2-yl)methoxy)phosphoryl)morpholin-2-yl) (dimethylamino)phosphoryl)-6-(5-methyl-2,4-dioxo-3,4-dihydropyrimidin-1(2H)-yl)morpholin-2-yl)methoxy)phosphoryl)-6-(6-benzamido-9H-purin-9-yl)morpholin-2-yl)methoxy)(dimethylamino)phosphoryl)morpholin-2-yl)methyl phosphonate (i.e., H-phosphonate-PMO[C^{Bz}-A^{Bz}-T-C^{Bz}-T]-ON (triethylamine salt));
   ((2S,6R)-6-(4-benzamido-2-oxopyrimidin-1(2H)-yl)-4-((((2S,6R)-4-((((2S,6R)-6-(6-benzamido-9H-purin-9-yl)-4-((((2S,6R)-6-(6-benzamido-9H-purin-9-yl)-4-((((2S,6R)-6-((2-cyanoethoxy)-2-(2-phenoxyacetamide)-9H-purin-9-yl)-4-tritylmorpholin-2-yl)methoxy)(dimethylamino)phosphoryl)morpholin-2-yl)methoxy)(dimethylamino)phosphoryl)morpholin-2-yl)methoxy)(dimethylamino)phosphoryl)-6-((2-cyanoethoxy)-2-(2-phenoxyacetamide)-9H-purin-9-yl)morpholin-2-yl)methoxy)(dimethylamino)phosphoryl)morpholin-2-yl)methylphosphonate (i.e., H-phosphonate-PMO[C^{Bz}-G^{CE,Pac}-A^{Bz}-A^{Bz}-G^{CE,Pac}]-ON);
   Triethylammonium ((2S,6R)-6-(4-benzamido-2-oxopyrimidin-1(2H)-yl)-4-((((2S,6R)-4-((((2S,6R)-6-(6-benzamido-9H-purin-9-yl)-4-((((2S,6R)-6-(6-benzamido-9H-purin-9-yl)-4-((((2S,6R)-6-((2-cyanoethoxy)-2-(2-phenoxyacetamide)-9H-purin-9-yl)-4-tritylmorpholin-2-yl)methoxy)(dimethylamino)phosphoryl)morpholin-2-yl)methoxy)(dimethylamino)phosphoryl)morpholin-2-yl)methoxy)(dimethylamino)phosphoryl)-6-((2-cyanoethoxy-2-(2-phenoxyacetamide)-9H-purin-9-yl)morpholin-2-yl)methoxy)(dimethylamino)phosphoryl)morpholin-2-yl)methylphosphonate (i.e. , H-phosphonate-PMO[C^{Bz}-G^{CE,Pac}-A^{Bz}-A^{Bz}-G^{CE,Pac}]-ON (triethylamine salt)) ;
   ((2S,6R)-4-((((2S,6R)-4-((((2S,6R)-4-((((2S,6R)-6-(4-benzamido-2-oxopyrimidin-1(2H)-yl)-4-tritylmorpholin-2-yl)methoxy)(dimethylamino)phosphoryl)-6-(5-methyl-2,4-dioxo-3,4-dihydropyrimidin-1(2H)-yl)morpholin-2-yl)methoxy)(dimethylamino)phosphoryl)-6-(5-methyl-2,4-dioxo-3,4-dihydropyrimidin-1(2H)-yl)morpholin-2-yl)methoxy)(dimethylamino)phosphoryl)-6-((2-cyanoethoxy)-2-(2-phenoxyacetamide)-9H-purin-9-yl)morpholin-2-yl)methoxy)(dimethylamino)phosphoryl)-6-((2-cyanoethoxy)-2-(2-phenoxyacetamide)-9H-purin-9-yl)morpholin-2-yl)methylphosphonate (i.e., H-phosphonate-PMO[G^{CE,Pac}-G^{CE,Pac}-T-T-C^{Bz}]-ON) ;
   Triethylammonium ((2S,6R)-4-((((2S,6R)-4-((((2S,6R)-4-((((2S,6R)-6-(4-benzamido-2-oxopyrimidin-1(2H)-yl)-4-tritylmorpholin-2-yl)methoxy) (dimethylamino)phosphoryl)-6-(5-methyl-2,4-dioxo-3,4-dihydropyrimidin-1(2H)-yl)morpholin-2-yl)methoxy)(dimethylamino)phosphoryl)-6-(5-methyl-2,4-dioxo-3,4-dihydropyrimidin-1(2H) -yl)morpholin-2-yl)methoxy)(dimethylamino)phosphoryl)-6-((2-cyanoethoxy)-2-(2-phenoxyacetamide)-9H-purin-9-yl)morpholin-2-yl)methoxy)(dimethylamino)phosphoryl)-6-((2-cyanoethoxy)-2-(2-phenoxyacetamide)-9H-purin-9-yl)morpholin-2-yl)methylphosphonate (i.e., H-phosphonate-PMO[G^{CE,Pac}-G^{CE,Pac}-T-T-C^{Bz}]-ON (triethylamine salt)) ;
   ((2S,6R)-6-(4-benzamido-2-oxopyrimidin-1(2H)-yl)-4-((((2S,6R)-6-(4-benzamido-2-oxopyrimidin-1(2H)-yl)-4-tritylmorpholin-2-yl)methoxy)(dimethylamino)phosphoryl)morpholin-2-yl)methylphosphonate (i.e., H-phosphonate-PMO[C^{Bz}-C^{Bz}]-ON) ;
   Triethylammonium ((2S,6R)-6-(4-benzamido-2-oxopyrimidin-1(2H)-yl)-4-((((2S,6R)-6-(4-benzamido-2-oxopyrimidin-1(2H)-yl)-4-tritylmorpholin-2-yl)methoxy)(dimethylamino)phosphoryl)morpholin-2-yl)methylphosphonate (i.e., H-phosphonate-PMO[C^{Bz}-C^{Bz}]-ON (triethylamine salt));
   ((2S,6R)-4-((dimethylamino)(((2S,6R)-6-(5-methyl-2,4-dioxo-3,4-dihydropyrimidin-1(2H)-yl)-4-tritylmorpholin-2-yl)methoxy)phosphoryl)-6-(5-methyl-2,4-dioxo-3,4-dihydropyrimidin-1(2H)-yl)morpholin-2-yl)methylphosphonate (i.e., H-phosphonate-PMO[T-T]-ON);
   Triethylammonium (2S,6R)-4-((dimethylamino)(((2S,6R)-6-(5-methyl-2,4-dioxo-3,4-dihydropyrimidin-1(2H)-yl)-4-tritylmorpholin-2-yl)methoxy)phosphoryl)-6-(5-methyl-2,4-dioxo-3,4-dihydropyrimidin-1(2H)-yl)morpholin-2-yl)methylphosphonate (i.e., H-phosphonate-PMO[T-T]-ON (triethylamine salt));
   ((2S,6R)-4-((dimethylamino)(((2S,6R)-4-((dimethylamino)(((2S,6R)-6-(5-methyl-2,4-dioxo-3,4-dihydropyrimidin-1(2H)-yl)-4-tritylmorpholin-2-yl)methoxy)phosphoryl)-6-(5-methyl-2,4-dioxo-3,4-dihydropyrimidin-1(2H)-yl)methoxy)phosphoryl)-6-(5-methyl-2,4-dioxo-3,4-dihydropyrimidin-1(2H)-yl)morpholin-2-yl)methylphosphonate (i.e., H-phosphonate-PMO[T-T-T]-ON);
   Triethylammonium ((2S,6R)-4-((dimethylamino)(((2S,6R)-4-((dimethylamino)(((2S,6R)-6-(5-methyl-2,4-dioxo-3,4-dihydropyrimidin-1(2H)-yl)-4-tritylmorpholin-2-yl)methoxy)phosphoryl)-6-(5-methyl-2,4-dioxo-3,4-dihydropyrimidin-1(2H)-yl)methoxy)phosphoryl)-6-(5-methyl-2,4-dioxo-3,4-dihydropyrimidin-1(2H)-yl)morpholin-2-yl)methylphosphonate (i.e., H-phosphonate-PMO[T-T-T]-ON (triethylamine salt));
   ((2S,6R)-4-((dimethylamino)(((2S,6R)-4-((dimethylamino)(((2S,6R)-4-((dimethylamino) ((((2S,6R)-6-(5-methyl-2,4-dioxo-3,4-dihydropyrimidin-1(2H)-yl)-4-tritylmorpholin-2-yl)methoxy)phosphoryl-6-(5-methyl-2,4-dioxo-3,4-dihydropyrimidin-1(2H)-yl)methoxy)phosphoryl-6-(5-methyl-2,4-dioxo-3,4-dihydropyrimidin-1(2H)-yl)methoxy)phosphoryl)-6-(5-methyl-2,4-dioxo-3,4-dihydropyrimidin-1(2H)-yl)morpholin-2-yl)methylphosphonate (i.e., H-phosphonate-PMO[T-T-T-T]-ON);
   Triethylammonium ((2S,6R)-4-((dimethylamino)(((2S,6R)-4-((dimethylamino)(((2S,6R)-4-((dimethylamino)((((2S,6R)-6-(5-methyl-2,4-dioxo-3,4-dihydropyrimidin-1(2H)-yl)-4-tritylmorpholin-2-yl)methoxy)phosphoryl)-6-(5-methyl-2,4-dioxo-3,4-dihydropyrimidin-1(2H)-yl)methoxy)phosphoryl)-6-(5-methyl-2,4-dioxo-3,4-dihydropyrimidin-1(2H)-yl)methoxy)phosphoryl)-6-(5-methyl-2,4-dioxo-3,4-dihydropyrimidin-1(2H)-yl)morpholin-2-yl)methylphosphonate (i.e., H-phosphonate-PMO[T-T-T-T]-ON (triethylamine salt));
   1,8-diazabicyclo(5.4.0)-7-undecenium ((2S,6R)-6-(4-benzamido-2-oxopyrimidin-1(2H)-yl)-4-((((2S,6R)-4-((((2S,6R)-6-(4-benzamido-2-oxopyrimidin-1(2H)-yl)-4-((dimethylamino)(((2S,6R)-6-(5-methyl-2,4-dioxo-3,4-dihydropyrimidin-1(2H)-yl)-4-tritylmorpholin-2-yl)methoxy)phosphoryl)morpholin-2-yl) (dimethylamino)phosphoryl)-6-(5-methyl-2,4-dioxo-3,4-dihydropyrimidin-1(2H)-yl)morpholin-2-yl)methoxy)phosphoryl)-6-(6-benzamido-9H-purin-9-yl)morpholin-2-yl)methoxy)(dimethylamino)phosphoryl)morpholin-2-yl)methylphosphonate (i.e., H-phosphonate-PMO[C^{Bz}-A^{Bz}-T-C^{Bz}-T]-ON (DBU salt)); and
   1,8-diazabicyclo(5.4.0)-7-undecenium ((2S,6R)-4-(((2S,6R)-4-(((2S,6R)-6-(6-benzamidopurin-9-yl)-4-(((2S,6R)-6-(6-benzamidopurin-9-yl)-4-(((2S,6R)-4-(((2S,6R)-6-(6-(2-cyanoethoxy)-2-((2-phenoxyacetyl)amino)purin-9-yl)-4-(((2S,6R)-4-(((2S,6R)-6-(6-(2-cyanoethoxy)-2-((2-phenoxyacetyl)amino)purin-9-yl-4-(dimethylamino-(((2S,6R)-4-(dimethylamino-(((2S,6R)-6-(5-methyl-2,4-dioxo-pyrimidin-1-yl)-4-trityl-morpholin-2-yl)methoxy)phosphoryl)-6-(5-methyl-2,4-dioxo-pyrimidin-1-yl)morpholin-2-yl)methoxy)phosphoryl)morpholin-2-yl)methoxy-(dimethylamino)phosphoryl-6-(5-methyl-2,4-dioxo-pyrimidin-1-yl)morpholin-2-yl)methoxy-(dimethylamino)phosphoryl)morpholin-2-yl)methoxy-(dimethylamino)phosphoryl)-6-(6-(3-cyanopropoxy-2-((2-phenoxyacetyl)amino)purin-9-yl)morpholin-2-yl)methoxy-(dimethylamino)phosphoryl)morpholin-2-yl)methoxy-(dimethylamino)phosphoryl)morpholin-2-yl)methoxy-(dimethylamino)phosphoryl)-6-(6-(2-cyanoethoxy)-2-((2-phenoxyacetyl)amino)purin-9-yl)morpholin-2-yl)methoxy-(dimethylamino)phosphoryl)-6-(5-methyl-2,4-dioxo-pyrimidin-1-yl)morpholin-2-yl)methylphosphinate (i.e., H-phosphonate-PMO[T-G^{CE, Pac}-A^{Bz}-A^{Bz}-G^{CE, Pac}-G^{CE, Pac}-T-G^{CE, Pac}-T-T]-ON (DBU salt)).
<10> The compound of the formula [B-0-1] as defined in <2> wherein
   B^{P} is a nucleobase optionally protected;
   Q¹ is trityl or dimethoxytrityl;
   L' is O⁻(HDBU)⁺; and
   W is O.
<11> The compound of the formula [B-0-1] according to <10> selected from the group consisting of:
   1,8-diazabicyclo(5.4.0)-7-undecenium ((2S,6R)-6-(6-benzamido-9H-purin-9-yl)-4-tritylmorpholin-2-yl)methylphosphonate;
   1,8-diazabicyclo(5.4.0)-7-undecenium ((2S,6R)-6-(4-benzamido-2-oxopyrimidin-1(2H)-yl)-4-tritylmorpholin-2-yl)methylphosphonate;
   1,8-diazabicyclo(5.4.0)-7-undecenium ((2S,6R)-6-(5-methyl-2,4-dioxo-3,4-dihydropyrimidin-1(2H)-yl)-4-tritylmorpholin-2-yl)methylphosphonate;
   1,8-diazabicyclo(5.4.0)-7-undecenium ((2S,6R)-6-((2-cyanoethoxy)-2-(2-phenoxyacetamido)-9H-purin-9-yl)-4-tritylmorpholin-2-yl)methylphosphonate; and
   1,8-diazabicyclo(5.4.0)-7-undecenium ((2S,6R)-6-(2-(2-phenylacetamide)-6-((4-(pivaloyloxy)benzyl)oxy)-9H-purin-9-yl)-4-tritylmorpholin-2-yl)methylphosphonate.

### EFFECTS OF THE INVENTION

An oligonucleic acid compound is a compound having a structure in which two or more nucleoside units are connected via phosphate bonds. In order to prepare an oligonucleic acid compound, it is necessary to carry out a condensation reaction many times to form a phosphate bond between adjacent nucleoside units.

According to the present invention, since a phosphate bond can be efficiently formed, it can be expected that the preparation time of the oligonucleic acid compound is shortened as a result.

### EMBODIMENTS FOR CARRYING OUT THE INVENTION

The invention is described in detail below.

### Description of terms

Examples of the "nucleobase" include adenine, guanine, hypoxanthine, cytosine, thymine, uracil, and modified bases thereof. Examples of such modified bases include, but are not limited to, pseudouracil, 3-methyluracil, dihydrouracil, 5-alkylcytosines (for example, 5-methylcytosine), 5-alkyluracils (for example, 5-ethyluracil), 5-halouracils (5-bromouracil), 6-azapyrimidine, 6-alkylpyrimidines (6-methyluracil), 2-thiouracil, 4-thiouracil, 4-acetylcytosine, 5-(carboxyhydroxymethyl)uracil, 5'-carboxymethylaminomethyl-2-thiouracil, 5-carboxymethylaminomethyluracil, 1-methyladenine, 1-methylhypoxanthine, 2,2-dimethylguanine, 3-methylcytosine, 2-methyladenine, 2-methylguanine, N⁶-methyladenine, 7-methylguanine, 5-methoxyaminomethyl-2-thiouracil, 5-methylaminomethyluracil, 5-methylcarbonylmethyluracil, 5-methyloxyuracil, 5-methyl-2-thiouracil, 2-methylthio-N⁶-isopentenyladenine, uracil-5-oxyacetic acid, 2-thiocytosine, purine, 2,6-diaminopurine, 2-aminopurine, isoguanine, indole, imidazole, and xanthine. The amino group or hydroxyl group of the nucleobase for B^{P} may be protected.

Examples of the "optionally protected nucleobase" include both unprotected "nucleobase" and protected "nucleobase", such as adenine, guanine, hypoxanthine, cytosine, thymine, and uracil, wherein the amino group and/or hydroxyl group is unprotected or protected.

The amino-protecting group is not particularly limited as long as it is used as a protecting group for a nucleic acid, and specific examples thereof include benzoyl, 4-methoxybenzoyl, acetyl, propionyl, butyryl, isobutyryl, phenylacetyl, phenoxyacetyl, 4-tert-butylphenoxyacetyl, 4-isopropylphenoxyacetyl, and (dimethylamino)methylene. As the amino-protecting group, benzoyl, acetyl, phenylacetyl, and 4-tert-butylphenoxyacetyl are preferable. Examples of the hydroxyl-protecting group include 2-cyanoethyl, 4-nitrophenethyl, phenylsulfonylethyl, methylsulfonylethyl, trimethylsilylethyl, phenyl optionally substituted with 1 to 5 electron-withdrawing groups at any substitutable positions, diphenylcarbamoyl, dimethylcarbamoyl, diethylcarbamoyl, methylphenylcarbamoyl, 1-pyrolidinylcarbamoyl, morpholinocarbamoyl, 4-(tert-butylcarboxy)benzyl, 4-[(dimethylamino)carboxy]benzyl, and 4-(phenylcarboxy)benzyl (see, for example, WO2009/064471A1). As the hydroxyl-protecting group, 2-cyanoethyl, 4-nitrophenethyl, and 4-(tert-butylcarboxy)benzyl are preferable. As a protecting group for the hydroxyl group at the 6-position of guanine is preferably 2-cyanoethyl.

In one embodiment, examples of the protected nucleobase include those shown below. 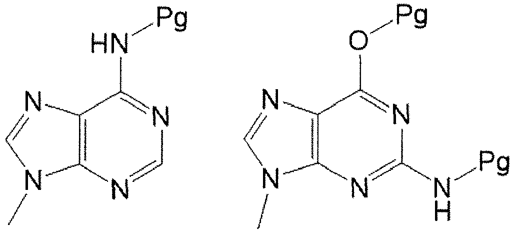 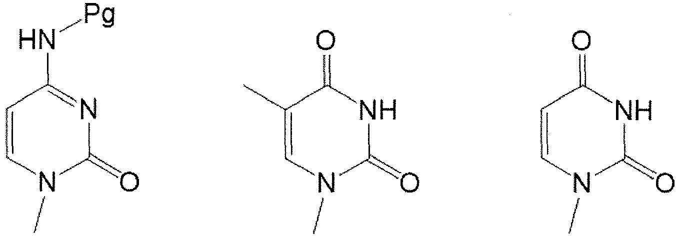 wherein
Pg represents a protecting group.

A more specific embodiment of the protected nucleobase includes, but is not limited to, adenine (A^{Bz}) having an amino group protected by benzoyl, cytosine (C^{Bz}) having an amino group protected by benzoyl, and guanine (G^{CE,Pac}) having a hydroxyl group protected by 2-cyanoethyl and an amino group protected by phenoxyacetyl.

The term "a long-chain alkyl" refers to, for example, a linear or branched alkyl having 10 to 300 carbon atoms, preferably a linear or branched alkyl having 10 to 100 carbon atoms, and more preferably a linear or branched alkyl having 10 to 30 carbon atoms.

Examples of "a long-chain alkyl" moieties in "a long-chain alkyl-carbonyl" and "a long-chain alkyloxy" include the same as defined for "a long-chain alkyl".

The term "a long-chain alkenyl" refers to, for example, a linear or branched alkenyl having 10 to 300 carbon atoms, preferably a linear or branched alkenyl having 10 to 100 carbon atoms, and more preferably a linear or branched alkenyl having 10 to 30 carbon atoms.

Examples of "a long-chain alkenyl" moieties in "a long-chain alkenyloxy" and "a long-chain alkenyl-carbonyl" include the same as defined for "a long-chain alkenyl".

Examples of "halogen" include a fluorine atom, a chlorine atom, a bromine atom, and an iodine atom.

Examples of "5- to 6-membered saturated cyclic amino" include a 5- to 6-membered saturated cyclic amino group having one or two nitrogen atoms and optionally one oxygen or sulfur atom as the ring-constituting atoms, and specific examples thereof include 1-pyrrolidinyl, 1-imidazolidinyl, piperidino, 1-piperazinyl, 1-tetrahydropyrimidinyl, 4-morpholino, 4-thiomorpholino, 1-homopiperazinyl, and oxazolidin-3-yl.

The term "C₁₋₆ alkyl" refers to a linear or branched alkyl having 1 to 6 carbon atoms, and specific examples thereof include methyl, ethyl, n-propyl, isopropyl, n-butyl, isobutyl, sec-butyl, tert-butyl, n-pentyl and n-hexyl.

The term "C₁₋₆ alkoxy" refers to a linear or branched alkoxy having 1 to 6 carbon atoms, and specific examples thereof include methoxy, ethoxy, n-propoxy, isopropoxy, n-butoxy, isobutoxy, sec-butoxy, tert-butoxy, n-pentyloxy and n-hexyloxy.

Examples of "C₁₋₆ alkoxy" moiety in "C₁₋₆ alkoxy-C₁₋₆ alkyl" include the same as defined for "C₁₋₆ alkoxy".

Examples of "C₁₋₆ alkyl" moieties in "di(C₁₋₆ alkyl) amino" , mono(amino substituted with a group removable under basic condition-C₁₋₆ alkyl)amino, di(amino substituted with a group removable under basic condition-C₁₋₆ alkyl) amino, mono(C₁₋₆ alkyl)amino-C₁₋₆ alkyl, di(C₁₋₆ alkyl)amino-C₁₋₆ alkyl, tri(C₁₋₆ alkyl)ammonio-C₁₋₆ alkyl, mono(C₁₋₆ alkyl)amino, di(C₁₋₆ alkyl) amino, tri(C₁₋₆ alkyl) ammonio, mono(amino-C₁₋₆ alkyl)amino and di(amino-C₁₋₆ alkyl)amino include the same as defined for "C₁₋₆ alkyl".

The term "C₂₋₁₀ alkylene" refers to a divalent group produced by removing one hydrogen atom from different carbon atoms constituting a linear or branched alkyl having 2 to 10 carbon atoms, and examples thereof include ethylene group, propylene group, isopropylene group, butylene group, pentylene group, and hexylene group. Such "alkylene" may be substituted with 1 to 12 halogen atoms at any substitutable positions. As the "alkylene" for L¹, ethylene is particularly preferable.

The term "C₆₋₁₀ arylene" refers to a divalent group produced by removing two hydrogen atoms from two different carbon atoms constituting a monocyclic or polycyclic aromatic hydrocarbon having 6 to 10 carbon atoms, and examples thereof include phenylene and naphthylene. Such "arylene" may be substituted with 1 to 6 halogen atoms at any substitutable positions. As the "arylene" for L¹, phenylene is particularly preferable.

Examples of "C₁₋₆ alkyl" moieties of "1, 1, 3, 3-tetra(C₁₋₆ alkyl) guanidyl", "C₁₋₆ alkoxy-C₁₋₆ alkyl", "di(C₁₋₆ alkyl) amino" , "di(C₁₋₆ alkyl)amino-C₁₋₆ alkyl", "tri(C₁₋₆ alkyl) ammonio" , "tri(C₁₋₆ alkyl) ammonio-C₁₋₆ alkyl", "mono(C₁₋₆ alkyl)amino substituted with a removable group under a basic condition", "mono(C₁₋₆ alkyl)amino-C₁₋₆ alkyl substituted with a removable group under a basic condition", "mono(amino substituted with a group removable under basic condition-C₁₋₆ alkyl)amino", and "di(amino substituted with a group removable under basic condition-C₁₋₆ alkyl)amino" include the same as defined for "C₁₋₆ alkyl".

Examples of "a removable group under an acidic condition" include trityl, monomethoxytrityl, tert-butyldimethylsilyl and dimethoxytrityl.

Examples of "a removable group under a basic condition" include trifluoroacetyl.

Examples of "a removable group under a neutral condition" include a group that can be removed by the action of tetrabutylammonium fluoride or hydrogen trifluoride/triethylamine salt, and specific examples thereof include 2-cyanoethoxymethoxy, 2-cyanoethoxy-2-ethoxy, and tert-butyldimethylsilyl.

Examples of "a silicon substituent group" include triphenylsilyl, diisopropylphenylsilyl, tert-butyldimethylsilyl, and tert-butyldiphenylsilyl.

Examples of "aryl" include phenyl.

Examples of "heteroaryl" include pyridyl, pyrimidyl, pyridazil, pyrazinyl, thienyl, and furanyl.

For "solid-phase support", any support can be used so long as it is conventionally used in a solid-phase synthesis of nucleic acids, peptides, peptide nucleic acids, sugars, or the like. Examples thereof include controlled pore glass (CPG), oxalylated controlled pore glass (see, for example, Nucleic Acids Research, Vol. 19, 1527 (1991)), TentaGel support-aminopolyethylene glycol derivatized support (see, for example, Tetrahedron Letters, Vol. 34, 3373 (1993)), Poros-polystyrene/divinylbenzene copolymers, polystyrene resins, and polyacrylamide resins.

Examples of "a soluble polymer soluble in an organic solvent" include non-crosslinked styrene polymers and polyethylene glycol derivatives.

Examples of "a soluble polymer soluble in an organic solvent" moiety of " (a soluble polymer soluble in an organic solvent)-oxy" and " (a soluble polymer soluble in an organic solvent)-amino" include the same as defined for "a soluble polymer soluble in an organic solvent".

Examples of "non-crosslinked styrene polymers" include derivatives of polystyrene not crosslinked with divinylbenzene and having a spacer such as polyethylene glycol (TentaGel series, ArgoGel series).

Examples of "polyethylene glycol derivatives" include derivatives of polyethylene glycol having a molecular weight of 100 to 40,000 and also a substituent (SUNBRIGHT (registered trademark) series).

### <Elongation Reaction>

One embodiment of the invention is a method for obtaining a compound of the formula [C-1] by reacting a compound of the formula [A-1] with a compound of the formula [B-1]: 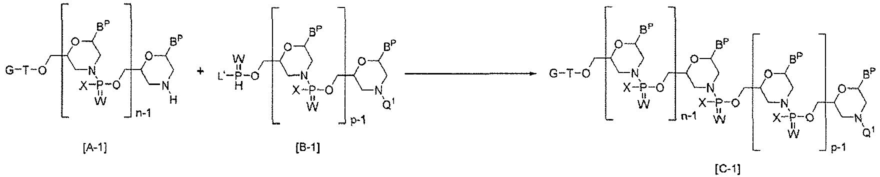 wherein
B^{P}, Q¹, W, X, G, T, n, L' and p are as defined above, and the method may comprise
Step (1): condensation; and
Step (2) oxidative amination.

One embodiment of the invention is a method for obtaining a compound of the formula [C-1] comprising that a compound of the formula [A-1]: 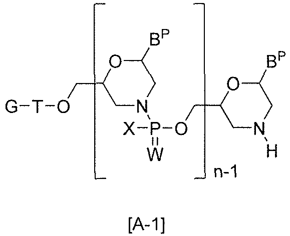 wherein
B^{P}, W, X, G, T, n and p are as defined above, and a compound of the formula [B-1]: 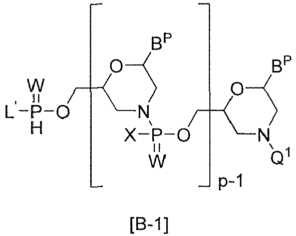 wherein
B^{P}, Q¹, W, X, L' and p are as defined above, are subjected to
   Step (1) a treatment with at least one condensation agent selected from the group consisting of Phosphorus Reagent 1, Phosphorus Reagent 2 and onium reagent in the presence of a base, and then
   Step (2) a treatment with an oxidizing agent and an organic amine,
      to obtain the compound of the formula [C-1]: 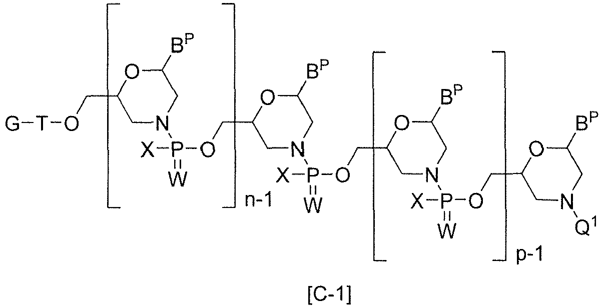 wherein
   B^{P}, Q¹, W, X, G, T, n and p are as defined above.

### <Step (1): Condensation>

### <Condensation agent in Step (1)>

In one embodiment of the invention, at least one selected from the group consisting of Phosphorus Reagent 1, Phosphorus Reagent 2 and onium reagent may be used as a condensation agent.

### <Example of the condensation agent in Step (1)>

In one embodiment of the invention, at least one selected from the group consisting of Phosphorus Reagent 1, Phosphorus Reagent 2 and onium reagent may be used as a condensation agent, and examples include diphenyl chlorophosphate, bis(2,6-dimethylphenyl)chlorophosphate, bis(2-oxo-3-oxazolidinyl)phosphinic chloride, PyBrop (bromotripyrrolidinophosphonium hexafluorophosphate), PyBOP ((benzotriazol-1-yloxy)tripyrrolidinophosphonium hexafluorophosphate), PyClop (chlorotripyrrolidinophosphonium hexafluorophosphate), PyAOP ((7-azabenzotriazol-1-yloxy)tripyrrolidinophosphonium hexafluorophosphate), PyNTP (3-nitro-1,2,4-triazol-1-yl-tris(pyrrolidin-1-yl)phosphonium hexafluorophosphate), HATU (1-[bis(dimethylamino)methylene]-1H-1,2,3-triazolo[4,5-b]pyridinium 3-oxide hexafluorophosphate), COMU ((1-cyano-2-ethoxy-2-oxoethylideneaminooxy)dimethylamino-morpholino-carbenium hexafluorophosphate), HBTU (1-[bis(dimethylamino)methylene]-1H-benzotriazolium 3-oxide hexafluorophosphate), BOI (2-(benzotriazol-1-yl)oxy-1,3-dimethylimidazolidinium hexafluorophosphate), and DMINTP (1-(1',3'-dimethyl-1H-imidazol-2'-yl)-3-nitro-1,2,4-triazole hexafluorophosphate), preferably diphenyl chlorophosphate, bis(2,6-dimethylphenyl)chlorophosphate, PyBrop and BOI, and more preferably diphenyl chlorophosphate, bis(2,6-dimethylphenyl)chlorophosphate and PyBrop.

In one embodiment of the invention, the Phosphorus Reagent 1 is a compound of the formula [P-2]: 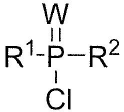 wherein
W is an oxygen atom or a sulfur atom; and
R¹ and R² are each the same or different and are each selected from the group consisting of an aromatic ring-O- optionally substituted and a heterocyclic ring optionally substituted, preferably phenyl, 2,6-dimethylphenyl and 2-oxo-oxazolidide.

The reagent is preferably one selected from the group consisting of diphenyl chlorophosphate, bis(2,6-dimethylphenyl)chlorophosphate and bis(2-oxo-3-oxazolidinyl)phosphinic chloride, and more preferably diphenyl chlorophosphate and bis(2,6-dimethylphenyl)chlorophosphate.

In one embodiment of the invention, the Phosphorus Reagent 2 is a compound of the formula [P-3]: 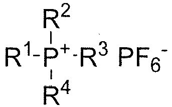 wherein
R¹, R², R³ and R⁴ are each the same or different and are each selected from the group consisting of halogen, C₁₋₆ alkylamino, di (C₁₋₆ alkyl) amino, cyclic C₁₋₆ amino optionally substituted, aromatic amino optionally substituted, aromatic ring optionally substituted and hetero ring-O- optionally substituted; or
R¹ and R² are taken together with the phosphorous atom to which they are attached to form a ring structure optionally substituted, and R³ and R⁴ are each the same or different and are each selected from the group consisting of halogen, C₁₋₆ alkylamino, di (C₁₋₆ alkyl) amino, cyclic C₁₋₆ amino optionally substituted, aromatic amino optionally substituted, aromatic ring optionally substituted and hetero ring-O- optionally substituted; and
preferably, R¹, R² and R³ are cyclic C₁₋₆ amino optionally substituted, and R⁴ is halogen, aromatic amino optionally substituted, or hetero ring-O- optionally substituted, and
more preferably, R¹, R² and R³ are pyrrolidyl, and R⁴ is chloro, bromo, benzotriazol-1-yloxy, 7-azabenzotriazol-1-yloxy or 3-nitro-1,2,4-triazol-1-yl.

The reagent is preferably one selected from the group consisting of PyBrop, pyBOP, PyClop, PyAOP and PyNTP, more preferably PyBrop.

In one embodiment of the invention, the onium reagent is a compound of the formula [O]: 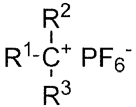 wherein
R¹, R², R³ and R⁴ are each the same or different and are each selected from the group consisting of halogen, C₁₋₆ alkylamino, di(C₁₋₆ alkyl)amino, cyclic C₁₋₆ amino optionally substituted, aromatic amino optionally substituted, aromatic ring optionally substituted, hetero ring-O- optionally substituted and C₁₋₆ arkylidene aminooxy optionally substituted; or
R¹ and R² are taken together with the carbon atom to which they are attached to form a ring structure, and R³ is selected from the group consisting of halogen, C₁₋₆ alkylamino, di(C₁₋₆ alkyl)amino, cyclic C₁₋₆ amino optionally substituted, aromatic amino optionally substituted, aromatic ring optionally substituted, and hetero ring-O- optionally substituted,
preferably, R¹ and R² are each the same or different and are each selected from the group consisting of di(C₁₋₆ alkyl)amino and cyclic C₁₋₆ amino optionally substituted, and R³ is hetero ring-O- optionally substituted or C₁₋₆ arkylidene aminooxy optionally substituted; or R¹ and R² are taken together with the carbon atom to which they are attached to form a ring structure, and aromatic amino optionally substituted, and
more preferably, R¹ and R² are dimethylamino, and R³ is O-(7-azabenzotriazol-1-yl) or O-(benzotriazol-1-yl) ; or R¹ is dimethylamino, R² is morpholino, and R³ is 1-cyano-2-ethoxy-2-oxoethylidenaminooxy.

The reagent is preferably one selected from the group consisting of HATU, COMU, HBTU, BOI and DMINTP, and more preferably BOI.

### <Base in Step (1)>

In one embodiment of the invention, the base may be at least one selected from the group consisting of primary amines, secondary amines, tertiary amines, cyclic amines and aromatic amines, and examples include diisopropylethylamine, triethylamine, N-ethylmorpholine, pyridine, lutidine, collidine, N-methylimidazole, N,N-dimethylimidazole and 1-benzylimidazole, preferably, diisopropylethylamine, pyridine, N-methylimidazole and 1-benzylimidazole, and more preferably, pyridine and N-methylimidazole.

### <Combination of condensation agent and base in Step (1)>

In one embodiment of the invention, the combination of the condensation agent and the base is not limited, but examples include the combinations of diphenyl chlorophosphate and pyridine; diphenyl chlorophosphate and N-methylimidazole; bis(2,6-dimethylphenyl)chlorophosphate and pyridine; bis(2,6-dimethylphenyl)chlorophosphate and N-methylimidazole; PyBroP and pyridine; PyBroP and N-methylimidazole; PyBroP and diisopropylethylamine; and BOI and diisopropylethylamine; preferably the combinations of diphenyl chlorophosphate and pyridine; diphenyl chlorophosphate and N-methylimidazole; bis(2,6-dimethylphenyl)chlorophosphate and pyridine; bis(2,6-dimethylphenyl)chlorophosphate and N-methylimidazole; PyBroP and pyridine; PyBroP and N-methylimidazole; PyBroP and diisopropylethylamine; and more preferably the combination of diphenyl chlorophosphate and pyridine; diphenyl chlorophosphate and N-methylimidazole; bis(2,6-dimethylphenyl)chlorophosphate and N-methylimidazole; PyBroP and N-methylimidazole; and PyBroP and diisopropylethylamine.

### <Amount of the condensation agent in Step (1)>

In one embodiment of the invention, the condensation agent may be used in an amount, for example, within the range of 0.5 to 100 molar equivalent, preferably 1 to 50 molar equivalent, and more preferably 2 to 20 molar equivalent, with respect to the compound of the formula [B-1].

### <Amount of the base in Step (1)>

In one embodiment of the invention, the base may be used in an amount, for example, within the range of 1 to 100 molar equivalent, preferably 2 to 50 molar equivalent, and more preferably 2 to 40 molar equivalent, with respect to the compound of the formula [A-1].

### <Reaction Solvent in Step (1)>

In one embodiment of the invention, the solvent used in Step (1) is not limited and may be at least one selected from the group consisting of dichloromethane, chloroform, 1,2-dichloroethane, tetrahydrofuran, toluene, acetonitrile and ethyl acetate.

### <Reaction Temperature in Step (1)>

In one embodiment of the invention, the reaction temperature in Step (1) is not limited and may be -10 to 80°C, preferably 0 to 60°C, and more preferably 10 to 40°C.

### <Reaction Time in Step (1)>

In one embodiment of the invention, the reaction time in Step (1) is not limited and may be 1 minutes to 12 hours, preferably 2 minutes to 4 hours, and more preferably 5 minutes to 2 hours.

### <(2) :oxidative amination>

### <Oxidizing agent in Step (2)>

In one embodiment of the invention, the oxidizing agent is not limited so long as it can oxidize a phosphorus atom, and examples include iodine and carbon tetrachloride, preferably a solution of iodine in dichloromethane, a solution of iodine in chloroform, a solution of iodine in tetrahydrofuran, and more preferably a solution of iodine in tetrahydrofuran.

### <Amount of the oxidizing agent in Step (2)>

In one embodiment of the invention, the condensation agent may be used in an amount, for example, within the range of 0.5 to 100 molar equivalent, preferably 1 to 50 molar equivalent, and more preferably 2 to 10 molar equivalent, with respect to the compound of the formula [B-1].

### <Organic amine in Step (2)>

In one embodiment of the invention, the organic amine is not limited and may be at least one selected from the group consisting of primary amines, secondary amines, tertiary amines, cyclic amines and aromatic amines, and examples include dimethylamine, diisopropylamine, piperidine, pyrrolidine, N-methylpiperazine and morpholine, preferably dimethylamine, diisopropylamine, piperidine or pyrrolidine, and more preferably dimethylamine .

### <Amount of the organic amine in Step (2)>

In one embodiment of the invention, the organic amine may be used in an amount, for example, within the range of 1 to 200 molar equivalent, preferably 2 to 100 molar equivalent, and more preferably 2 to 50 molar equivalent, with respect to the compound of the formula [B-1].

### <Reaction Solvent in Step (2)>

In one embodiment of the invention, the solvent used in Step (2) is not limited and may be at least one selected from the group consisting of dichloromethane, chloroform, 1,2-dichloroethane, tetrahydrofuran, toluene, acetonitrile and ethyl acetate.

### <Reaction Temperature in Step (2)>

In one embodiment of the invention, the reaction temperature in Step (2) is not limited and may be -10 to 80°C, preferably 0 to 60°C, and more preferably 0 to 40°C.

### <Reaction Time in Step (2)>

In one embodiment of the invention, the reaction time in Step (1) is not limited and may be 1 minute to 4 hours, preferably 2 minutes to 2 hours, and more preferably 2 minutes to 1 hours.

### <Compound of formula [A-1]>

### <Synthesis of compound of formula [A-1]>

One embodiment of the invention comprises a process to obtain a compound of the formula [A-1] from a compound of the formula [A-1-0]: 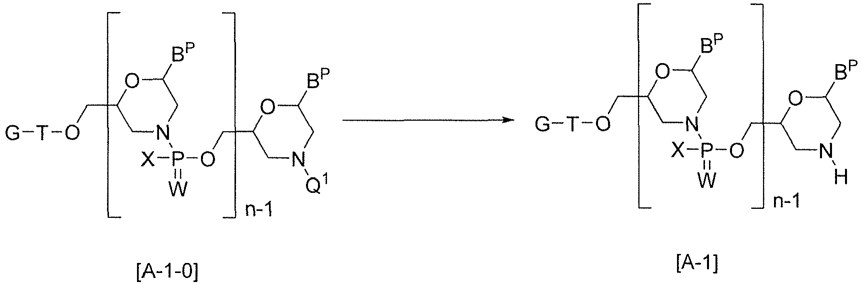 wherein
B^{P}, Q¹, W, X, G, T and n are as defined above, and
n is an integer from 1 to 50, preferably from 1 to 30, more preferably from 1 to 25, and most preferably from 1 to 15.

The compound of the formula [A-1-0] can be prepared using a method known in the art.

### <Compound of formula [B-1]>

One embodiment of the invention comprises a compound of the formula [B-1] wherein p=1: 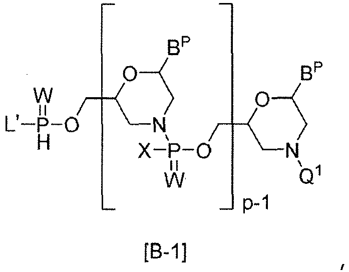 examples of which include those shown in the following table.

**[Table 1]**

| **Abbreviation** | **Chemical Structure** | **Abbreviation** | **Chemical Structure** |
|---|---|---|---|
| morA-HP | 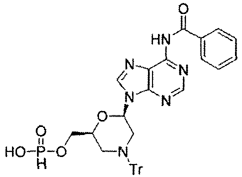 | morA-HP2 | 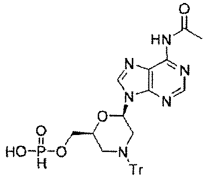 |
| morC-HP | 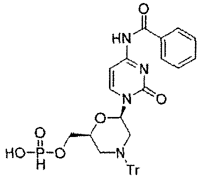 | morC-HP2 | 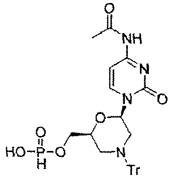 |
| morU-HP | 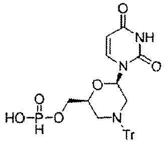 | morT-HP | 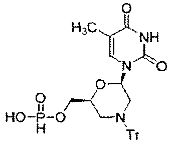 |
| morG-HP | 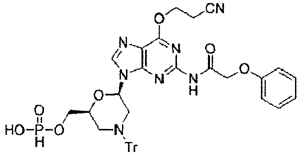 | morG-HP2 | 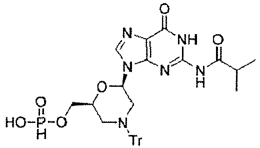 |
| morG-HP3 | 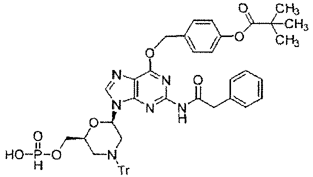 | | |

### <Preparation of compound of formula [B-1]>

One embodiment of the invention may comprise a process to obtain a compound of the formula [B-1] from a compound of the formula [B-1-0]: 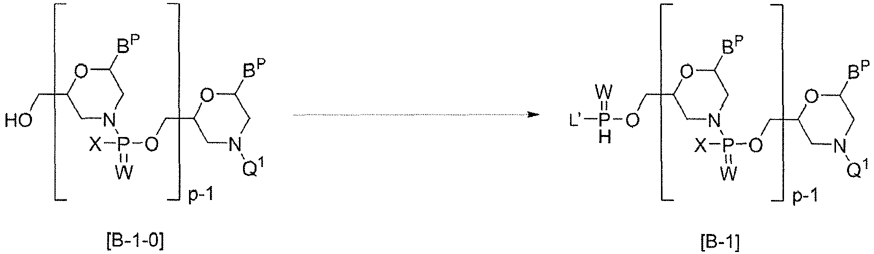 wherein
B^{P}, Q¹, W, X, L' and p are as defined above, and the process may comprise
Step (1): phosphonation;
Step (2): hydrolysis; and optionally
Step (3): ion exchange.

The compound of the formula [B-1-0] can be prepared using a method known in the art.

Examples of a compound of the formula [B-1-0] wherein p=1 include those shown in the following table.

**[Table 2]**

| **Abbreviation** | **Chemical Structure** | **Abbreviation** | **Chemical Structure** |
|---|---|---|---|
| morA-OH | 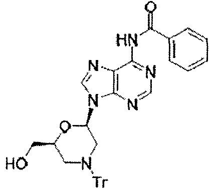 | morA-OH2 | 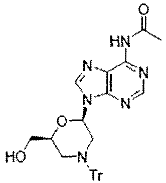 |
| morC-OH | 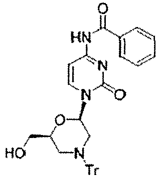 | morC-OH2 | 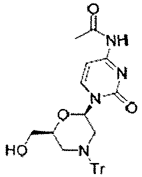 |
| morU-OH | 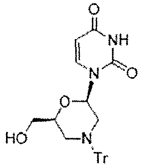 | morT-OH | 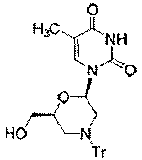 |
| morG-OH | 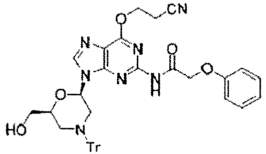 | morG-OH2 | 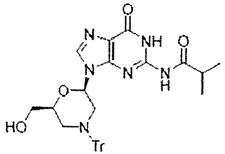 |
| morG-OH3 | 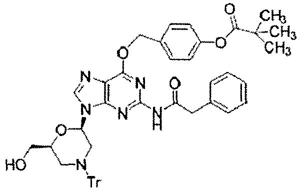 | | |

### <Step (1): phosphonation>

One embodiment of the invention comprises a step wherein a compound of the formula [B-1-0]: 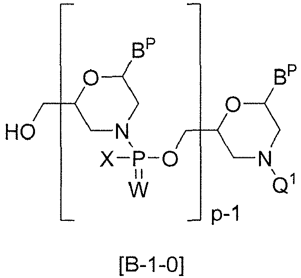 wherein
B^{P}, Q¹, W, X and p are as defined above, is treated with a compound of the formula [P-1]: 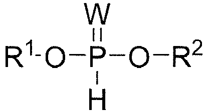 and a base
   wherein
W is an oxygen atom or a sulfur atom, and
R¹ and R² are each the same or different and are each selected from the group consisting of H, C₁₋₆ alkyl optionally substituted, phenyl optionally substituted, and PH(=O)-OH,
   to obtain a compound of the formula [B-1'-0]: 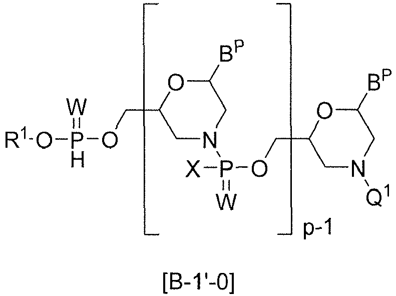 wherein
B^{P}, Q¹, W, X, R¹ and p are as defined above.

### <Reagent for phosphonylation in Step (1)>

In one embodiment of the invention, the compound of the formula [P-1]: 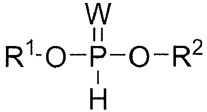 wherein
W is as defined above, and
R¹ and R² are each the same or different and are each selected from the group consisting of H, C₁₋₆ alkyl optionally substituted, phenyl optionally substituted, and PH(=O)-OH;
preferably R¹ is H, C₁₋₆ alkyl optionally substituted or phenyl optionally substituted,
R² is C₁₋₆ alkyl optionally substituted, phenyl optionally substituted or PH(=O)-OH, and
more preferably R¹ and R² are phenyl,
is preferably diphenyl phosphite, phosphorous acid, bis(1,1,1,3,3,3-hexafluoropropan-2-yl)phosphonate or diphosphonic acid, more preferably diphenyl phosphite.

### <Base in Step (1)>

In one embodiment of the invention, the base is at least one selected from the group consisting of primary amines, secondary amines, tertiary amines, cyclic amines and aromatic amines, and examples include N-ethylmorpholine, pyridine, triethylamine, and N-methylimidazole, preferably N-ethylmorpholine or pyridine, and more preferably N-ethylmorpholine.

### <Combination of reagent for phosphonylation and base in Step (1) >

In one embodiment of the invention, the combination of the reagent for phosphonylation and the base is not limited, but examples include the combinations of diphenyl phosphite and N-ethylmorpholine; diphenyl phosphite and pyridine; diphenyl phosphite and N-methylimidazole; phosphorous acid and pyridine; preferably diphenyl phosphite and N-ethylmorpholine; diphenyl phosphite and pyridine; and more preferably diphenyl phosphite and N-ethylmorpholine.

### <Amount of reagent for phosphonylation in Step (1)>

In one embodiment of the invention, the compound of the formula [P-1] may be used in an amount, for example, within the range of 1 to 100 molar equivalent, preferably 1 to 40 molar equivalent, and more preferably 2 to 20 molar equivalent, with respect to the compound of the formula [B-1-0] .

### <Amount of the base in Step (1)>

In one embodiment of the invention, the base may be used in an amount, for example, within the range of 1 to 100 molar equivalent, preferably 1 to 40 molar equivalent, and more preferably 4 to 40 molar equivalent, with respect to the compound of the formula [B-1-0].

### <Reaction Solvent in Step (1)>

In one embodiment of the invention, the solvent used in Step (1) is not limited and may be at least one selected from the group consisting of dichloromethane, chloroform, 1,2-dichloroethane, tetrahydrofuran, toluene and ethyl acetate.

### <Reaction Temperature in Step (1)>

In one embodiment of the invention, the reaction temperature in Step (1) is not limited and may be -10 to 60°C, preferably 0 to 50°C, and more preferably 20 to 50°C.

### <Reaction Time in Step (1)>

In one embodiment of the invention, the reaction time in Step (1) is not limited and may be 5 minutes to 24 hours, preferably 10 minutes to 18 hours, and more preferably 30 minutes to 12 hours.

### <Step (2): hydrolysis>

One embodiment of the invention comprises a step wherein a compound of the formula [B-1'-0]: 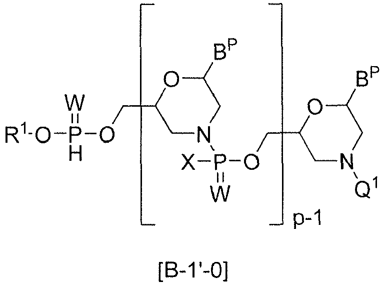 wherein
B^{P}, Q¹, W, X, R¹ and p are as defined above,
   is treated with a hydrolyzing solution to obtain a compound of the formula [B-1]: 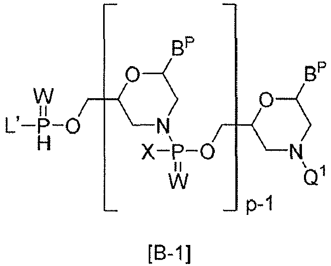 wherein
B^{P}, Q¹, W, X and p are as defined above, and L' is OH, -O⁻N⁺H(aliphatic amine), -O⁻N⁺H(cyclic amine) or -O-N⁺H(aromatic amine).

In one embodiment of the invention, in the formula [B-1], if B^{P}, Q¹, W, X and p are as defined above, and L' is -O-N⁺H(aliphatic amine), -O⁻N⁺H(cyclic amine) or -O-N⁺H(aromatic amine), then the compound of the formula [B-1] is an aliphatic amine salt, a cyclic amine salt or an aromatic amine salt. For example, it is a salt that the compound of the formula [B-1] wherein B^{P}, Q¹, W, X and p are as defined above, and L' is -OH, forms with aliphatic amine, cyclic amine or aromatic amine.

In one embodiment of the invention, in the formula [B-1], B^{P}, Q¹, W, X and p are as defined above, and L' is preferably -O⁻N⁺H(C₁₋₆ alkyl)₃ or -O⁻N⁺H (cyclic amine), and -O⁻N⁺H(C₁₋₆ alkyl)₃ is, for example, -O⁻N⁺H(CH₂ CH₃)₃, and -O⁻N⁺H(cyclic amine) is, for example, -O⁻(HDBU)⁺, and DBU is 1,8-diazaabicyclo[5.4.0]undec-7-ene.

### <Hydrolyzing solution in Step (2)>

In one embodiment of the invention, the hydrolyzing solution is, for example, an aqueous solution containing a base, which is at least one selected from the group consisting of aliphatic amines (primary amines, secondary amines, tertiary amines), cyclic amines and aromatic amines. The aliphatic amine is, for example, trimethylamine, triethylamine, diisopropylethylamine, or the like, the cyclic amine is, for example, diazaabicycloundecene (DBU:1,8-diazabicyclo[5.4.0]undec-7-ene), piperidine, piperazine, morpholine, N-methylmorpholine, or the like, and the aromatic amine is, for example, pyridine, imidazole, N-methylimidazole, or the like.

In one embodiment of the invention, the hydrolyzing solution is, for example, an aqueous solution containing a base, which is at least one selected from the group consisting of triethylamine, diazaabicycloundecene, N-methylimidazole, and N-methylmorpholine, and the solution is preferably an aqueous solution containing the base and carbon dioxide, more preferably an aqueous triethylammonium bicarbonate solution or aqueous diazaabicycloundecene bicarbonate solution.

### <Amount of hydrolyzing solution in Step (2)>

In one embodiment of the invention, the hydrolyzing solution may be used in an amount, for example, within the range of 1 to 100 molar equivalent, preferably 1 to 50 molar equivalent, and more preferably 2 to 20 molar equivalent, with respect to the compound of the formula [B-1'-0].

### <Reaction solvent in Step (2)>

In one embodiment of the invention, the solvent used in Step (2) is not limited and may be at least one selected from the group consisting of dichloromethane, chloroform, 1,2-dichloroethane, tetrahydrofuran, toluene, acetonitrile and ethyl acetate.

### <Reaction Temperature in Step (2)>

In one embodiment of the invention, the reaction temperature in Step (2) is not limited and may be -10 to 60°C, preferably 0 to 40°C, and more preferably 0 to 30°C.

### <Reaction Time in Step (2)>

In one embodiment of the invention, the reaction time in Step (2) is not limited and may be 1 minutes to 24 hours, preferably 10 minutes to 12 hours, and more preferably 10 minutes to 2 hours.

### <Step (3): Ion exchange>

One embodiment of the invention comprises a step wherein a compound of the formula [B-1'-0]: 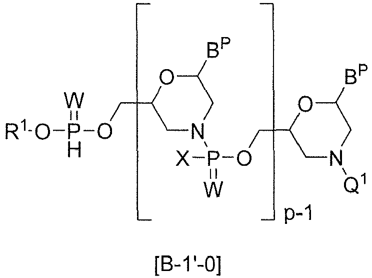 wherein
B^{P}, Q¹, W, X, R¹ and p are as defined above,
   is treated with a hydrolyzing solution to obtain a compound of the formula [B-1']: 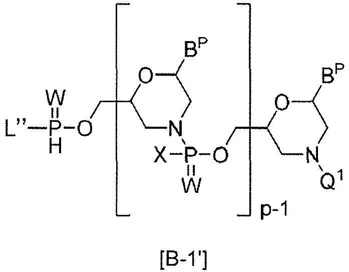 wherein
B^{P}, Q¹, W, X and p are as defined above, and L" is O⁻N+H(C₁₋₆ alkyl)₃,
   and the compound is further treated with a hydrolyzing solution to obtain a compound of the formula [B-1]: 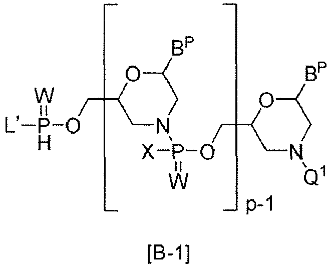 wherein
B^{P}, Q¹, W, X and p are as defined above, L' is -O⁻N⁺H(cyclic amine) such as -O⁻(HDBU)⁺.

### <Hydrolyzing solution in Step (3)>

In one embodiment of the invention, the hydrolyzing solution is, for example, an aqueous solution containing a cyclic amine, preferably an aqueous solution containing a cyclic amine and carbon dioxide, and more preferably an aqueous diazaabicycloundecene bicarbonate solution.

### <Amount of hydrolyzing solution in Step (3)>

In one embodiment of the invention, the hydrolyzing solution may be used in an amount, for example, within the range of 1 to 50 molar equivalent, preferably 1 to 50 molar equivalent, and more preferably 2 to 20 molar equivalent, with respect to the compound of the formula [B-1'-0] or the compound of the formula[B-1'].

### <Reaction solvent in Step (3)>

In one embodiment of the invention, the solvent used in Step (3) is not limited and may be at least one selected from the group consisting of dichloromethane, chloroform, 1,2-dichloroethane, tetrahydrofuran, toluene and ethyl acetate.

### <Reaction Temperature in Step (3)>

In one embodiment of the invention, the reaction temperature in Step (3) is not limited and may be -10 to 60°C, preferably 0 to 40°C, and more preferably 0 to 30°C.

### <Reaction Time in Step (3)>

In one embodiment of the invention, the reaction time in Step (3) is not limited and may be 1 minutes to 24 hours, preferably 10 minutes to 12 hours, and more preferably 10 minutes to 2 hours.

### <Removal of intramolecular Q¹ of Compound [C-1]>

One embodiment of the invention comprises a step for removing Q¹ from a compound of the formula [C-1] to obtain a compound of the formula [C-0-1]: 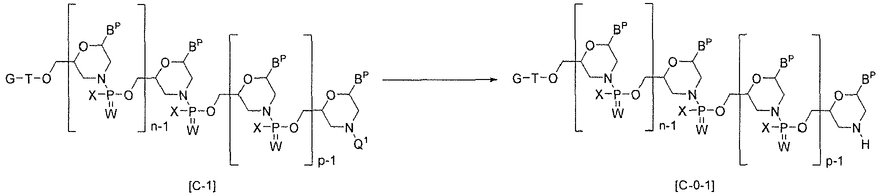 wherein
B^{P}, W, X, G, T, n and p are as defined above, and Q¹ is a group removable under acidic condition, preferably trityl, monomethoxytrityl or dimethoxytrityl, more preferably trityl or dimethoxytrityl, and most preferably trityl.

One embodiment of the invention comprises a step wherein a compound of the formula [C-1]: 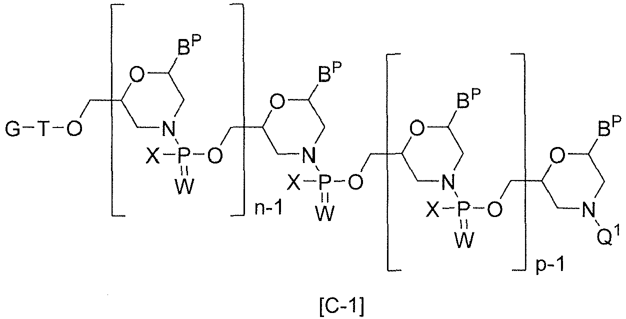 wherein
B^{P}, Q¹, W, X, G, T, n and p are as defined above,
   is treated with an acid only or with an acid and a scavenger to obtain a compound of the formula [C-0-1]: 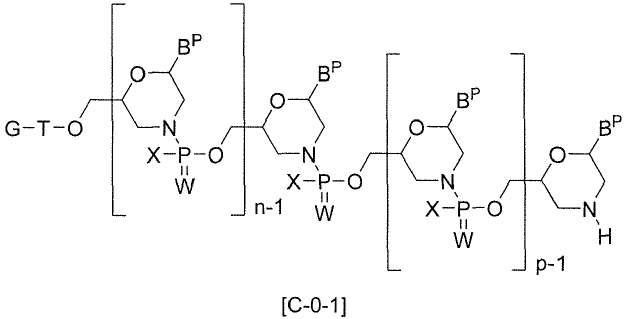 wherein
B^{P}, W, X, G, T, n and p are as defined above.

### <Acid: Reagent for removing Q¹>

In one embodiment of the invention, the acid is not limited so long as it is generally used in the art and may be trifluoroacetic acid, cyanopyridinetrifluoroacetic acid, triethylamine trifluoroacetic acid, cyanoacetic acid, trichloro acetic acid, phosphoric acid, methanesulfonic acid, p-toluenesulfonic acid, or hydrochloric acid. When using the acid, a base such as triethylamine may be used in combination to adjust acidity.

In one embodiment of the invention, the acid may be a deblocking solution commercially available for nucleic acid synthesis, such as Deblocking Solution-1 (3w/v% trichloroacetic acid/dichloromethane solution, FUJIFILM Wako Pure Chemical Corporation), or Deblocking Mix (3% dichloroacetic acid/dichloromethane solution, Glen Research Corporation).

### <Amount of the acid: Reagent for removing Q¹>

In one embodiment of the invention, the acid may be used in an amount, for example, within the range of 1 to 500 molar equivalent, preferably 2 to 200 molar equivalent, and more preferably 2 to 50 molar equivalent, with respect to the compound of the formula [C-1].

### < Concentration of the acid: Reagent for removing Q¹>

In one embodiment of the invention, the acid is suitably diluted to a concentration, for example, within the range of 1% to 80%, preferably 2% to 50%, using a reaction solvent.

### <Reaction solvent for removing Q¹>

In one embodiment of the invention, the solvent for removing Q¹ is not limited so long as it is a solvent generally used in the art, and a single solvent may be used, or two or more solvents may be used in combination as a mixed solvent.

In one embodiment of the invention, examples of the solvent for removing Q¹ include aromatic solvents such as benzene, toluene, xylene, mesitylene and the like; ester solvents such as ethyl acetate, isopropyl acetate and the like; aliphatic solvents such as hexane, pentane, heptane, octane, nonane, cyclohexane and the like; and halogenated solvents. These solvents may be used in combination as a mixed solvent.

In one embodiment of the invention, the solvent for removing Q¹ is, for example, chloroform, dichloromethane, 1,1-dichloroethane, 1,2-dichloroethane, 1,1,2-trichloroethane, 1,2-dichloroethylene, 2,2,2-trifluoroethanol or a mixture thereof, preferably one selected from the group consisting of chloroform, dichloromethane and 2,2,2-trifluoroethanol.

### <Scavenger: Reagent for removing Q¹>

In one embodiment of the invention, examples of the scavenger include ethanol, triisopropylsilane, 1-hydroxybenzotriazole, pyrrole, indole, 2,2,2-trifluoroethanol, methanol, anisole, mercaptoethanol and thioanisole, preferably one selected from the group consisting of ethanol, triisopropylsilane and 2,2,2-trifluoroethanol.

### <Amount of the scavenger: Reagent for removing Q¹>

In one embodiment of the invention, the scavenger may be used in an amount, for example, within the range of 1 to 100 molar equivalent, preferably 1 to 50 molar equivalent, and more preferably 1 to 20 molar equivalent, with respect to the compound of the formula [C-1].

### <Reaction Temperature for removing Q¹>

In one embodiment of the invention, the reaction temperature for removing Q¹ is not limited and may be -10 to 40°C, preferably 0 to 30°C, and more preferably 0 to 25°C.

### <Reaction Time for removing Q¹>

In one embodiment of the invention, the reaction time for removing Q¹ is not limited and may be 1 minute to 24 hours, preferably 10 minutes to 6 hours, and more preferably 10 minutes to 2 hours.

In one embodiment of the present invention, in a method for producing compound [C-0-1], the reaction to remove Q¹ may be carried out in situ by adding a solution containing an acid to the reaction mixture containing the compound [C-1], which is produced by the condensation reaction between compound [A-1] and compound [B-1].

In one embodiment of the invention, the solvent for the in-situ reaction is not limited so long as it is a solvent generally used in the art, and a single solvent may be used, or two or more solvents may be used in combination.

In one embodiment of the invention, examples of the solvent for the in-situ reaction include aromatic solvents such as benzene, toluene, xylene, mesitylene and the like; ester solvents such as ethyl acetate, isopropyl acetate and the like; aliphatic solvents such as hexane, pentane, heptane, octane, nonane, cyclohexane and the like; and halogenated solvents. These solvents may be used in combination.

One embodiment of the invention comprises a step wherein Q¹ is removed from a compound of the formula [A-0] to obtain a compound of the formula [A-1] : 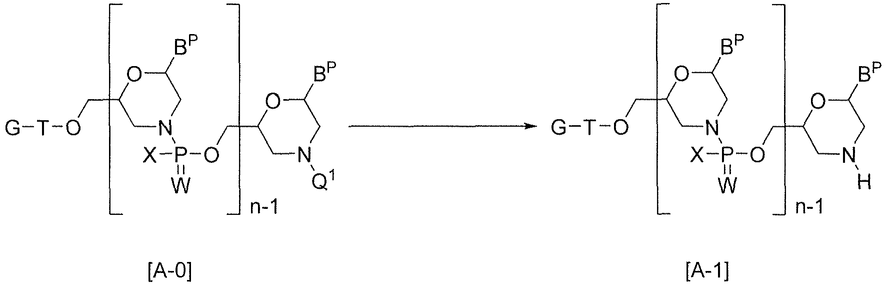 wherein
B^{P}, W, X, G, T, n and p are as defined above, and Q¹ is a group removable under acidic condition, preferably trityl, monomethoxytrityl or dimethoxytrityl, more preferably trityl or dimethoxytrityl, and most preferably trityl.

One embodiment of the invention comprises a step wherein a compound of the formula [A-0]: 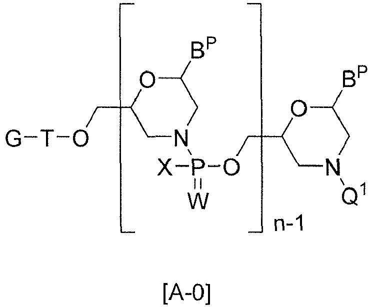 wherein
B^{P}, Q¹, W, X, G, T and n are as defined above, is treated with an acid only or with an acid and a scavenger to obtain a compound of the formula [A-1]: 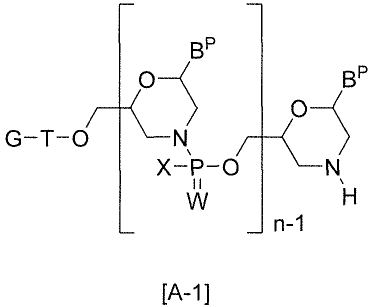 wherein
B^{P}, W, X, G, T and n are as defined above.

In one embodiment of the invention, in the step for removing Q¹ from a compound of the formula [A-0] to obtain a compound of the formula [A-1], the same "acid", "amount of the acid", "scavenger", "amount of the scavenger", "reaction solvent", "reaction time" and "reaction temperature" as described above in the section <Removal of intramolecular Q¹ of Compound [C-1]> can be used.

### <Removing hydroxyl-protecting group>

One embodiment of the invention comprises a step wherein G-T is removed from a compound of the formula [C-1] to obtain a compound of the formula [C-0-2]: 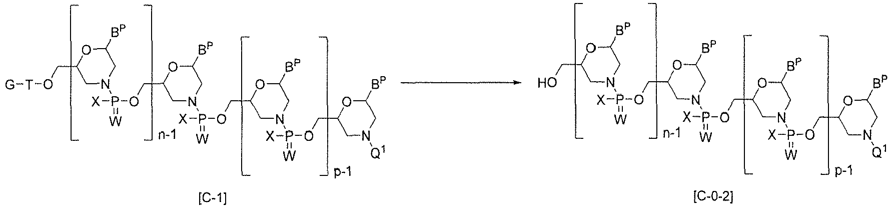 wherein
B^{P}, Q¹, W, X, G, T, n and p are as defined above.

One embodiment of the invention comprises that a compound of the formula [C-1]: 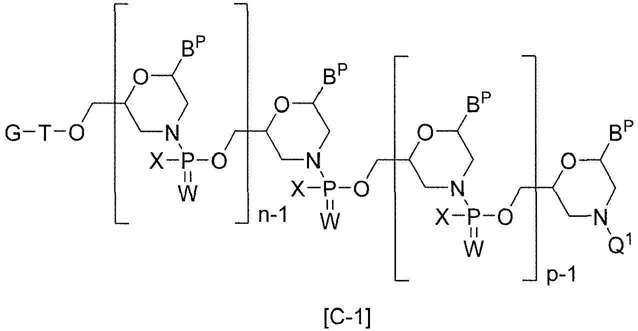 wherein
B^{P}, Q¹, W, X and p are as defined above, and G is
   (1) a silicon substituent group,
   (2) C₁₋₁₈ alkyl-carbonyl optionally substituted, C₁₋₁₈ alkoxy-carbonyl optionally substituted, a long-chain alkyl-carbonyl, or a long-chain alkoxy-carbonyl,
   (3) benzoyl substituted with one to five long-chain alkyloxy and/or long-chain alkenyloxy, or
   (4) a substituent group represented by the general formula [7] : 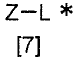 wherein
      * is a binding position with T;
      Z is
         (1) (a soluble polymer soluble in an organic solvent)-oxy,
         (2) (a soluble polymer soluble in an organic solvent)-amino,
         (3) a long-chain alkyloxy, benzoyl substituted with one to five long-chain alkyloxy and/or long-chain alkenyloxy, or benzyl substituted with one to five long-chain alkyloxy and/or long-chain alkenyloxy,
         (4) a solid-phase support, or
         (5) a substituent group represented by the following general formula [8A] to [8N]: 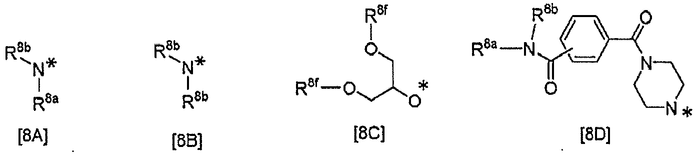 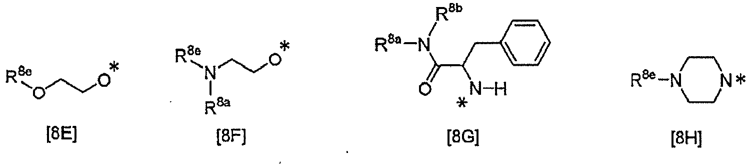 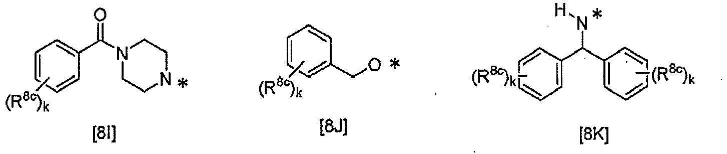 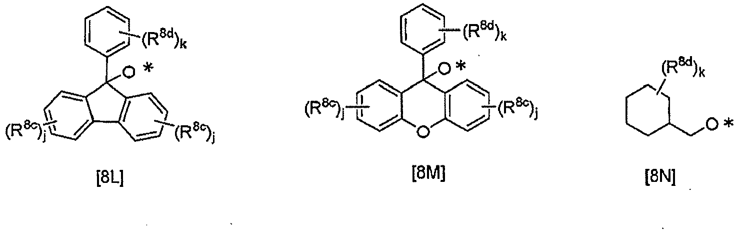
      wherein
         * is a binding position with L;
         j is an integer from 0 to 4;
         k is an integer from 0 to 5;
         R^{8a} is a hydrogen atom or C₁₋₆ alkyl;
         R^{8b} is each the same or different and is each a long-chain alkyl;
         R^{8c} is each the same or different and is each a substituent group of the general formulae selected from [9A] to [9E] 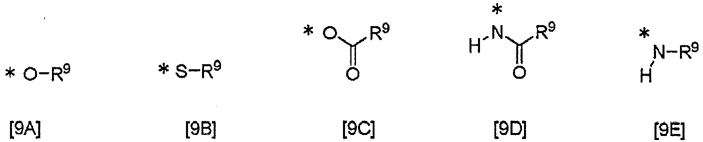
         wherein
            * is a binding position; and
            R⁹ is a long-chain alkyl and/or a long-chain alkenyl;
         R^{8d} is each the same or different and is each a hydrogen atom, halogen, a long-chain alkyl optionally substituted with 1 to 13 halogen atoms or a long-chain alkyloxy optionally substituted with 1 to 13 halogen atoms;
         R^{8e} is
            (1) a long-chain alkyl,
            (2) a long-chain alkyl-carbonyl, or
            (3) benzoyl substituted with one to five long-chain alkyloxy and/or long-chain alkenyloxy; and
         R^{8f} is
            (1) a long-chain alkyl,
            (2) a long-chain alkyl-carbonyl, or
            (3) a long-chain alkenyl-carbonyl; and
               L is a group of the general formula [10]: 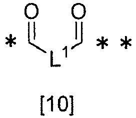
               wherein
                  * is a binding position with Z;
                  ** is a binding position with T; and
                  L¹ is C₂₋₁₀ alkylene optionally substituted or C₆₋₁₀ arylene optionally substituted; or a group of the general formula [10-1]: 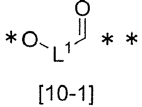
               wherein
                  * is a binding position with Z;
                  ** is a binding position with T; and
                  L¹ is C₁₋₁₀ alkylene optionally substituted or C₆₋₁₀ arylene optionally substituted;
n is an integer from 1 to 50; and
T is a single bond or a substituent group of the general formula [11], provided that T is a single bond when G is a silicon substituent group: 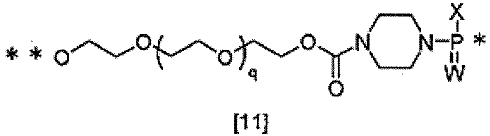
wherein
   X and W are as defined above;
   * is a binding position with **O, *O or *N of the above formulae [4a] to [4d];
   ** is a binding position with G; and
   q is an integer from 0 to 10,
is treated under a condition for removing the hydroxyl-protecting group to obtain a compound of the formula [C-0-2] : 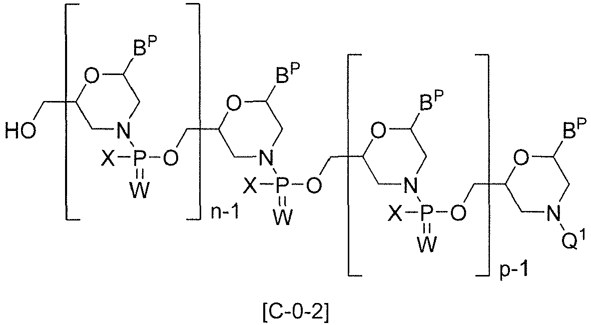 wherein
B^{P}, Q¹, W, X, n and p are as defined above.

In one embodiment, the compound of the formula [C-1]: 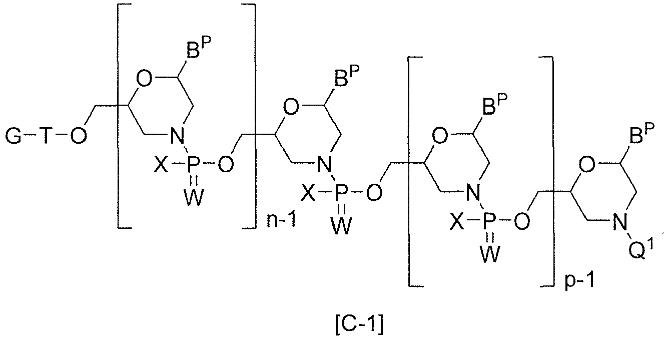
is a compound
wherein
   B^{P}, Q¹, W, X and p are as defined above, and
   G is
      (1) a silicon substituent group,
      (2) C₁₋₁₈ alkyl-carbonyl optionally substituted, C₁₋₁₈ alkoxy-carbonyl optionally substituted, a long-chain alkyl-carbonyl, or a long-chain alkoxy-carbonyl, or
      (3) benzoyl substituted with one to five long-chain alkyloxy and/or long-chain alkenyloxy, and
   n is an integer from 1 to 50; and
   T is a single bond.

In one embodiment, the compound of the formula [C-1]: 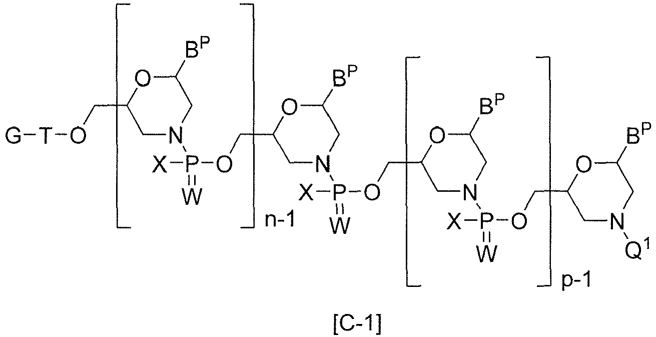
is a compound
wherein
   B^{P}, Q¹, W, X and p are as defined above, and
   G is
(4) a substituent group represented by the general formula [7] :

   Z-L * [7]

   wherein
   * is a binding position with T;
   Z is
   (3) a long-chain alkyloxy, benzoyl substituted with one to five long-chain alkyloxy and/or long-chain alkenyloxy, or benzyl substituted with one to five long-chain alkyloxy and/or long-chain alkenyloxy,
   (4) a solid-phase support, or
   (5) a substituent group represented by the following general formula [8A] , [8B], [8E] or [8F] : 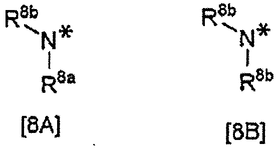 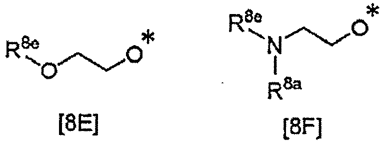
   wherein
      * is a binding position with L;
      R^{8a} is a hydrogen atom or C₁₋₆ alkyl;
      R^{8b} is each the same or different and is each a long-chain alkyl;
      R^{8e} is
         (1) a long-chain alkyl,
         (2) a long-chain alkyl-carbonyl, or
         (3) benzoyl substituted with one to five long-chain alkyloxy and/or long-chain alkenyloxy; and
   L is a group of the general formula [10]: 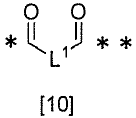 wherein
      * is a binding position with Z;
      ** is a binding position with T;
   L¹ is C₂₋₁₀ alkylene optionally substituted or C₆₋₁₀ arylene optionally substituted; or
      a group of the general formula [10-1]: 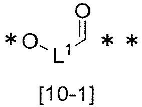
   wherein
      * is a binding position with Z;
      ** is a binding position with T;
      L¹ is C₁₋₁₀ alkylene optionally substituted or C₆₋₁₀ arylene optionally substituted;

   n is an integer from 1 to 50; and
   T is a single bond or a substituent group of the general formula [11], provided that T is a single bond when G is a silicon substituent group: 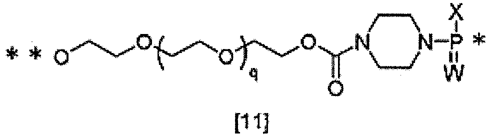
   wherein
      X and W are as defined above;
      * is a binding position with **O, *O or *N of the above formulae [4a] to [4d] ;
      ** is a binding position with G; and
      q is an integer from 0 to 10.

In one embodiment, the compound of the formula [C-1]: 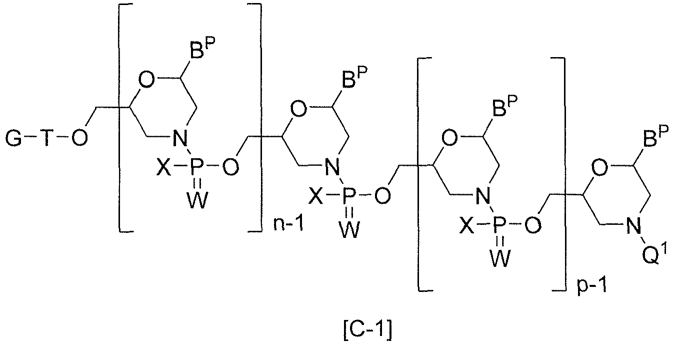
is a compound
wherein
   B^{P}, Q¹, W, X, n and p are as defined above, and
   G is selected from the group consisting of the formulae: 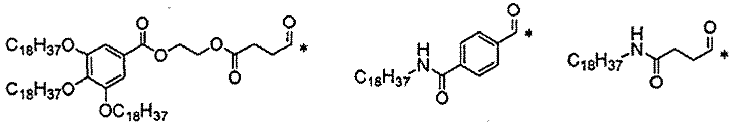 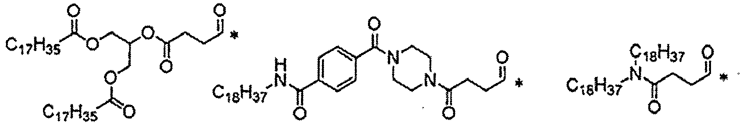 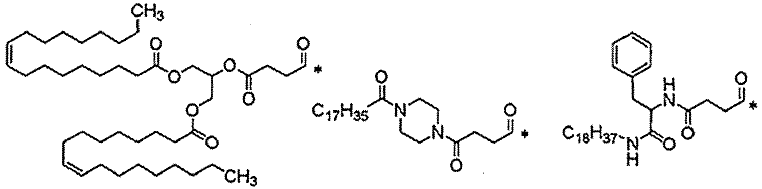 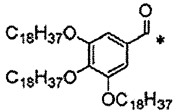
   wherein
      * is a binding position with T; and
      T is a single bond.

### <Condition for removing hydroxyl-protecting group>

In one embodiment of the invention, the conditions for removing the hydroxyl-protecting group can be determined depending on the type or property of the protecting group in the compound of the formula [C-1], and the compound of the formula [C-0-2] can be obtained by removing the hydroxyl-protecting group.

In one embodiment of the invention, the condition for removing the hydroxyl-protecting group is, for example, a condition as described in "Green's PROTECTING GROUPS in ORGANIC SYNTHESIS, 4th Edition, 2006".

In one embodiment of the invention, when G is (1) a silicon substituent group as defined above and T is a single bond, the condition for removing the hydroxyl-protecting group comprises, for example, treatment with tetrabutylammonium fluoride.

In one embodiment of the invention, when G is (2) C₁₋₁₈ alkyl-carbonyl optionally substituted, C₁₋₁₈ alkoxy-carbonyl optionally substituted, a long-chain alkyl-carbonyl or a long-chain alkoxy-carbonyl, or (3) benzoyl substituted with one to five long-chain alkyloxy and/or long-chain alkenyloxy, and T is a single bond, the condition for removing the hydroxyl-protecting group comprises, for example, treatment with sodium methoxide.

In one embodiment of the invention, when G is (4) a substituent group represented by the general formula [7]: 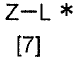 wherein
Z, L and * are as defined above, and
T is a single bond, the condition for removing the hydroxyl-protecting group comprises, for example, treatment with (1) ammonia water, (2) ammonia water/ethanol or (3) a mixed solution of ammonia water and methylamine.

### <Condition for removing hydroxyl-protecting group: Amount of reagents>

In one embodiment of the invention, the reagents may be used in an amount, for example, within the range of 1 to 200 molar equivalent, preferably 2 to 100 molar equivalent, and more preferably 2 to 50 molar equivalent, with respect to the compound of the formula [C-1].

### <Condition for removing hydroxyl-protecting group: Reaction solvent>

In one embodiment of the invention, the solvent used for removing the hydroxyl-protecting group is not limited so long as it is a solvent generally used in the art, and a single solvent may be used, or two or more solvents may be used in combination.

In one embodiment of the invention, examples of the solvent used for removing the hydroxyl-protecting group include aromatic solvents such as benzene, toluene, xylene, mesitylene and the like; ester solvents such as ethyl acetate, isopropyl acetate and the like; aliphatic solvents such as hexane, pentane, heptane, octane, nonane, cyclohexane and the like; and halogenated solvents. These solvents may be used in combination.

### <Condition for removing hydroxyl-protecting group: Reaction Temperature>

In one embodiment of the invention, the reaction temperature for removing the hydroxyl-protecting group is not limited and may be 0 to 80°C, preferably 10 to 60°C, and more preferably 20 to 40°C.

### <Condition for removing hydroxyl-protecting group: Reaction Time>

In one embodiment of the invention, the reaction time for removing the hydroxyl-protecting group is not limited and may be 10 minutes to 24 hours, preferably 10 minutes to 12 hours, and more preferably 30 minutes to 6 hours.

### <Final deprotection step, isolation step for nucleic acid compound>

In case where the compound [C-1] has a protecting group in its molecule, a treatment for deprotection, which is adopted depending on the type or properties of the protecting group, can be conducted to prepare a compound wherein all the protecting groups have been removed. For example, according to the method for deprotection as described in "Green's PROTECTING GROUPS in ORGANIC SYNTHESIS, 4th Edition, 2006", all the protecting groups in the compound can be removed. Specifically, for example, by conducting a treatment with (1) ammonia water, (2) ammonia water/ethanol, or (3) a mixed aqueous solution containing ammonia water and methylamine, the protecting groups on the substituent [6] and the amino group or the hydroxyl group of the nucleobase in the molecule of the compound [C-1] can be removed.

In addition, a removable group under an acidic condition on the amino-protecting group at the 3'-position of the 3'-terminal nucleoside of the compound [C-1] can be removed, for example, by conducting a treatment with the "acid" as described above in the section <Removal of intramolecular Q¹ of Compound [C-1]> or with a solution of hydrochloric acid or acetic acid diluted with a suitable solvent.

### Purification and isolation steps

The compound [C-1] wherein all the protecting groups have been removed can be isolated from a reaction mixture by conventional methods for isolation and purification, for example, extraction, concentration, neutralization, filtration, centrifugation, recrystallization, C8 to C18 reverse phase column chromatography, cation exchange column chromatography, anion exchange column chromatography, gel filtration column chromatography, high performance liquid chromatography, dialysis, and ultra-filtration, alone or in combination (see, for example, WO1991/09033A1).

When purifying the desired compound by reverse phase chromatography, for example, a mixed solution containing 20 mM triethylamine/acetate buffer and acetonitrile can be used as an elution solvent.

When purifying the desired compound by ion exchange chromatography, for example, a mixed solution of 1 M NaCl aqueous solution and 10 mM sodium hydroxide aqueous solution, or a solution of 0.3 M NaCl in 50 mM phosphate buffer can be used.

### EXAMPLES

Hereinafter, the present invention is described in more detail in the following Reference Examples, Comparative Examples, and Test Examples, but the present invention is not limited thereto. The compound names were generated using software (ChemDraw or BIOVIA Draw).

The term "conversion yield (%)" means the ratio at which a starting material is converted to an object product, and is calculated by {peak area (%) corresponding to object product detected by high performance liquid chromatography (hereinafter, referred to as "HPLC")} ÷ {peak area (%) corresponding to starting material detected by HPLC + peak area (%) corresponding to object product detected by HPLC} x 100.

### Conditions for HPLC:

0.5 mg of the product was dissolved in acetonitrile or 20% water/acetonitrile, and HPLC analysis was performed under the following conditions. The conversion efficiency was calculated by using the integral value of a peak area obtained by absorption at UV = 264 nm by HPLC.

### <ODS Condition 1>

Column: Waters XBridge C18 (2.5 *µ*m, 4.6 x 50 mm), 60°C
Detection wavelength: 264 nm
Mobile phase A: 20 mM AcONH₄ aq.
Mobile phase B: MeCN
Flow rate: 0.75 mL/min
Gradient: 40-95% B (0-15 min), 95% B (15-24 min), 40% B (24-30 min)

### <ODS Condition 2>

Column: Waters XBridge C8 (5 *µ*m, 4.6 x 75 mm), 60°C
Detection wavelength: 264 nm
Mobile phase A: 50 mM TEAA aq.
Mobile phase B: MeOH
Flow rate: 0.75 mL/min
Gradient: 70-95% B (0-20 min), 95% B (20-26 min), 75% B (26-35 min)

### <LC/MS Condition 1>

Equipment:
Ultra-high performance fluid chromatograph ACQUITY UPLC (Waters)
Quadrupole time-of-flight mass spectrometer SYNAPT-MS (Waters)
Column: ACQUITY UPLC BEH C18 1.7 *µ*m, 2.1 x 50 mm (Waters) Temperature: 50°C
Flow rate: 0.2 mL/min
Mobile phase: 10 mM ammonia water
Mobile phase: MeCN
Gradient: 50-95% B (4 min)
Detector 1: UV 264 nm
Detector 2: Quadrupole time-of-flight mass spectrometer Ionization method: ESI+
Measuring range: 100-2000 m/z

### <LC/MS Condition 2>

Equipment:
Ultra-high performance fluid chromatograph ACQUITY UPLC (Waters)
Quadrupole time-of-flight mass spectrometer SYNAPT-MS (Waters)
Column: ACQUITY UPLC BEH C18 1.7 gm, 2.1 x 50 mm (Waters) Temperature: 50°C
Flow rate: 0.2 mL/min
Mobile phase A: 10 mM ammonia water
Mobile phase B: MeOH
Gradient: 70-95% B (4 min)
Detector 1: UV 264 nm
Detector 2: Quadrupole time-of-flight mass spectrometer Ionization method: ESI+
Measuring range: 100-2000 m/z

### <LC/MS Condition 3>

Equipment:
Agilent 6100 series single quadrupole LC/MS system (Agilent)
Column: Waters XBridge C18 (3.5 um, 4.6 x 150 mm)
Temperature: 35°C
Flow rate: 1.0 mL/min
Mobile phase A: 20 mM ammonium acetate in water
Mobile phase B: MeCN
Gradient: 20-99% B (15 min)
Detector 1: UV 264 nm
Detector 2: Quadrupole time-of-flight mass spectrometer
Ionization method: ESI+
Measuring range: 250-3000 m/z

### Reference Example 1

[(2S,6R)-6-(5-methyl-2,4-dioxo-3,4-dihydropyrimidin-1(2H)-yl)morpholin-2-yl]methyl 4-(octadecylamino)-4-oxobutanoate (hereinafter referred to as "G1-suc-morT-OFF")

### Step 1: Preparation of 4-(octadecylamino)-4-oxobutanoic acid (hereinafter, referred to as "G1-suc")

Succinic anhydride (8.96 g, 1.1 eq.) and triethylamine (17 mL, 1.5 eq.) were added to a solution of octadecan-1-amine (21.94 g) in dichloromethane (500 mL), and the mixture was stirred at room temperature for 7 hours. The mixture was concentrated under reduced pressure, 150 mL of acetone was added to the residue, and the mixture was stirred for 16 hours. The precipitate was filtered under reduced pressure, washed with acetone (400 mL), and then dried under reduced pressure at 30°C for 3 hours to obtain G1-suc (29.1 g, 96.6%) as white powder.

### Step 2: Preparation of [(2S,6R)-6-(5-methyl-2,4-dioxo-3,4-dihydropyrimidin-1(2H)-yl)morpholin-2-yl]methyl 4-(octadecylamino)-4-oxobutanoate (hereinafter, referred to as "G1-suc-morT-OFF")

1-Ethyl-3-(3-dimethylaminopropyl)carbodiimide hydrochloride (8.56 g, 1.2 eq.) was added to a solution of G1-suc (14.4 g) in tetrahydrofuran (150 mL), and the mixture was stirred at room temperature. Then, 1-((2R,6S)-6-(hydroxymethyl)-4-tritylmorpholin-2-yl) -5-methylpyrimidine-2,4 (1H,3H)-dione (hereinafter, referred to as "morT-OH") (18 g, 1.0 eq.) and 4.57 g of 4-(N,N-dimethylamino)pyridine were added to the mixture, and the mixture was stirred at 70°C in a water bath for 1 hour. The mixture was cooled to room temperature, then a 0.1 M aqueous solution of sodium dihydrogen phosphate was added to the mixture, and the mixture was stirred for a while. Then, the aqueous layer was removed, and the organic layer was washed once with a 0.1 M aqueous solution of sodium dihydrogen phosphate and once with saturated brine diluted 2-fold with water. The aqueous layers were combined and extracted with dichloromethane, and the organic layers were combined and dried over anhydrous sodium sulfate. After filtration, the solvent was distilled off, and drying was performed under reduced pressure to obtain [(2S,6R)-6-(5-methyl-2,4-dioxo-3,4-dihydropyrimidin-1(2H)-yl)-4-tritylmorpholin-2-yl]methyl 4-(octadecylamino)-4-oxobutanoate (hereinafter referred to as "G1-suc-morT-ON") (white amorphous, 28.1 g, 89.5%). The product was dissolved in 140 mL of dichloromethane, and 20 mL of 2,2,2-trifluoroethanol and 10.3 mL of triisopropylsilane were added while stirring in an ice bath. Then, 5.1 mL of trifluoroacetic acid was added dropwise to the mixture. One hour after the completion of the dropping, the reaction solution was poured into a solution which had been prepared by adding ice to 100 mL of a saturated aqueous solution of sodium bicarbonate. After confirming that the pH of the aqueous layer was 7 to 8, the aqueous layer was extracted with dichloromethane. The organic layers were combined, dried over anhydrous sodium sulfate, filtered and concentrated. Column chromatography purification was performed with silica gel using a dichloromethane-methanol mixed solution as a mobile phase, and drying under reduced pressure to obtain 19.89 g of G1-suc-morT-OFF as a powder.

¹H-NMR (CDCl₃) : δ8.90 (1H, bs); 7.25 (1H, d, J = 1.6 Hz); 5.72 (1H, dd, J = 9.6 Hz, 2.6 Hz); 5.65 (1H, m); 4.14 (2H, d, J = 5.2 Hz); 3.98 (1H, m); 3.23 (2H, dd, J = 12.8 Hz, 7.0 Hz); 3.12 (2H, dd, J = 12 Hz, 2.6 Hz); 2.95 (2H, dd, J = 12.8 Hz, 1.8 Hz); 2.60-2.75 (4H, m); 2.47 (2H, t, J = 6.8 Hz); 1.95 (3H, d, J = 1.6 Hz); 1.48 (2H, m), 1.21-1.34 (29H, m); 0.88 (3H, t, J = 6.4 Hz)
ESI-MS (+): 593.36 (M+H) (LC/MS Condition 1)

### Reference Example 1-1

The following compounds were prepared in the same manner as described in Reference Example 1.
(1) <[(2S,6R)-6-(6-benzamido-9H-purin-9-yl)-4-tritylmorpholin-2-yl]methyl 4-(octadecylamino)-4-oxobutanoate> (G1-suc-morA-ON), <[(2S,6R)-6-(6-benzamido-9H-purin-9-yl)morpholin-2-yl]methyl 4-(octadecylamino)-4-oxobutanoate> (G1-suc-morA-OFF)
(2) <[(2S,6R)-6-(4-benzamino-2-oxopyrimidin-1(2H)-yl)-4-tritylmorpholin-2-yl]methyl 4-(octadecylamino)-4-oxobutanoate> (G1-suc-morC-ON), <[(2S,6R)-6-(4-benzamido-2-oxopyrimidin-1(2H)-yl)morpholin-2-yl]methyl 4-(octadecylamino)-4-oxobutanoate> (G1-suc-morC-OFF)
(3) <[(2S,6R)-6-(6-(2-cyanoethoxy)-2-(phenoxyacetamido)-9H-purin-9-yl)-4-tritylmorpholin-2-yl]methyl 4-(octadecylamino)-4-oxobutanoate> (G1-suc-morG-ON), <[(2S,6R)-6-(6-(2-cyanoethoxy)-2-(phenoxyacetamido)-9H-purin-9-yl)morpholin-2-yl]methyl 4-(octadecylamino)-4-oxobutanoate> (G1-suc-morG-OFF)

### Reference Example 2: {[(2S,6R)-6-(5-methyl-2,4-dioxo-3,4-dihydropyrimidin-1-yl)morpholin-2-yl]methyl}{2-octadecanoyloxy-1-[(octadecanoyloxymethyl)ethyl] }succinate (hereinafter, referred to as "G2-suc-morT-OFF")

### Step 1: Preparation of 4-((1,3-bis(stearoyloxy)propan-2-yl)oxy)-4-oxobutanoic acid (hereinafter, referred to as "G2-suc")

Dichloromethane (8 mL) was added to 1 g (1.60 mmol) of 2-hydroxypropan-1,3-diyl distearate, then 176 mg (1.76 mmol) of succinic anhydride and 293 mg (2.40 mmol) of 4-(N,N-dimethylamino)pyridine were added to the mixture, and the mixture was stirred at room temperature for 16 hours. After completion of the reaction, a 1 M aqueous solution of sodium dihydrogen phosphate was added to the reaction solution, the solution was extracted with dichloromethane, the extract was dried over sodium sulfate, and the solvent was distilled off to obtain G2-suc (1.40 g).

### Step 2: Preparation of {[(2S,6R)-6-(5-methyl-2,4-dioxo-3,4-dihydropyrimidin-1-yl)-4-tritylmorpholin-2-yl]methyl}{2-octadecanoyloxy-1-[(octadecanoyloxymethyl)ethyl]}succinate (hereinafter, referred to as "G2-suc-morT-ON")

Dichloromethane (5.2 mL) was added to G2-suc (900 mg, 1.24 mmol) and 277 mg (1.45 mmol) of 1-ethyl-3-(3-dimethylaminopropyl)carbodiimide hydrochloride, then 500 mg (1.03 mmol) of morT-OH and 132 mg (1.09 mmol) of 4-(N,N-dimethylamino)pyridine were added to the mixture, and the mixture was stirred at room temperature for 16 hours. After completion of the reaction, a 0.1 M aqueous solution of sodium dihydrogen phosphate was added to the reaction solution, the solution was extracted with dichloromethane, the extract was dried over sodium sulfate, and the solvent was distilled off. The obtained residue was purified by silica gel chromatography to obtain G2-suc-morT-ON (1.09 g, 89%) .

¹H-NMR (CDCl₃): δ8.04 (1H, s); 7.17-7.51 (15H, m); 6.98 (1H, s); 6.12 (1H, dd, J = 9.6 Hz, 2.4 Hz); 5.25 (1H, m); 4.34-4.37 (1H, m); 4.26-4.30 (2H, m); 4.11-4.16 (2H, m); 4.00-4.08 (2H, m); 3.35 (1H, d, J = 11.2 Hz); 3.10 (1H, d, J = 11.6 Hz); 2.60 (4H, s); 2.30 (4H, t, J = 7.6 Hz); 1.83 (3H, s); 1.38-1.44 (2H, m); 1.24 (60H, m); 0.87 (6H, t, J = 6.8 Hz)

### Step 3: Preparation of G2-suc-morT-OFF

Dichloromethane (4.2 mL) was added to G2-suc-morT-ON, and the mixture was stirred at 0°C. Then, 127 *µ*L (0.62 mmol) of triisopropylsilane and 64 *µ*L (0.82 mmol) of trifluoroacetic acid were added to the mixture at 0°C, and the mixture was stirred at room temperature for 1 hour. After completion of the reaction, a saturated aqueous solution of sodium bicarbonate was added to the reaction solution, the solution was extracted with dichloromethane, the extract was dried over sodium sulfate, and the solvent was distilled off. The obtained residue was purified by silica gel chromatography to obtain G2-suc-morT-OFF (373 mg, 95%).

¹H-NMR (CDCl₃): δ8.04 (1H, bs); 7.24 (1H, s); 5.70 (1H, d, J = 2 Hz); 5.21-5.26 (1H, m); 4.28-4.31 (2H, m); 4.13-4.17 (4H, m); 3.96-4.00 (1H, m); 3.11 (1H, dd, J = 12.4, 2 Hz); 2.94 (1H, dd, J = 12.8, 2.4 Hz); 2.57-2.65 (6H, m); 2.32 (4H, t, J = 7.6 Hz); 1.95 (3H, s); 1.25 (60H, m); 0.88 (6H, t, J = 7.6 Hz)

### Reference Example 3: 1,3-bis(oleoyloxy)propan-2-yl [{(2S,6R)-6-(5-methyl-2,4-dioxo-3,4-dihydropyrimidin-1(2H)-yl)morpholin-2-yl}methyl] succinate (hereinafter, referred to as "G3-suc-morT-OFF")

### Step 1: Preparation of 1,3-bis(oleoyloxy)propan-2-yl [{(2S,6R)-6-(5-methyl-2,4-dioxo-3,4-dihydropyrimidin-1(2H)-yl)-4-tritylmorpholin-2-yl}methyl]succinate (hereinafter, referred to as "G3-suc-morT-ON")

Using 2-hydroxypropan-1,3-diyl diolate as a starting material, 4-((1,3-bis(oleoyloxy)propan-2-yl)oxy)-4-oxobutanoic acid (hereinafter referred to as "G3-suc") was prepared in the same manner as Step 1 of Reference Example 2. Then, G3-suc-morT-ON was prepared in the same manner as Step 2 of Reference Example 2.

¹H-NMR (CDCl₃): δ8.00 (1H, s); 7.17-7.51 (15H, m); 6.99 (1H, s); 6.09-6.12 (1H, m); 5.29-5.38 (4H, m); 5.20-5.25 (1H, m); 4.33-4.37 (1H, m) ; 4.26-4.30 (2H, m) ; 4.12-4.16 (2H, m) ; 4.00-4.09 (2H, m); 3.35 (1H, d, J = 11.6 Hz); 2.15 (1H, d, J = 11.6 Hz); 2.60 (4H, m); 2.30 (4H, t, J = 7.2 Hz); 1.97-2.02 (8H, m); 1.83 (3H, s) ; 1.57-1.61 (2H, m); 1.28 (44H, m); 0.89 (6H, t, J = 6.8 Hz)

### Step 2: Preparation of G3-suc-morT-OFF

The compound was prepared in the same manner as Step 3 of Reference Example 2.

¹H-NMR (CDCl₃): δ7.97 (1H, bs); 7.24 (1H, s) ; 5.69-5.72 (1H, m); 5.29-5.38 (4H, m); 5.21-5.25 (1H, m); 4.27-4.31 (2H, m); 4.13-4.17 (4H, m); 3.97-3.99 (1H, m); 3.11 (1H, d, J = 12 Hz); 2.94 (1H, d, J = 13.2 Hz); 2.57-2.67 (4H, m) 2.31 (4H, t, J = 7.6 Hz); 1.99-2.00 (11H, m); 1.26-1.29 (46H, m); 0.87 (6H, t, J = 6.8 Hz)

### Reference Example 4: {(2S,6R)-6-(5-methyl-2,4-dioxo-3,4-dihydropyrimidin-1(2H)-yl)morpholin-2-yl}methyl 4-oxo-4-(4-stearoylpiperazin-1-yl)butanoate (hereinafter, referred to as "G4-suc-morT-OFF")

### Step 1: Preparation of 4-oxo-4-(4-stearoylpiperazin-1-yl)butanoic acid (hereinafter, referred to as "G4-suc")

26 mL of tetrahydrofuran was added to 1.68 g (5.91 mmol) of stearic acid, 1.13 g (5.91 mmol) of 1-ethyl-3-(3-dimethylaminopropyl)carbodiimide hydrochloride, and 0.79 g (5.91 mmol) of 1-hydroxybenzotriazole, then 1.45 mL (10.7 mmol) of triethylamine and 1 g (5.37 mmol) of piperazine-1-carboxylic acid tert-butyl were added to the mixture, and the mixture was stirred at room temperature for 16 hours. After completion of the reaction, a saturated aqueous solution of sodium bicarbonate was added to the reaction solution, the solution was extracted with dichloromethane, the extract was dried over sodium sulfate, and the solvent was distilled off. The obtained residue was purified by silica gel chromatography to obtain 4-stearoylpiperazine-1-carboxylic acid tert-butyl (1.64 g; 67%). 18 mL of dichloromethane was added thereto, the mixture was stirred at 0°C, 2.77 mL (36.2 mmol) of trifluoroacetic acid was added to the mixture at 0°C, and the mixture was stirred at room temperature for 2 hours. After completion of the reaction, a saturated aqueous solution of sodium bicarbonate was added to the reaction solution, the solution was extracted with dichloromethane, the extract was dried over sodium sulfate, and the solvent was distilled off to obtain 1-(piperazin-1-yl)octadecane-1-one (1.30 g). 18 mL of dichloromethane was added to 1.3 g (3.70 mmol) of the crude product, then 0.41 g (4.10 mmol) of succinic anhydride and 0.77 mL (5.50 mmol) of triethylamine were added to the mixture, and the mixture was stirred at room temperature for 2 hours. After completion of the reaction, the reaction solution was distilled off, acetone was added to the residue, and the residue was slurry washed at room temperature for 16 hours. The insoluble material was collected by filtration under reduced pressure, washed with acetone, and dried to obtain G4-suc (1.20 g).

### Step 2: Preparation of {(2S,6R)-6-(5-methyl-2,4-dioxo-3,4-dihydropyrimidin-1(2H)-yl)-4-tritylmorpholin-2-yl}methyl 4-oxo-4-(4-stearoylpiperazin-1-yl)butanoate (hereinafter, referred to as "G4-suc-morT-ON")

Tetrahydrofuran (10 mL) was added to G4-suc (982 mg, 2.17 mmol) and 555 mg (2.90 mmol) of 1-ethyl-3-(3-dimethylaminopropyl)carbodiimide hydrochloride, and the mixture was stirred at 70°C. Then, morT-OH (1 g, 2.07 mmol) and 265 mg (2.17 mmol) of 4-(N,N-dimethylamino)pyridine were added to the mixture, and the mixture was stirred at 70°C for 30 minutes. After completion of the reaction, the reaction solution was cooled to room temperature, a 0.1 M aqueous solution of sodium dihydrogen phosphate was added to the reaction solution, the solution was extracted with dichloromethane, the extract was dried over sodium sulfate, and the solvent was distilled off. The obtained residue was purified by silica gel chromatography to obtain G4-suc-morT-ON (1.68 g, 89%).

¹H-NMR (CDCl₃): δ8.00 (1H, s) ; 7.16-7.50 (15H, m); 6.97 (1H, s) ; 6.10 (1H, d, J = 8Hz) ; 4.34-4.36 (1H, m); 4.04 (2H, d, J = 4.8 Hz); 3.57-3.64 (4H, m); 3.44-3.48 (4H, m); 3.32-3.34 (1H, m); 3.09-3.12 (1H, m); 2.60-2.64 (4H, m); 2.31 (2H, t, J = 7.6 Hz); 1.82 (3H, s); 1.23-1.42 (32H, m); 0.86 (3H, t, J = 6.8)

### Step 3: Preparation of G4-suc-morT-OFF

The compound was prepared in the same manner as Step 3 of Reference Example 2.

¹H-NMR (CDCl₃): δ8.32 (1H, bs); 7.23 (1H, s) ; 5.67-5.70 (1H, m); 4.12-4.19 (2H, m); 3.96-4.01 (1H, m); 3.47-3.67 (8H, m); 3.10-3.13 (1H, m); 2.95-2.98 (1H, m); 2.60-2.72 (4H, m); 2.33 (2H, t, J = 7.2 Hz); 1.95 (3H, s); 1.25-1.31 (32H, m); 0.88 (3H, t, J = 7.6 Hz)

### Reference Example 5: [(2S,6R)-6-(5-methyl-2,4-dioxo-3,4-dihydropyrimidin-1(2H)-yl)morpholin-2-yl]methyl 4-(octadecylcarbamoyl)benzoate (hereinafter, referred to as "G5-tpa-morT-OFF")

### Step 1: Preparation of [(2S,6R)-6-(5-methyl-2,4-dioxo-3,4-dihydropyrimidin-1(2H)-yl)-4-tritylmorpholin-2-yl]methyl 4-(octadecylcarbamoyl)benzoate (hereinafter, referred to as "G5-tpa-morT-ON")

The compound was prepared in the same manner as Step 2 of Reference Example 2, using 4-(octadecylcarbamoyl)benzoic acid.

¹H-NMR (CDCl₃): δ8.24 (1H, s) ; 7.97 (2H, d, J = 8 Hz); 7.77 (2H, d, J = 8 Hz) ; 7.17-7.46 (15H, m); 6.95 (1H, s) ; 6.12-6.16 (1H, m); 4.49-4.51 (1H, m); 4.25-4.33 (2H, m); 3.42-3.47 (2H, m); 3.35-3.38 (1H, m) ; 3.21-3.24 (1H, m); 1.79 (3H, s); 1.23-1.44 (34H, m); 0.86 (3H, t, J = 6.8 Hz)

### Step 2: Preparation of G5-tpa-morT-OFF

The compound was prepared in the same manner as Step 3 of Reference Example 2.

¹H-NMR (CDCl₃) : δ8.24 (1H, bs) ; 8.11 (2H, d, J = 8.4 Hz); 7.84 (2H, d, J = 8.4 Hz) ; 7.24 (1H, s) ; 6.14-6.17 (1H, m) ; 5.74-5.77 (1H, m); 4.40-4.45 (2H, m); 4.13-4.19 (1H, m) ; 3.45-3.50 (2H, m); 3.14-3.18 (1H, m); 3.05-3.08 (1H, m) ; 1.93 (3H, s); 1.26-1.41 (34H, m); 0.89 (3H, t, J = 7.6 Hz)

### Reference Example 6: {(2S,6R)-6-(5-methyl-2,4-dioxo-3,4-dihydropyrimidin-1(2H)-yl)morpholin-2-yl}methyl 4-(4-(4-(octadecylcarbamoyl)benzoyl)piperazin-1-yl)-4-oxobutanoate (hereinafter referred to as "G6-suc-morT-OFF")

### Step 1: Preparation of 4-(4-(4-(octadecylcarbamoyl)benzoyl)piperazin-1-yl)-4-oxobutanoic acid (hereinafter referred to as "G6-suc")

The compound was prepared in the same manner as Step 1 of Reference Example 4, using 4-(octadecylcarbamoyl)benzoic acid instead of stearic acid.

### Step 2: Preparation of {(2S,6R)-6-(5-methyl-2,4-dioxo-3,4-dihydropyrimidin-1(2H)-yl)-4-tritylmorpholin-2-yl}methyl 4-[4-{4-(octadecylcarbamoyl)benzoyl}piperazin-1-yl] -4-oxobutanoate (hereinafter referred to as "G6-suc-morT-ON")

The compound was prepared in the same manner as Step 2 of Reference Example 2.

¹H-NMR (CDCl₃) : δ8.08 (1H, bs); 7.81 (2H, d, J = 7.6 Hz) ; 7.16-7.50 (17H, m); 6.97 (1H, s) ; 6.08-6.10 (1H, m); 4.33-4.39 (1H, m); 4.02-4.04 (2H, m); 3.31-3.79 (11H, m); 3.08-3.11 (1H, m); 2.60-2.69 (4H, m); 1.81 (3H, s) ; 1.23-1.44 (34H, m); 0.86 (3H, t, J = 6.4 Hz)

### Step 3: Preparation of G6-suc-morT-OFF

4.6 mL of dichloromethane and 0.4 mL of 2,2,2-trifluoroethanol were added to 493 mg (0.47 mmol) of G6-suc-morT-ON, and the mixture was stirred at 0°C. Then, 145 *µ*L (0.70 mmol) of triisopropylsilane and 53 *µ*L (0.70 mmol) of trifluoroacetic acid were added to the mixture at 0°C, and the mixture was stirred at room temperature for 1 hour. After completion of the reaction, a saturated aqueous solution of sodium bicarbonate was added to the reaction solution, the solution was extracted with dichloromethane, the extract was dried over sodium sulfate, and the solvent was distilled off. The obtained residue was purified by silica gel chromatography to obtain G6-suc-morT-OFF (372 mg; 98%).

¹H-NMR (CDCl₃): δ8.05 (1H, bs); 7.79 (2H, d, J = 7.6 Hz); 7.45 (2H, d, J = 7.6 Hz) ; 7.23 (1H, s) ; 6.08-6.11 (1H, m) ; 5.67-5.69 (1H, m); 4.10-4.15 (2H, m); 3.96-3.99 (1H, m); 3.36-3.79 (8H, m); 3.08-3.11 (1H, m); 2.93-2.96 (1H, m); 2.57-2.70 (6H, m); 1.92 (3H, s); 1.23-1.38 (34H, m); 0.86 (3H, t, J = 7.2 Hz)

### Reference Example 7: {(2S,6R)-6-(5-methyl-2,4-dioxo-3,4-dihydropirimidin-1(2H)-yl)morpholin-2-yl}methyl 3,4,5-tris(octadecyloxy)benzoate (hereinafter referred to as "G7-morT-OFF")

### Step 1: Preparation of { (2S,6R)-6-(5-methyl-2,4-dioxo-3,4-dihydropyrimidin-1(2H)-yl)-4-tritylmorpholin-2-yl}methyl 3,4,5-tris(octadecyloxy)benzoate (hereinafter referred to as "G7-morT-ON")

The compound was prepared in the same manner as Step 2 of Reference Example 2, using 3,4,5-trioctadecoxybenzoic acid.

¹H-NMR (CDCl₃): δ7.90 (1H, bs); 7.12-7.45 (17H, m); 6.97 (1H, s); 6.12-6.14 (1H, m); 4.46-4.51 (1H, m); 4.28-4.32 (1H, m); 4.16-4.20 (1H, m); 3.90-4.00 (6H, m); 3.37-3.40 (1H, m); 3.22-3.25 (1H, m); 1.78-1.82 (5H, m); 1.23-1.50 (96H, m); 0.86 (9H, t, J = 6.8 Hz)

### Step 2: Preparation of G7-morT-OFF

The compound was prepared in the same manner as Step 3 of Reference Example 2.

¹H-NMR (CDCl₃): δ7.98 (1H, bs); 7.22 (3H, m); 5.69-5.72 (1H, m); 4.32-4.36 (2H, m); 4.08-4.12 (1H, m); 3.94-4.01 (6H, m); 3.11-3.14 (1H, m); 3.02-3.05 (1H, m); 2.64-2.72 (2H, m); 1.90 (3H, m); 1.23-1.45 (96H, m); 0.86 (9H, t, J = 7.2 Hz)

### Reference Example 8: {(2S,6R)-6-(5-methyl-2,4-dioxo-3,4-dihydropyrimidin-1(2H)-yl)morpholin-2-yl} methyl (2-[{3,4,5-tris(octadecyloxy)benzoyloxy}oxy]ethyl) succinate (hereinafter referred to as "G8-suc-morT-OFF")

### Step 1: Preparation of 2-hydroxyethyl 3,4,5-trioctadecyloxybenzoate

8.1 mL of chloroform was added to 1.5 g (1.60 mmol) of 3,4,5-trioctadecyloxy benzoic acid, 370 mg (1.90 mmol) of 1-ethyl-3-(3-dimethylaminopropyl)carbodiimide hydrochloride, and 240 mg (1.90 mmol) of 4-(N,N-dimethylamino)pyridine, then 120 mg (1.90 mmol) of ethylene glycol was added to the mixture, and the mixture was stirred at room temperature for 3 hours. After completion of the reaction, a 1 M aqueous solution of sodium dihydrogen phosphate was added to the reaction solution, the solution was extracted with dichloromethane, the extract was dried over sodium sulfate, and the solvent was distilled off. The obtained residue was purified by silica gel chromatography to obtain 2-hydroxyethyl 3,4,5-trioctadecyloxybenzoate (882 mg; 56%).

### ¹H-NMR (CDCl₃): δ7.26 (2H, s) ; 4.45-4.47 (2H, m); 3.95-4.03 (8H, m); 1.25-1.52 (96H, m); 0.88 (9H, t, J = 7.2 Hz)

### Step 2: Preparation of {(2S,6R)-6-(5-methyl-2,4-dioxo-3,4-dihydropyrimidin-1(2H)-yl)-4-tritylmorpholin-2-yl}methyl (2-[{3,4,5-tris(octadecyloxy)benzoyloxy}oxy]ethyl)succinate (hereinafter referred to as "G8-suc-morT-ON")

4-Oxo-4-(2-[f3,4,5-tris(octadecyloxy)benzoyl}oxy]ethoxy)butanoic acid (hereinafter referred to as "G8-suc") was obtained in the same manner as Step 1 of Reference Example 2, and then G8-suc-morT-ON was obtained in the same manner as Step 2 of Reference Example 2.

### ¹H-NMR (CDCl₃): δ7.87 (1H, bs); 7.12-7.43 (17H, m); 6.97 (1H, s); 6.07-6.10 (1H, m); 4.33-4.46 (5H, m); 3.91-4.07 (8H, m); 3.31-3.34 (1H, m); 3.07-3.10 (1H, m); 2.56-2.60 (4H, m) ; 1.68-1.80 (5H, m); 1.23-1.50 (96H, m); 0.86 (9H, t, J = 7.2 Hz)

### Step 3: Preparation of G8-suc-morT-OFF

G8-suc-morT-OFF was obtained in the same manner as Step 3 of Reference Example 2.

¹H-NMR (CDCl₃): δ7.96 (1H, bs); 7, 23 (3H, m); 5.67-5.69 (1H, m); 4.40-4.47 (5H, m); 3.94-4.11 (8H, m); 3.09-3.12 (1H, m); 2.89-2.92 (1H, m); 2.53-2.65 (6H, m); 1.90 (3H, s); 1.23-1.45 (96H, m); 0.86 (9H, t, J = 6.8 Hz)

### Reference Example 9: {(2S,6R)-6-(5-methyl-2,4-dioxo-3,4-dihydropyrimidin-1(2H)-yl)morpholin-2-yl)methyl 4-(dioctadecylamino)-4-oxobutanate (hereinafter, referred to as "G9-suc-morT-OFF")

### Step 1: Preparation of {(2S,6R)-6-(5-methyl-2,4-dioxo-3,4-dihydropyrimidin-1(2H)-yl)-4-tritylmorpholin-2-yl}methyl 4-(dioctadecylamino)-4-oxobutanoate (hereinafter, referred to as "G9-suc-morT-ON")

4-(Dioctadecylamino)-4-oxobutanoic acid (hereinafter referred to as "G9-suc") was prepared in the same manner as Step 1 of Reference Example 2, using N-octadecane-1-amine as a starting material. Then, G9-suc-morT-ON was prepared in the same manner as Step 2 of Reference Example 2.

¹H-NMR (CDCl₃): δ7.88 (1H, bs); 7.17-7.43 (15H, m); 6.98 (1H, s); 6.06-6.09 (1H, m); 4.31-4.35 (1H, m); 4.01-4.03 (2H, m); 3.08-3.34 (8H, m); 2.52-2.64 (4H, m); 1.82 (3H, s); 1.23-1.52 (64H, m); 0.85 (6H, t, J = 6.8 Hz)

### Step 2: Preparation of G9-suc-morT-OFF

The compound was prepared in the same manner as Step 3 of Reference Example 2.

¹H-NMR (CDCl₃): δ8.14 (1H, bs); 7.27 (1H, s); 5.68-5.72 (1H, m): 4.12-4.20 (2H, m); 3.98-4.01 (1H, m); 3.10-3.29 (5H, m); 2.94-2.97 (1H, m); 2.60-2.70 (6H, m); 1.95 (3H, s); 1.25-1.49 (64H, m); 0.88 (6H, t, J = 7.2 Hz)

### Reference Example 10: {(2S,6R)-6-(5-methyl-2,4-dioxo-3,4-dihydropyrimidin-1(2H)-yl)morpholin-2-yl}methyl4-[{1-(octadecylamino)-1-oxo-3-phenylpropan-2-yl}amino] -4-oxobutanoate (hereinafter, referred to as "G10-suc-morT-OFF")

### Step 1: Preparation of {(2S,6R)-6-(5-methyl-2,4-dioxo-3,4-dihydropyrimidin-1(2H)-yl)-4-tritylmorpholin-2-yl}methyl 4-[{1-(octadecylamino)-1-oxo-3-phenylpropan-2-yl}amino]-4-oxobutanoate (hereinafter, referred to as "G10-suc-morT-ON")

9.4 mL of tetrahydrofuran was added to 500 mg (1.88 mmol) of 2-tert-butoxycarbonylamino-3-phenyl-propanoic acid, then 652 *µ*L (3.77 mmol) of N-ethyl-N-isopropyl-propane-2-amine, 46 mg (0.38 mmol) of 4-(N,N-dimethylamino)pyridine, and 505 mg (2.64 mmol) of 1-ethyl-3-(3-dimethylaminopropyl)carbodiimide hydrochloride and octadec-1-amine were added to the mixture, and the mixture was stirred at room temperature for 5 hours. After completion of the reaction, a 1 M aqueous solution of sodium dihydrogen phosphate was added to the reaction solution, the solution was extracted with dichloromethane, the extract was dried over sodium sulfate, and the solvent was distilled off. The obtained residue was purified by silica gel chromatography to obtain tert-butoxycarbonylamino-N-octadecyl-3-phenyl-propanamide (779 mg, 80%).

¹H-NMR (CDCl₃): δ7.18-7.28 (5H, m); 5.57 (1H, bs); 5.06 (1H, bs); 4.20-4.26 (1H, m); 2.95-3.12 (4H, m); 1.40 (9H, s); 1.14-1.28 (32H, m); 0.86 (3H, t, J = 6.8 Hz)

15 mL of dichloromethane was added to 779 mg (1.51 mmol) of tert-butoxycarbonylamino-N-octadecyl-3-phenyl-propanamide, then 1.74 mL (22.61 mmol) of trifluoroacetic acid was added to the mixture, and the mixture was stirred at room temperature for 3 hours. After completion of the reaction, the solvent was distilled off to obtain 2-amino-N-octadecyl-3-phenyl-propanamide (620 mg). The same reaction as in Step 1 of Reference Example 2 was carried out on the crude product to prepare G10-suc. Then, the same reaction as in Step 2 of Reference Example 2 was carried out to prepare G10-suc-morT-ON.

¹H-NMR (CDCl₃): δ8.08 (1H, bs); 7.16-7.32 (20H, m); 6.98 (1H, s); 6.29-6.31 (1H, m); 6.10-6.12 (1H, m); 5.56-5.59 (1H, m); 4.51-4.57 (1H, m); 4.35-4.37 (1H, m); 4.02 (2H, d, J = 5.6 Hz); 3.73-3.77 (1H, m); 2.92-3.33 (6H, m); 2.39-2.67 (4H, m); 1.84 (3H, s) ; 1.21-1.45 (32H, m); 0.88 (3H, t, J = 7.6 Hz)

### Step 2: Preparation of G10-suc-morT-OFF

The object product was obtained in the same manner as Step 3 of Reference Example 2.

¹H-NMR (CDCl₃): δ8.33 (1H, bs); 7.16-7.32 (6H, m); 6.38-6.40 (1H, m); 5.67-5.71 (2H, m); 4.54-4.58 (1H, m); 4.08-4.16 (3H, m); 3.94-4.01 (1H, m); 2.91-3.17 (5H, m); 2.46-2.79 (5H, m); 1.94 (3H, s); 1.13-1.36 (32H, m); 0.88 (3H, t, J = 7.6Hz)

The chemical formulae of the compounds described above in Reference Examples 1 to 10 are shown below in Table 3.

**[Table 3]**

| **Abbreviation** | **Chemical structure** |
|---|---|
| G1-suc-morT-OFF | 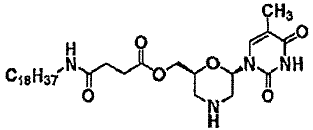 |
| G2-suc-morT-OFF | 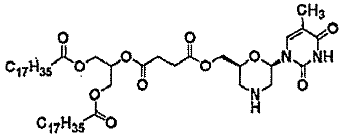 |
| G3-suc-morT-OFF | 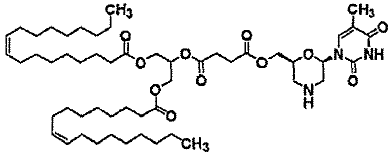 |
| G4-suc-morT-OFF | 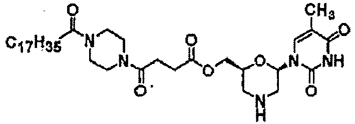 |
| G5-tpa-morT-OFF | 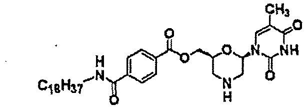 |
| G6-suc-morT-OFF | 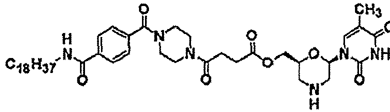 |
| G7-morT-OFF | 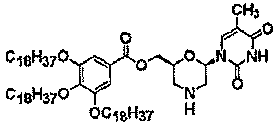 |
| G8-suc-morT-OFF | 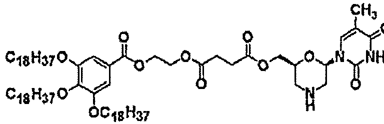 |
| G9-suc-morT-OFF | 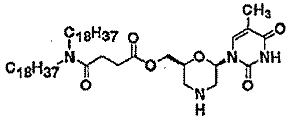 |
| G10-suc-morT-OFF | 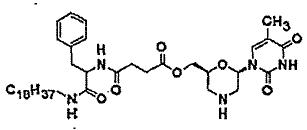 |

### Example 1: Preparation of H-phosphonate monomer

### Example 1-1: Preparation of triethylammonium [(2S,6R)-6-(6-benzamido-9H-purin-9-yl)-4-tritylmorpholin-2-yl]methylphosphonate (H-phosphonate-PMO [A^{Bz}] -ON (triethylamine salt)) and diazabicycloundecenium [(2S,6R)-6-(6-benzamido-9H-purin-9-yl)-4-tritylmorpholin-2-yl]methylphosphonate (H-phosphonate-PMO[A^{Bz}] -ON (DBU salt))

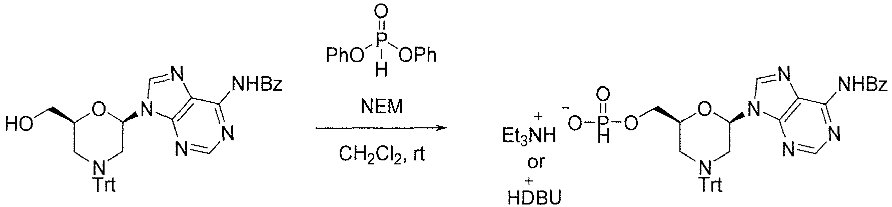

N-(9-((2R,6S) -6-(hydroxymethyl)-4-tritylmorpholin-2-yl)-9H-purin-6-yl)benzamide 600 mg (1.06 mmol) and 1.3 mL (10.06 mmol) of N-ethylmorpholine were dissolved in 10 mL of dichloromethane. To this solution, 481 µL (2.51 mmol) of diphenyl phosphite was added and stirred at room temperature for 2 hours. After 2 hours, 100 mL of 1 M triethylammonium bicarbonate solution was added and stirred at room temperature. After 1 hour, the organic layer was separated, dried with sodium sulfate, and the solvent was removed. The resulting residue was purified by silica gel chromatography to afford H-phosphonate-PMO[A^{Bz}]-ON (triethylamine salt) (720 mg, 94%).
ESI-MS (+): 660.2 (LC/MS condition 1)
¹H-NMR (CDCl₃): 9.02 (brs, 1H); 8.80 (s, 1H); 8.01 (s, 2H) ; 7.99 (d, J=1.4Hz, 1H); 7.57-7.62 (m, 2H); 7.49-7.53 (m, 7H); 7.30 (d, J=7.3, 7.3Hz, 6H); 7.19 (d, J=7.3, 7.3Hz, 3H); 6.4 0 (dd, J=9.6, 2.3Hz, 1H); 4.43-4.49 (m, 1H); 3.81-3.95 (m, 2 H); 3.50 (ddd, J=11.0, 2.3, 2.3Hz, 1H); 3.27 (ddd, J=11.9, 2 .3, 2.3Hz, 1H); 2.95 (q, J=7.3Hz, 7.2H); 1.81 (dd, J=10.0, 1 0.0Hz, 1H); 1.61 (dd, J=11.0, 11.0Hz, 1H); 1.23 (t, J=7.3Hz, 10.8H)
   500 mg of the obtained H-phosphonate-PMO[A^{Bz}]-ON (triethylamine salt) was dissolved in 5 mL of chloroform, 20 mL of 0.2 M diazabicycloundecene bicarbonate solution was added, and stirred at room temperature for 10 min. After 10 minutes, the organic layer was separated, dried over sodium sulfate, and the solvent was removed to afford 490 mg of H-phosphonate-PMO [A^{Bz}] -ON (DBU salt).
¹H-NMR (CDCl₃):9.01 (brs, 1H); 8.80 (s, 1H); 8.61-8.63 (m, 1 H); 8.04 (s, 1H); 8.00 (d, J=7.3Hz, 2H); 7.70 (dddd, J=7.5, 7.5, 1.8, 1.8Hz, 1H); 7.58-7.61 (m, 2H); 7.49-7.53 (m, 6H); 7.27-7.32 (m, 6H); 7.18 (d, J=7.3Hz, 3H); 6.40 (dd, J=9.6, 2.3Hz, 1H); 4.44-4.49 (m, 1H); 3.82-3.97 (m, 2H); 3.49 (ddd, J=11.0, 2.3, 2.3Hz, 1H); 3.34-3.43 (m, 7H); 3.28 (ddd, J=1 1.9, 2.3, 2.3Hz, 1H); 2.80 (d, J=3.2Hz, 2H); 1.93 (dddd, J= 6.0, 6.0, 6.0, 6.0Hz, 2H); 1.79 (dd, J=10.5, 10.5Hz, 1H); 1. 59-1.70 (m, 10H)

### Example 1-2:

In the same manner as Example 1-1, triethylammonium [(2S,6R)-6-(4-benzamido-2-oxopyrimidin-1-yl)-4-tritylmorpholin-2-yl] methylphosphonate (H-phosphonate-PMO[C^{Bz}]-ON), and triethylammonium [(2S,6R)-6-(5-methyl-2,4-dioxo-3,4-dihydropyrimidin-1(2H)-yl)-4-tritylmorpholin-2-yl]methylphosphonate (H-phosphonate-PMO[T]-ON) were prepared.

### Example 2: Preparation of H-phosphonate oligomer

### Example 2-1: Preparation of H-Phosphonate-PMO [C^{Bz}-A^{Bz}] -ON

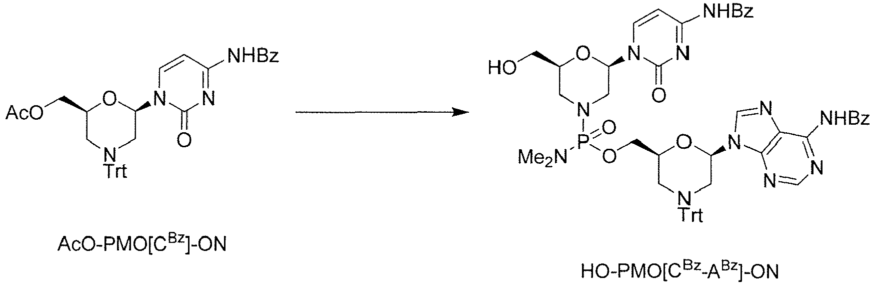

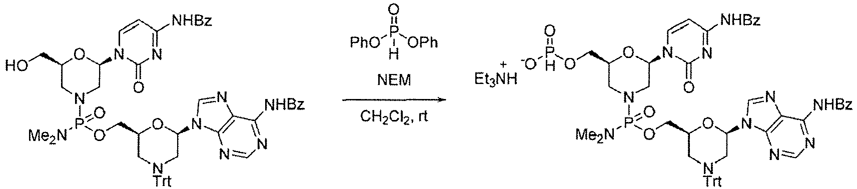

HO-PMO [C^{Bz}-A^{Bz}] -ON was obtained from AcO-PMO [C^{Bz}] -ON by known methods. AcO-PMO [C^{Bz}-A^{Bz}] -ON (3.9 g, 3.7 mmol) was suspended in 50 mL of tetrahydrofuran. To this solution, 148 µL (0.739 mmol) of 5 M sodium methoxide/methanol solution was added 10 times every 5 minutes, followed by 740 µL (3.70 mmol) of 5 M sodium methoxide/methanol solution (total 11.09 mmol). 5 minutes after the final addition, acetic acid was added and washed with brine and saturated sodium bicarbonate. The organic layer was dried with sodium sulfate, and the solvent was removed. The resulting residue was dissolved in dichloromethane, and diisopropyl ether was added to reprecipitate and obtain HO-PMO [C^{Bz}-A^{Bz}] -ON (3.67 g, 96%) . 1.5 g of HO-PMO[C^{Bz}-A^{Bz}]-ON (1.5 mmol) and 1.9 mL (15 mmol) of N-ethylmorpholine were dissolved in 15 mL of dichloromethane. To this solution was added 1.1 mL (5.9 mmol) of diphenyl phosphite and stirred at 40°C overnight. After the stirring overnight, 100 mL of 1 M triethylammonium bicarbonate solution was added and stirred at room temperature. After 1 hour, the organic layer was separated, dried with sodium sulfate, and the solvent was removed. The resulting residue was purified by silica gel chromatography to afford H-phosphonate-PMO[C^{Bz}-A^{Bz})-ON (triethylamine salt) (1.65 g, 95%).
ESI-MS (+): 1079.4 (LC/MS condition 1)

### Example 2-2: Preparation of H-Phosphonate-PMO [C^{Bz}-A^{Bz}-T-C^{Bz}-T] -ON

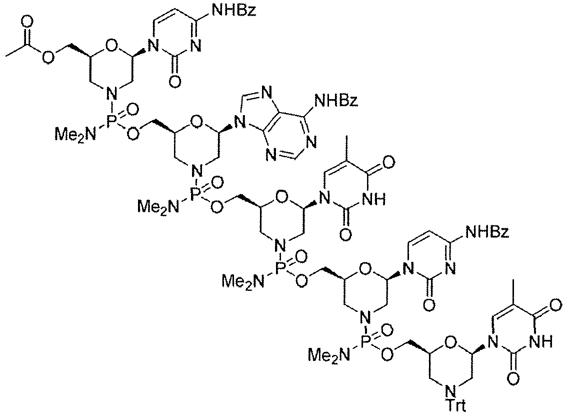

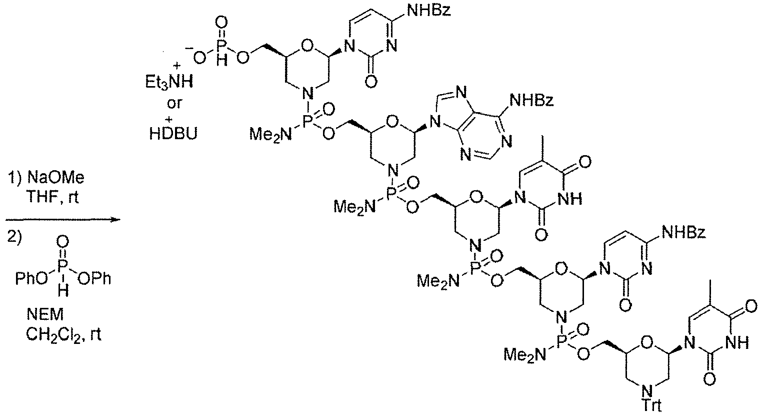

AcO-PMO [C^{Bz}-A^{Bz}-T-C^{Bz}-T] -ON was obtained from AcO-PMO [C^{Bz}] -ON by known method. AcO-PMO [C^{Bz}-A^{Bz}-T-C^{Bz}-T] -ON (4 g, 1.88 mmol) was suspended in 38 mL of tetrahydrofuran. To this solution, 450 µL (2.26 mmol) of 5 M sodium methoxide/methanol solution was added six times every 30 minutes (total 13.56 mmol). 30 minutes after the final addition, acetic acid was added, and washed with brine. The organic layer was dried with sodium sulfate, and the solvent was removed. The resulting residue was dissolved in dichloromethane, diisopropyl ether was added, and reprecipitated to afford HO-PMO [C^{Bz}-A^{Bz}-T-C^{Bz}-T] -ON (3.92 g, 99%) .

6.2 g of HO-PMO [C^{Bz}-A^{Bz}-T-C^{Bz}-T] -ON (3.0 mmol) and 7.4 mL (60 mmol) of N-ethylmorpholine were dissolved in 30 mL of dichloromethane. To this solution was added 4.6 mL (24 mmol) of diphenyl phosphite and stirred at 40°C overnight. After the stirring overnight, 300 mL of 1 M triethylammonium bicarbonate solution was added and stirred at room temperature. After 1 hour, the organic layer was separated, dried with sodium sulfate, and the solvent was removed. The resulting residue was purified by silica gel chromatography to afford H-phosphonate-PMO [C^{Bz}-A^{Bz}-T-C^{Bz}-T] -ON (triethylamine salt)(6.56 g, 98%).

1 g of the resulting product was dissolved in 5 mL of chloroform, and 20 mL of 0.2 M diazabicycloundecene bicarbonate solution was added and stirred at room temperature for 10 minutes. After 10 minutes, the organic layer was separated, dried over sodium sulfate, and the solvent was removed to afford 960 mg of H-phosphonate-PMO [C^{Bz}-A^{Bz}-T-C^{Bz}-T] -ON (DBU salt).
ESI-MS (+): 2158.7 (LC/MS condition 2)

### Example 2-3: Preparation of H-phosphonate-PMO [C^{Bz}-G^{CE,Pac}-A^{Bz}-A^{Bz}-G^{CE,Pac}] -ON

TBDPSO-PMO [C^{Bz}-G^{CE,Pac}-A^{Bz}-A^{Bz}-G^{CE,Pac}] -ON was obtained from TBDPSO-PMO [C^{Bz}] -ON by known method. TBDPSO-PMO[C^{Bz}-G^{CE,Pac}-A^{Bz}-A^{Bz}-G^{CE,Pac}] -ON (19 g, 7.07 mmol) and 3.0 mL (53.0 mmol) of acetic acid were suspended in 140 mL of tetrahydrofuran. To this solution was added 14.1 g (35.4 mmol) of tetrabutylammonium fluoride trihydrate and stirred at room temperature. After 1 hour, water was added, extracted with dichloromethane, dried with sodium sulfate, and the solvent was removed. The resulting residue was purified by silica gel chromatography to afford HO-PMO[C^{Bz}-G^{CE,Pac}-A^{Bz}-A^{Bz}-G^{CE,Pac}] -ON (10.7 g, 62%). HO-PMO [C^{Bz}-G^{CE,Pac}-A^{Bz}-A^{Bz}-G^{CE,Pac}] -ON (1 g, 0.408 mmol) and 1.0 mL (8.15 mmol) of N-ethylmorpholine were dissolved in 4.0 mL of dichloromethane. To this solution, 937 µL (4.89 mmol) of diphenyl phosphite was added and stirred at room temperature. After 1 hour, 40 mL of 1 M triethylammonium bicarbonate solution was added, and the mixture was stirred at room temperature. After 10 minutes, the organic layer was separated, dried with sodium sulfate, and the solvent was removed. The resulting residue was purified by silica gel chromatography to afford H-phosphonate-PMO [C^{Bz}-G^{CE,Pac}-A^{Bz}-A^{Bz}-G^{CE,Pac}] -ON (triethylamine salt) (870 mg, 85%). 500 mg of the resulting product was dissolved in 5 mL of chloroform, and 20 mL of 0.2 M diazabicycloundecene bicarbonate solution was added and stirred at room temperature. After 10 minutes, the organic layer was separated, dried over sodium sulfate, and the solvent was removed to afford 490 mg of H-phosphonate-PMO [C^{Bz}-G^{CE,Pac}-A^{Bz}-A^{Bz}-G^{CE,Pac}] -ON (DBU salt).
ESI-MS (+): 2606.9 (LC/MS condition 2)

### Example 2-4: Preparation of H-phosphonate-PMO [G^{CE,Pac}-G^{CE,Pac}-T-T-C^{Bz}] -ON

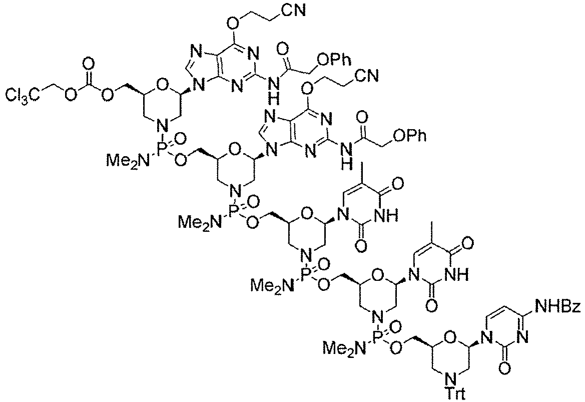

### TrocO-PMO[G^{CE,Pac}-G^{CE,Pac-}T-T-C^{Bz}]-ON

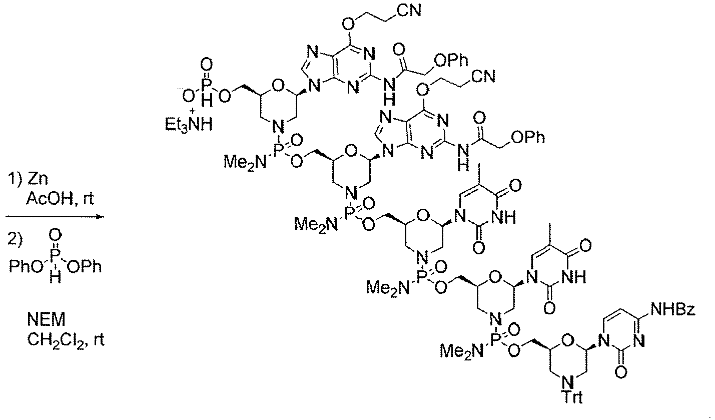

TrocO-PMO [G^{CE,Pac}-G^{CE,Pac}-T-T-C^{Bz}] -ON (1 g, 0.40 mmol) was dissolved in 6.0 mL of tetrahydrofuran. To this solution, 230 µL (4.01 mmol) of acetic acid and 262 mg (4.01 mmol) of zinc powder were added and stirred at room temperature. After 1 hour, 115 µL (2.01 mmol) of acetic acid and 131 mg (4.01 mmol) of zinc powder were added and stirred for 1 hour. After 1 hour, the reaction solution was filtered through Celite, and the solvent was removed from the filtrate. The resulting residue was purified by reversed-phase purification to afford HO-PMO [G^{CE,Pac}-G^{CE,Pac}-T-T-G^{Bz}] -ON (591.16 mg, 64%).

HO-PMO [G^{CE,Pac}-G^{CE,Pac}-T-T-C^{Bz}] -ON (441 mg, 0.19 mmol) and 241 µL (1.90 mmol) of N-ethylmorpholine were dissolved in 1.9 mL of dichloromethane. To this solution, 145 µL (0.76 mmol) of diphenyl phosphite was added and stirred at 40°C for 1 hour. After 1 hour, 398 µL of triethylamine was added. The reaction mixture was then dropped into 4.5 mL of diisopropyl ether containing 2% triethylamine. White precipitate that formed after the end of the drop was filtered by suction and dried under reduced pressure. The resulting white powder was purified by reversed-phase purification to afford H-phosphonate-PMO [G^{CE, Pac}-G^{CE,Pac}-T-T-C^{Bz}] -ON (211 mg, 45%).
ESI-MS (+): 2382.8 (M+H) (LC/MS condition 3)

### Example 2-5:

The H-phosphonates shown below were prepared in the same manner.

**[Table 4]**

| H-phosphonates | ESI-MS(+) (LC/MS condition 3) |
|---|---|
| C^{Bz}-C^{Bz} | 1056.4 (M+H) |
| T-T | 900.3 (M+Na) |
| T-T-T | 1208.4 (M+H) |
| T-T-T-T-T | 1868.6 (M+H) |

### Example 3: PMO Oligomer Preparation

### Example 3-1: Preparation of G1-suc-PMO[C^{Bz}-A^{Bz}]-ON

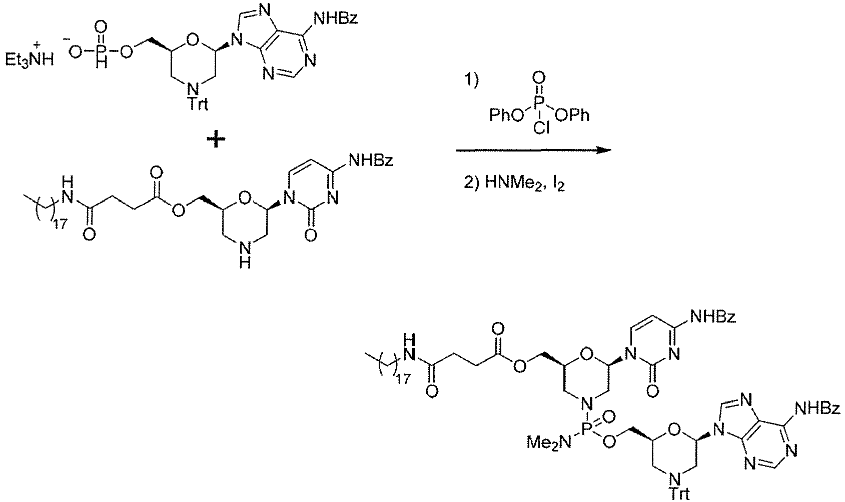

G1-suc-PMO [C^{Bz}] -OFF (17 mg, 0.025 mmol) and H-phosphonate-PMO [A^{Bz}] -ON (triethylamine salt) (29 mg, 0.0375 mmol) were dissolved in 500 µL of 20% pyridine/dichloromethane. To this solution was added 12 µL (0.0563 mmol) of diphenyl chlorophosphate, and the solution was stirred at room temperature. After 10 minutes, 100 µL of the reaction was mixed with 100 µL of an oxidizing solution (0.1 M iodine, 2 M dimethylamine/THF solution) and stirred for 10 minutes at room temperature. After completion of the reaction, a solution of 10% sodium thiosulfate in water was added to the reaction mixture, and the organic and aqueous layers were separated. The organic layer was diluted 20-fold with a solution of 80% acetonitrile in water and analyzed by HPLC (starting material Rt: 11.4 min, object product Rt: 17.2 min, conversion yield 92.1%, ODS Condition 1).
ESI-MS (+): 1367.8 (M+H) (LC/MS condition 1)

### Example 3-2: Preparation of G1-suc-PMO[C^{Bz}-A^{Bz}]-ON

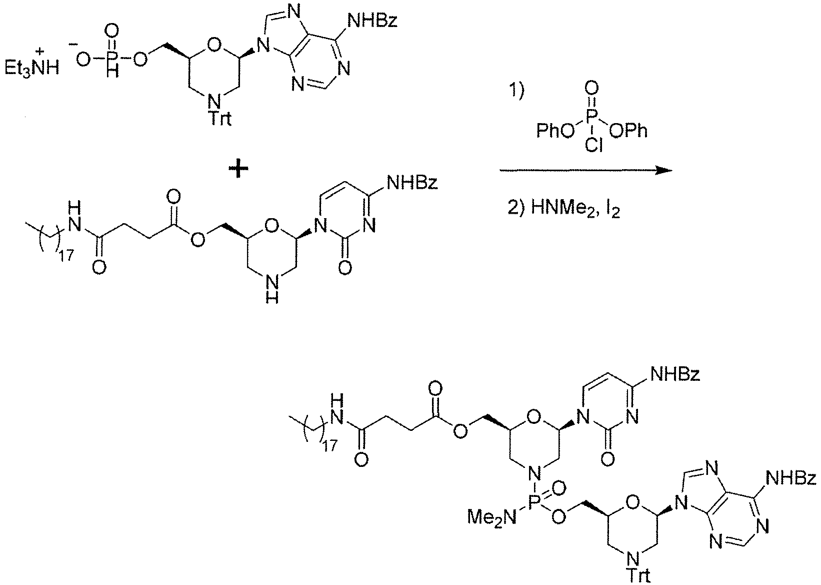

After mixing 2 mL of Liquid a (flow rate: 0.25 mL/min) with 2 mL of Liquid b (flow rate: 0.25 mL/min), the reaction was carried out in a 5 mL tube reactor at 60°C for 10 minutes. 100 µL of the resulting solution was mixed with 100 µL of an oxidizing solution (0.1 M iodine, 2 M dimethylamine/THF solution) and stirred for 10 minutes at room temperature. After completion of the reaction, a solution of 10% sodium thiosulfate in water was added to the reaction mixture, and the organic and aqueous layers were separated. The organic layer was diluted 20-fold with a solution of 80% acetonitrile in water and analyzed by HPLC (starting material Rt: 14.44 min, object product Rt: 17.28 min, conversion yield 95.6%, ODS Condition 1).
Liquid a: 82 µL (0.394 mmol) of diphenyl chlorophosphate was dissolved in 2.5 mL of anhydrous dichloromethane.
Liquid b: 200 mg (0.263 mmol) of H-phosphonate-PMO [A^{Bz}] -ON and G1-suc-PMO[C^{Bz}]-OFF (119 mg, 0.175 mmol) were dissolved in 2.5 mL of a 40% anhydrous pyridine/60% anhydrous dichloromethane solution.

### Example 3-3: Preparation of AcO-PMO [C^{Bz}-C^{Bz}] -ON

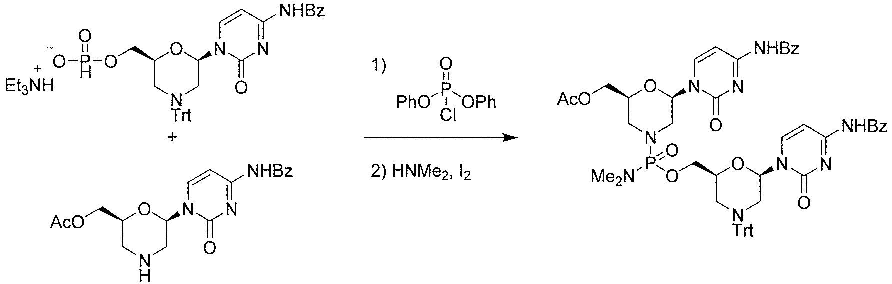

AcO-PMO [C^{Bz}] -OFF (9 mg, 0.025 mmol) and H-phosphonate-PMO [C^{Bz}] -ON (triethylamine salt) (29 mg, 0.0375 mmol) were dissolved in 500 µL of 20% pyridine/dichloromethane. To this solution was added 12 µL (0.0563 mmol) of diphenyl chlorophosphate, and the solution was stirred at room temperature. After 10 minutes, 100 µL of the solution was mixed with 100 µL of an oxidizing solution (0.1 M iodine, 2 M dimethylamine/THF solution) and stirred for 5 minutes at room temperature. After completion of the reaction, a solution of 10% sodium thiosulfate in water was added to the reaction mixture, and the organic and aqueous layers were separated. The organic layer was diluted 20-fold with a solution of 80% acetonitrile in water and analyzed by HPLC (starting material Rt: 5.3 min, object product Rt: 11.7, 11.8 min, conversion yield 99.7%, ODS Condition 1).
ESI-MS (+): 1056.2 (M + Na) (LC/MS condition 1)

### Example 3-4: Preparation of TBSO-PMO [C^{Bz}-C^{Bz}] -ON

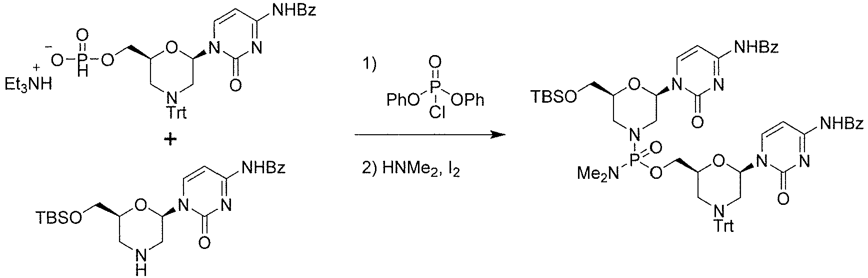

TBSO-PMO [C^{Bz}] -OFF (11 mg, 0.025 mmol) and H-phosphonate-PMO[C^{Bz}]-ON (triethylamine salt) (29 mg, 0.0375 mmol) were dissolved in 500 µL of 20% pyridine/dichloromethane. To this solution was added 12 µL (0.0563 mmol) of diphenyl chlorophosphate, and the solution was stirred at room temperature. After 10 minutes, 100 µL of the solution was mixed with 100 µL of an oxidizing solution (0.1 M iodine, 2 M dimethylamine/THF solution) and stirred for 5 minutes at room temperature. After completion of the reaction, a solution of 10% sodium thiosulfate in water was added to the reaction mixture, and the organic and aqueous layers were separated. The organic layer was diluted 20-fold with a solution of 80% acetonitrile in water and analyzed by HPLC (starting material Rt: 10.7 min, object product Rt: 14.9, 15.0 min, conversion yield 97.7%, ODS Condition 1). ESI-MS (+): 1106.4 (M+H) (LC/MS condition 1)

### Example 3-5: Preparation of G1-suc-PMO [T-G^{CE,Pac}-A^{Bz}] -ON

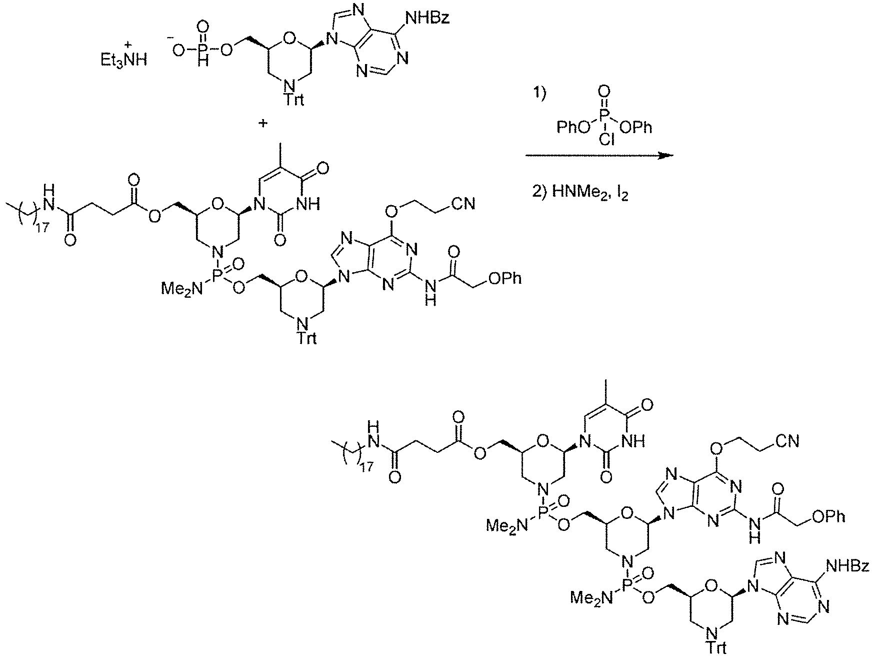

G1-suc-PMO[T-G^{CE,Pac}] -OFF (17 mg, 0.0123 mmol) and H-phosphonate-PMO[A^{Bz}]-ON (triethylamine salt) (14 mg, 0.0185 mmol) and 20 µL (0.247 mmol) of N-methylimidazole were dissolved in 250 µL of dichloromethane. To this solution was added 25 µL (0.123 mmol) of diphenyl chlorophosphate, and the mixture was stirred at 40°C. After 10 minutes, 100 µL of the solution was mixed with 100 µL of an oxidizing solution (0.1 M iodine, 2 M dimethylamine/THF solution) and stirred for 10 minutes at room temperature. After completion of the reaction, a solution of 10% sodium thiosulfate in water was added to the reaction mixture, and the organic and aqueous layers were separated. The organic layer was diluted 20-fold with a solution of 80% acetonitrile in water and analyzed by HPLC (starting material Rt: 12.9 min, object product Rt: 18.4, 19.1 min, conversion yield 89.5%, ODS Condition 2). ESI-MS (+): 1819.9 (LC/MS condition 2)

### Example 3-6: Preparation of G1-suc-PMO [T-G^{CE,Pac}-G^{CE,Pac}-A^{Bz}] -ON

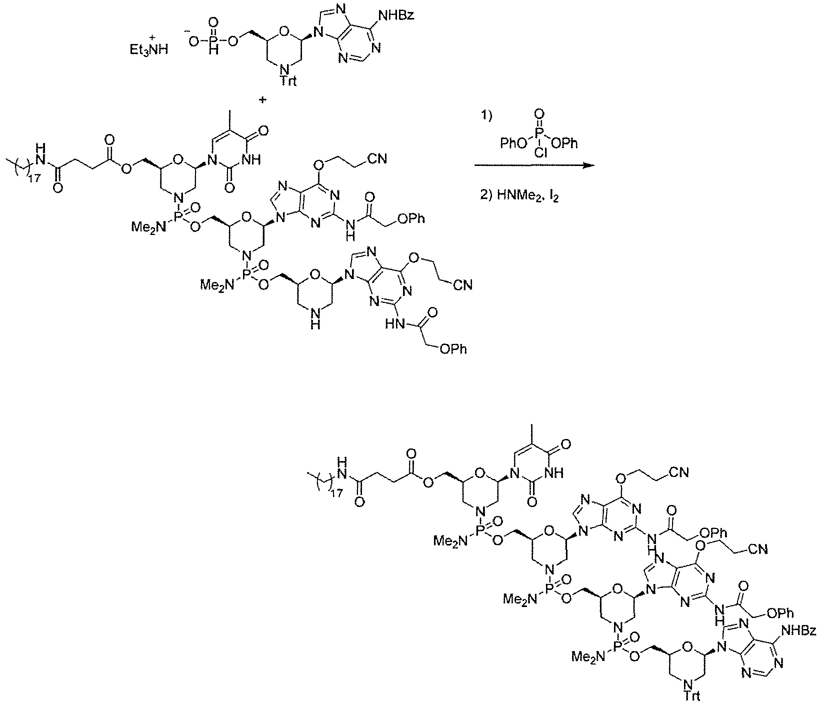

G1-suc-PMO [T-G^{CE,Pac}-G^{CE,Pac}] -OFF (21 mg, 0.0125 mmol) and H-phosphonate-PMO [A^{Bz}] -ON (triethylamine salt) (14 mg, 0.0188 mmol) and 20 µL (0.250 mmol) of N-methylimidazole were dissolved in 250 µL of dichloromethane. To this solution was added 25 µL (0.125 mmol) of diphenyl chlorophosphate, and the mixture was stirred at 40°C. After 10 minutes, 100 µL of the solution was mixed with 100 µL of an oxidizing solution (0.1 M iodine, 2 M dimethylamine/THF solution) and stirred for 10 minutes at room temperature. After completion of the reaction, a solution of 10% sodium thiosulfate in water was added to the reaction mixture, and the organic and aqueous layers were separated. The organic layer was diluted 20-fold with a solution of 80% acetonitrile in water and analyzed by HPLC (starting material Rt: 13.6 min, object product Rt: 19.5 min, conversion yield 91.6%, ODS Condition 2) .
ESI-MS (+): 2362.0 (LC/MS condition 2)

### Example 3-7: Preparation of G1-suc-PMO [T-G^{CE,Pac}-G^{CE,Pac}-G^{CE,Pac}-A^{Bz}-A^{Bz}] -ON

G1-suc-PMO [T-G^{CE,Pac}-G^{CE,Pac}-G^{CE,Pac}-A^{Bz}] -OFF (67 mg, 0.0251 mmol) and H-phosphonate-PMO [A^{Bz}] -ON (triethylamine salt) (29 mg, 0.0377 mmol) and 10 µL (0.126 mmol) of N-methylimidazole were dissolved in 250 µL of dichloromethane. To this solution was added 12 µL (0.0566 mmol) of diphenyl chlorophosphate, followed by stirring at 40°C. After 10 minutes, 100 µL of the solution was mixed with 100 µL of an oxidizing solution (0.1 M iodine, 2 M dimethylamine/THF solution) and stirred for 10 minutes at room temperature. After completion of the reaction, a solution of 10% sodium thiosulfate in water was added to the reaction mixture, and the organic and aqueous layers were separated. The organic layer was diluted 20-fold with a solution of 80% acetonitrile in water and analyzed by HPLC (starting material Rt: 14.4 min, object product Rt: 19.0, 19.2 min, conversion yield 97.9%, ODS Condition 2).
ESI-MS (+): 3347.4 (LC/MS condition 2)

### Example 3-8: Preparation of G1-suc-PMO[C^{Bz}-C^{Bz}-T-C^{Bz-}C^{Bz}-G^{CE,Pac}-G^{CE,pac}-T-T-C^{Bz}A^{Bz}] -ON (using diphenyl chlorophosphate as a condensation agent)

G1-suc-PMO [C^{Bz}-C^{Bz}-T-C^{Bz}-C^{Bz}-G^{CE,Pac}-G^{CE,Pac}-T-T-C^{Bz}] -OFF (28 mg, 0.00631 mmol) and H-phosphonate-PMO [A^{Bz}] -ON (triethylamine salt) (9.5 mg, 0.0126 mmol) and 10 µL (0.126 mmol) of N-methylimidazole were dissolved in 250 µL of dichloromethane. To this solution was added 13 µL (0.0631 mmol) of diphenyl chlorophosphate, followed by stirring at 40°C. After 10 minutes, 100 µL of the solution was mixed with 100 µL of an oxidizing solution (0.1 M iodine, 2 M dimethylamine/THF solution) and stirred for 5 minutes at room temperature. After completion of the reaction, a solution of 10% sodium thiosulfate in water was added to the reaction mixture, and the organic and aqueous layers were separated. The organic layer was diluted 20-fold with a solution of 80% acetonitrile in water and analyzed by HPLC (starting material Rt: 16.1 min, object product Rt: 19.5 min, conversion yield 94.4%, ODS Condition 2).
ESI-MS (+): 5117.9 (LC/MS condition 2)

### Example 3-9: Preparation of G1-suc-PMO [C^{Bz}-C^{Bz}-T-C^{Bz}-C^{Bz}-G^{CE,Pac}-G^{CE,Pac}-T-T-C^{Bz}-A^{Bz}] -ON (using bromotripyrrolidinophosphonium hexafluorophosphate as a condensation agent)

G1-suc-PMO [C^{Bz}-C^{Bz}-T-C^{Bz}-C^{Bz}-G^{CE,Pac}-G^{CE,Pac}-T-T-C^{Bz}] -OFF (28 mg, 0.00631 mmol) and H-phosphonate-PMO [A^{Bz}] -ON (triethylamine salt) (9.5 mg, 0.0126 mmol) and 10 µL (0.126 mmol) of N-methylimidazole were dissolved in 250 µL of dichloromethane. To this solution was added 29 mg (0.0631 mmol) of bromotripyrrolidinophosphonium hexafluorophosphate, followed by stirring at 40°C. After 10 minutes, 100 µL of the solution was mixed with 100 µL of an oxidizing solution (0.1 M iodine, 2 M dimethylamine/THF solution) and stirred for 5 minutes at room temperature. After completion of the reaction, a solution of 10% sodium thiosulfate in water was added to the reaction mixture, and the organic and aqueous layers were separated. The organic layer was diluted 20-fold with a solution of 80% acetonitrile in water and analyzed by HPLC (starting material Rt: 16.1 min, object product Rt: 19.5 min, conversion yield 94.0%, ODS Condition 2).

### Example 3-10: Preparation of G1-suc-PMO [C^{Bz}-C^{Bz}-T-C^{Bz}-C^{Bz}-G^{CE,Pac}-G^{CE,Pac}-T-T-C^{Bz}-A^{Bz}] -ON (using diphenylbis (2,6-dimethylphenyl)chlorophosphate as a condensation agent)

G1-suc-PMO [C^{Bz}-C^{Bz}-T-C^{Bz}-C^{Bz}-G^{CE,Pac}-G^{CE,Pac}-T-T-C^{Bz}] -OFF (28 mg, 0.00631 mmol) and H-phosphonate-PMO [A^{Bz}] -ON (triethylamine salt) (9.5 mg, 0.0126 mmol) and 10 µL (0.126 mmol) of N-methylimidazole were dissolved in 250 µL of dichloromethane. To this solution was added 21 mg (0.0631 mmol) of diphenylbis(2,6-dimethylphenyl)chlorophosphate, followed by stirring at 40°C. After 10 minutes, 100 µL of the solution was mixed with 100 µL of an oxidizing solution (0.1 M iodine, 2 M dimethylamine/THF solution) and stirred for 5 minutes at room temperature. After completion of the reaction, a solution of 10% sodium thiosulfate in water was added to the reaction mixture, and the organic and aqueous layers were separated. The organic layer was diluted 20-fold with a solution of 80% acetonitrile in water and analyzed by HPLC (starting material Rt: 16.1 min, object product Rt: 19.5 min, conversion yield 97.0%, ODS Condition 2).

### Example 3-11: Preparation of G1-suc-PMO [C^{Bz}-C^{Bz}-T-C^{Bz}-C^{Bz}-G^{CE,Pac}-G^{CE,Pac}-T-T-C^{Bz}-A^{Bz}] -ON (using bis (2-oxo-3-oxazolidinyl)phosphinic chloride as a condensation agent)

G1-suc-PMO [C^{Bz}-C^{Bz}-T-C^{Bz}-C^{Bz}-G^{CE,Pac}-G^{CE,Pac}-T-T-C^{Bz}] -OFF (28 mg, 0.00631 mmol) and H-phosphonate-PMO [A^{Bz}] -ON (triethylamine salt) (9.5 mg, 0.0126 mmol) and 10 µL (0.126 mmol) of N-methylimidazole were dissolved in 250 µL of dichloromethane. To this solution was added 16 mg (0.0631 mmol) of bis(2-oxo-3-oxazolidinyl)phosphinic chloride, and the mixture was stirred at 40°C. After 10 minutes, 100 µL of the solution was mixed with 100 µL of an oxidizing solution (0.1 M iodine, 2 M dimethylamine/THF solution) and stirred for 5 minutes at room temperature. After completion of the reaction, a solution of 10% sodium thiosulfate in water was added to the reaction mixture, and the organic and aqueous layers were separated. The organic layer was diluted 20-fold with a solution of 80% acetonitrile in water and analyzed by HPLC (starting material Rt: 16.1 min, object product Rt: 19.5 min, conversion yield 95.9%, ODS Condition 2).

### Example 3-12: Preparation of G1-suc-PMO[T-A^{Bz}]-ON (using piperidine instead of dimethylamine in the oxidizing solution)

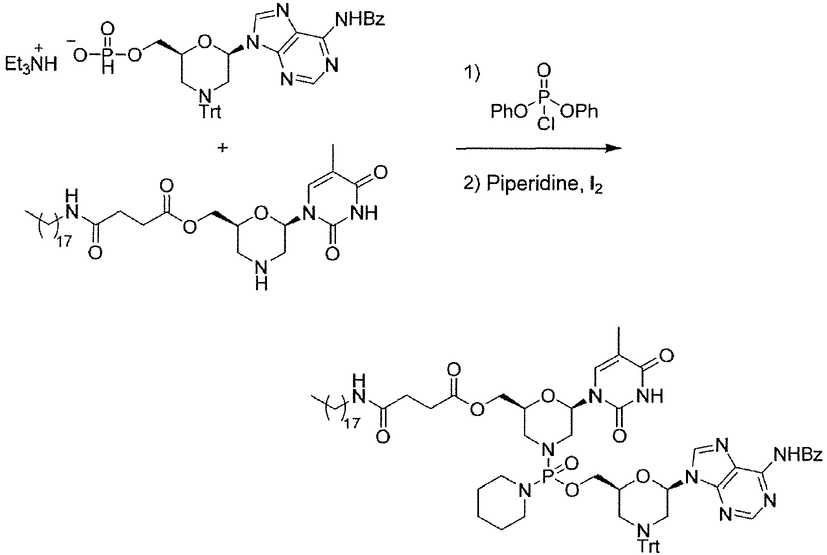

G1-suc-PMO[T]-OFF (15 mg, 0.025 mmol) and H-phosphonate-PMO[A^{Bz}]-ON (triethylamine salt) (29 mg, 0.0375 mmol) and 20 µL (0.253 mmol) of N-methylimidazole were dissolved in 500 µL of dichloromethane. To this solution was added 26 µL (0.127 mmol) of diphenyl chlorophosphate, and the mixture was stirred at room temperature. After 10 minutes, 100 µL of the solution was mixed with 100 µL of an oxidizing solution (0.1 M iodine, 2 M piperidine/THF solution) and stirred for 10 minutes at room temperature. After completion of the reaction, a solution of 10% sodium thiosulfate in water was added to the reaction mixture, and the organic and aqueous layers were separated. The organic layer was diluted 20-fold with a solution of 80% acetonitrile in water and analyzed by HPLC (starting material Rt: 12.0 min, object product Rt: 19.6, 20.1 min, conversion yield: 98.9%, ODS Condition 2).
ESI-MS (+): 1317.7 (LC/MS condition 2)

### Example 3-13:

The oligomers listed below were produced in a similar manner.

**[Table 5]**

| G1-suc-P MO-OFF | H-Phos phonate | Activating Agent | | Base | Conversion Yield (%) |
|---|---|---|---|---|---|
| T | A^{Bz} | diphenyl chlorophosphate | | N-methyl imidazole | 98.6 |
| T | C^{Bz} C^{Bz} | diphenyl chlorophosphate | | pyridine | 96.3 |
| A^{Bz} | A | diphenyl chlorophosphate | | pyridine | 97. 3 |
| A^{Bz} | C^{Bz} | diphenyl chlorophosphate | | pyridine | 86.9 |
| C^{Bz} | C^{Bz} | diphenyl chlorophosphate | | pyridine | 93.6 |
| C^{Bz} | T | diphenyl chlorophosphate | | pyridine | 96.3 |
| T- T | C^{Bz} | diphenyl chlorophosphate | | pyridine | 83.9 |
| A^{Bz}-C^{Bz} | A^{Bz} | diphenyl chlorophosphate | | pyridine | 9 6, 0 |
| A^{Bz}-C^{Bz} | C^{Bz} | diphenyl chlorophosphate | | pyridine | 95. 5 |
| A^{Bz}-G^{CE-}P^{ac} | C^{Bz} | diphenyl chlorophosphate | | pyridine | 81.3 |
| T- T- A^{Hz} | A^{Bz} | diphenyl chlorophosphate | | pyridine | 95.1 |
| T- T-A^{Bz} | C^{Bz} | diphenyl chlorophosphate | | pyridine | 98.0 |
| C^{Bz}-C^{Bz}-T-C^{Bz}-C^{Bz} | T | | bromotripyrolidino phosphonium= hexafluorophosphate | N-methyl imidazole | 95.9 |
| T-G^{CE,Pac}-G^{CE,Pac}-G^{CE} G^{CE,Pac}-G^{CE Pac}-A^{Bz}T-C^{Bz}-C^{Bz}-A^{B} -G^{CE,Pac} | A^{Bz} | diphenyl chlorophosphate | | N-methyl imidazole | 88.5 |

### Example 4: Preparation of PMO Oligomer

### Example 4-1: Preparation of G1-suc-PMO[A^{Bz}-C^{Bz}-T-T]-ON

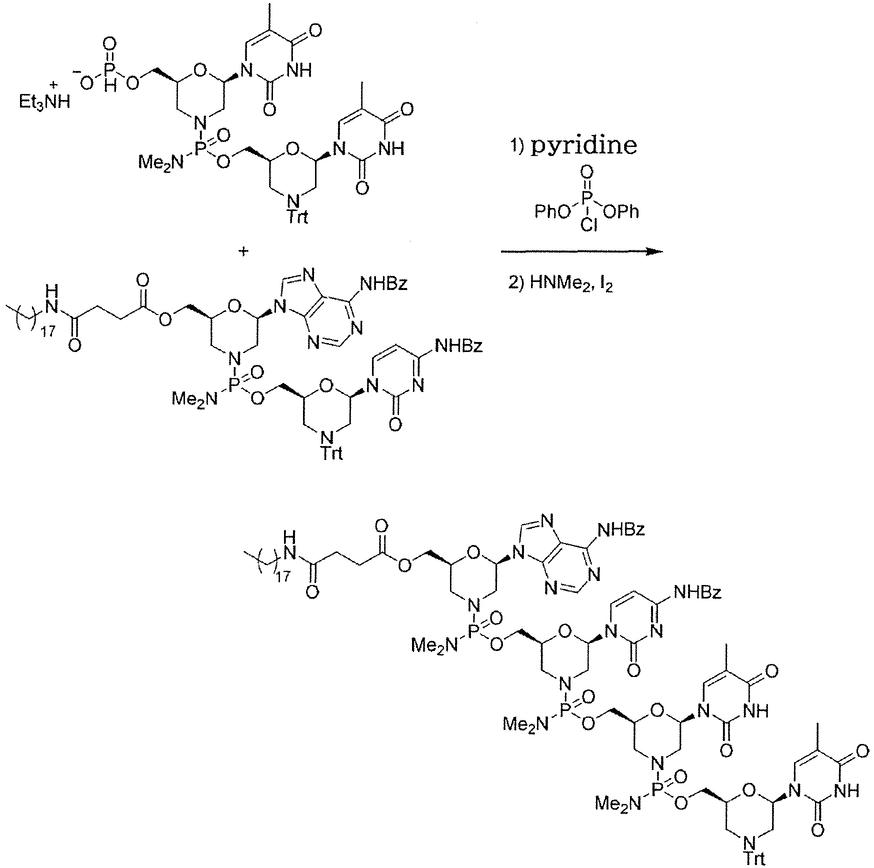

G1-suc-PMO[A^{Bz}-C^{Bz}]-OFF (28 mg, 0.025 mmol) and H-phosphonate-PMO[T-T]-ON (triethylamine salt) (37 mg, 0.0375 mmol) were dissolved in 500 µΣ, of 20% pyridine/dichloromethane. To this solution was added 12 µL (0.0563 mmol) of diphenyl chlorophosphate, and the solution was stirred at room temperature. After 10 minutes, 100 µL of the solution was mixed with 100 µΣ, of an oxidizing solution (0.1 M iodine, 2 M dimethylamine/THF solution) and stirred for 10 minutes at room temperature. After completion of the reaction, a solution of 10% sodium thiosulfate in water was added to the reaction mixture, and the organic and aqueous layers were separated. The organic layer was diluted 20-fold with a solution of 80% acetonitrile in water and analyzed by HPLC (starting material Rt: 13.8 min, object product Rt: 15.3 min, conversion yield 97.2%, ODS Condition 1).
ESI-MS (+): 2026.9 (LC/MS condition 2)

### Example 4-2: Preparation of G1-suc-PMO[A^{Bz}-C^{Bz}-T-T]-ON

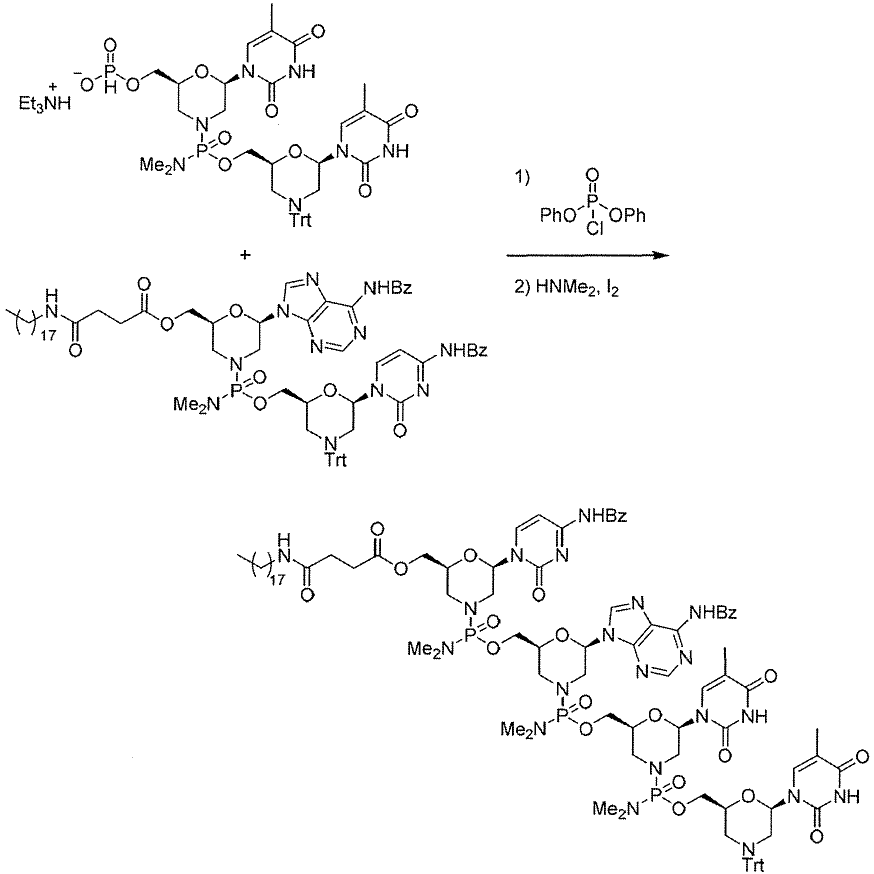

After mixing 2 mL of Liquid a (flow rate: 0.2 mL/min) with 2 mL of Liquid b (flow rate: 0.2 mL/min), the reaction was carried out in a 5 mL tube reactor at 60°C for 12.5 minutes. 100 µL of the resulting solution was mixed with 100 µL of an oxidizing solution (0.1 M iodine, 2 M dimethylamine/THF solution) and stirred for 10 minutes at room temperature. After completion of the reaction, a solution of 10% sodium thiosulfate in water was added to the reaction mixture, and the organic and aqueous layers were separated. The organic layer was diluted 20-fold with a solution of 80% acetonitrile in water and analyzed by HPLC (starting material Rt: 13.79 min, object product Rt: 15.27 min, conversion yield 95.0%, ODS Condition 1).
Liquid a: 82 µL (0.394 mmol) of diphenyl chlorophosphate was dissolved in 2.5 mL of anhydrous dichloromethane.
Liquid b: H-phosphonate-PMO[T-T]-ON (257 mg, 0.263 mmol) and G1-suc-PMO[A^{Bz}-C^{Bz}]-OFF (197 mg, 0.175 mmol) were dissolved in 2.5 mL of 40% anhydrous pyridine/60% anhydrous dichloromethane solution.

### Example 4-3: Preparation of G1-suc-PMO[T-T-A^{Bz}-T-T]-ON

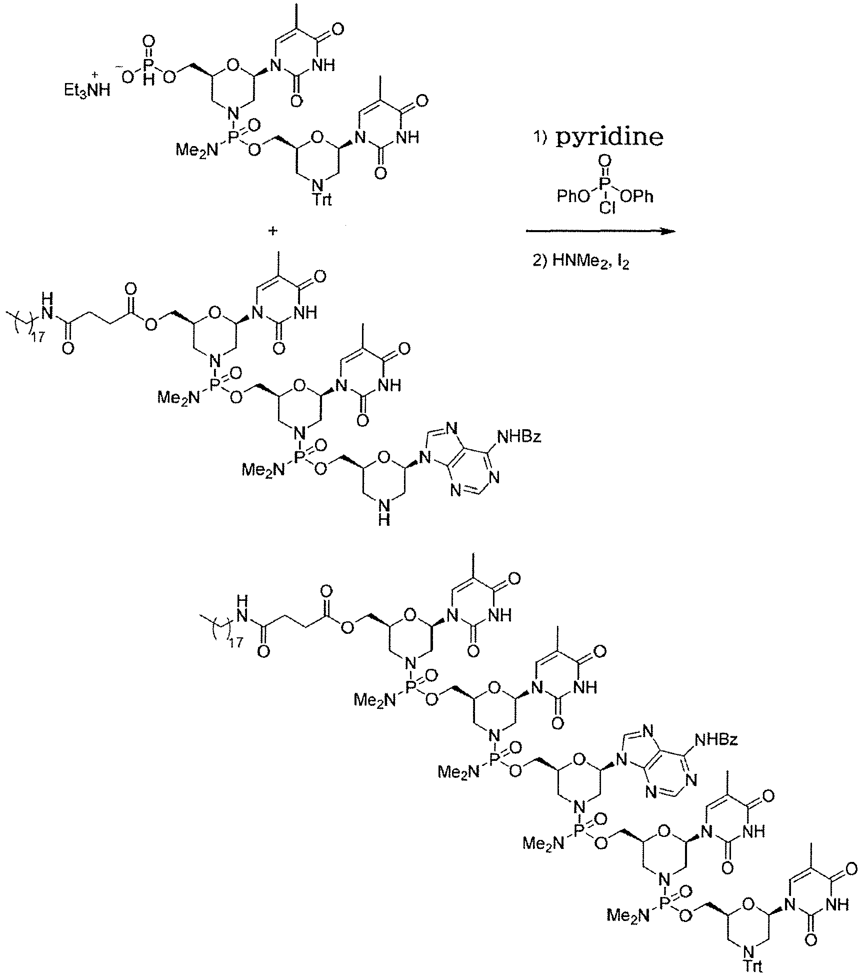

G1-suc-PMO[T-T-A^{Bz}]-OFF (34 mg, 0.025 mmol) and H-phosphonate-PMO[T-T] -ON (triethylamine salt) (37 mg, 0.0375 mmol) were dissolved in 500 µΣ, of 20% pyridine/dichloromethane. To this solution was added 12 µL (0.0563 mmol) of diphenyl chlorophosphate, and the solution was stirred at room temperature. After 10 minutes, 100 µL of the solution was mixed with 100 µL of an oxidizing solution (0.1 M iodine, 2 M dimethylamine/THF solution) and stirred for 10 minutes at room temperature. After completion of the reaction, a solution of 10% sodium thiosulfate in water was added to the reaction mixture, and the organic and aqueous layers were separated. The organic layer was diluted 20-fold with a solution of 80% acetonitrile in water and analyzed by HPLC (starting material Rt: 12.4 min, object product Rt: 14.2 min, conversion yield 88.9%, ODS Condition 1).
ESI-MS (+): 2268.0 (LC/MS condition 2)

### Example 4-4: Preparation of G1-suc-PMO[T-T-A^{Bz}-T-T]-ON

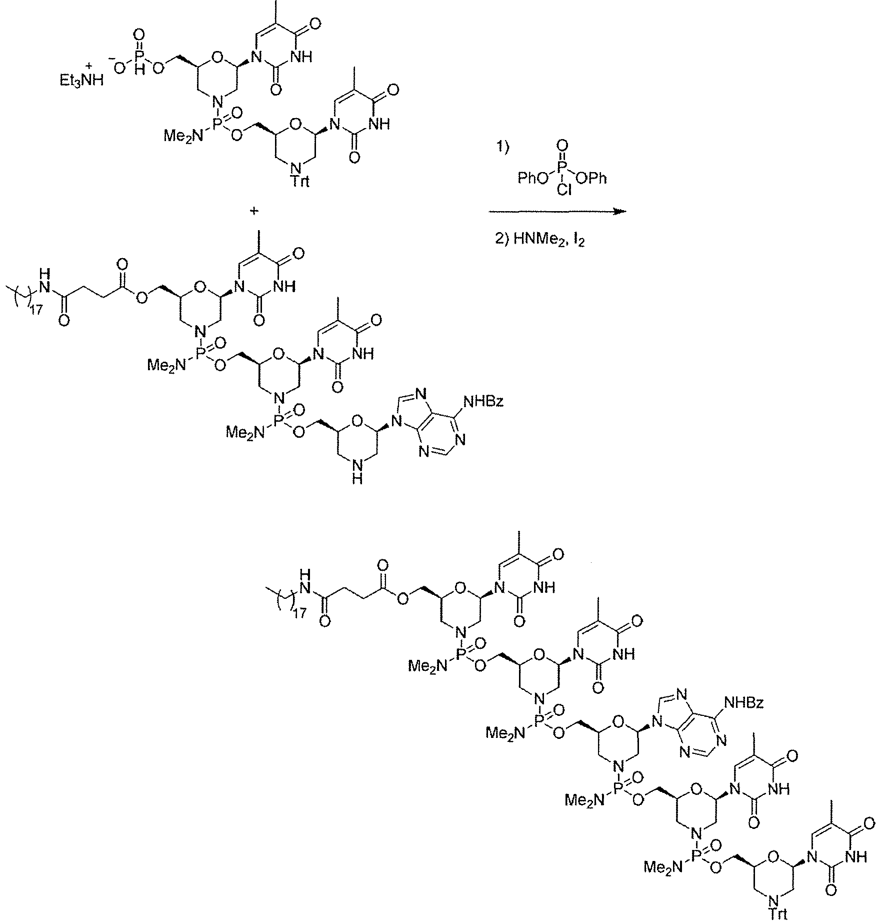

After mixing 2 mL of Liquid a (flow rate: 0.2 mL/min) with 2 mL of Liquid b (flow rate: 0.2 mL/min), the reaction was carried out in a 5 mL tube reactor at 60°C for 12.5 minutes. 100 µL of the resulting solution was mixed with 100 µL of an oxidizing solution (0.1 M iodine, 2 M dimethylamine/THF solution) and stirred for 10 minutes at room temperature. After completion of the reaction, a solution of 10% sodium thiosulfate in water was added to the reaction mixture, and the organic and aqueous layers were separated. The organic layer was diluted 20-fold with a solution of 80% acetonitrile in water and analyzed by HPLC (starting material Rt: 12.42 min, object product Rt: 14.24 min, conversion yield 94.1%, ODS Condition 1).
Liquid a: 82 µL (0.394 mmol) of diphenyl chlorophosphate was dissolved in 2.5 mL of anhydrous dichloromethane.
Liquid b: H-phosphonate-PMO[T-T]-ON (257 mg, 0.263 mmol) and G1-suc-PMO[T-T-A^{Bz}]-OFF (239 mg, 0.175 mmol) were dissolved in 2.5 mL of 40% anhydrous pyridine/60% anhydrous dichloromethane solution.

### Example 4-5: Preparation of G1-suc-PMO[T-G^{CE,Pac}-G^{CE,Pac}-G^{CE,Pac}-A^{Bz}-C^{Bz}-A^{Bz}-T-C^{Bz}-T]-ON (using triethylamine salt of H-phosphonate)

G1-suc-PMO[T-G^{CE,Pac}-G^{CE,Pac}-G^{CE,Pac}-A^{Bz}]-OFF (33 mg, 0.0123 mmol) and H-phosphonate-PMO [C^{Bz}-A^{Bz}-T-C^{Bz}-T]-ON (triethylamine salt) (40 mg, 0.0185 mmol) and 20 µL (0.247 mmol) of N-methylimidazole were dissolved in 250 µL of dichloromethane. To this solution, 26 µL (0.124 mmol) of diphenyl chlorophosphate was added, and the mixture was stirred at 40°C. After 10 minutes, 100 µL of the solution was mixed with 100 µL of an oxidizing solution (0.1 M iodine, 2 M dimethylamine/THF solution) and stirred for 5 minutes at room temperature. After completion of the reaction, a solution of 10% sodium thiosulfate in water was added to the reaction mixture, and the organic and aqueous layers were separated. The organic layer was diluted 20-fold with a solution of 80% acetonitrile in water and analyzed by HPLC (starting material Rt: 14.4 min, object product Rt: 18.6 min, conversion yield: 84.7%, target purity: 23.4%, ODS Condition 2) .
ESI-MS (+): 4845.8 (LC/MS condition 2)

### Example 4-6: Preparation of G1-suc-PMO[T-G^{CE,Pac}-G^{CE,Pac}-G^{CE,Pac}-A^{Bz}-C^{Bz}-A^{Bz}-T-C^{Bz}-T]-ON (using DBU salt of H-phosphonate)

G1-suc-PMO[T-G^{CE,Pac}-G^{CE,Pac}-G^{CE,Pac}-A^{Bz}]-OFF (33 mg, 0.0123 mmol) and H-phosphonate-PMO [C^{Bz}-A^{Bz}-T-C^{Bz}-T]-ON (DBU salt) (40 mg, 0.0185 mmol) and 20 µL (0.247 mmol) of N-methylimidazole were dissolved in 250 µL of dichloromethane. To this solution, 26 µL (0.124 mmol) of diphenyl chlorophosphate was added, and the mixture was stirred at 40°C. After 10 minutes, 100 µL of the solution was mixed with 100 µL of an oxidizing solution (0.1 M iodine, 2 M dimethylamine/THF solution) and stirred for 5 minutes at room temperature. After completion of the reaction, a solution of 10% sodium thiosulfate in water was added to the reaction mixture, and the organic and aqueous layers were separated. The organic layer was diluted 20-fold with a solution of 80% acetonitrile in water and analyzed by HPLC (starting material Rt: 14.4 min, object product Rt: 18.6 min, conversion yield: 95.8%, target purity: 53.1%, ODS Condition 2).

### Example 4-7: Preparation of G1-suc-PMO[C^{Bz}-C^{Bz}-T-C^{Bz-}C^{Bz}-G^{CE,Pac}-G^{CE,Pac}-T-T-C^{Bz}-C^{Bz}-G^{CE,Pac}-A^{Bz}-A^{Bz}-G^{CE,Pac}]-ON

G1- suc - PMO [C^{Bz}-C^{Bz}-T-C^{Bz}-C^{Bz}-G^{CE,Pac}-G^{CE,Pac}-T-T-C^{Bz}]-OFF (28 mg, 0.00631 mmol) and H-phosphonate-PMO[C^{Bz}-G^{CE,Pac}-A^{Bz}-A^{Bz}-G^{CE,Pac}]-ON (DBU salt) (32 mg, 0.0126 mmol) and 16 µL (0.126 mmol) of N-methylimidazole were dissolved in 250 µL of dichloromethane. To this solution was added 13 µL (0.0631 mmol) of diphenyl chlorophosphate, followed by stirring at 40°C. After 10 minutes, 100 µL of the solution was mixed with 100 µL of an oxidizing solution (0.1 M iodine, 2 M dimethylamine/THF solution) and stirred for 5 minutes at room temperature. After completion of the reaction, a solution of 10% sodium thiosulfate in water was added to the reaction mixture, and the organic and aqueous layers were separated. The organic layer was diluted 20-fold with a solution of 80% acetonitrile in water and analyzed by HPLC (starting material Rt: 16.1 min, object product Rt: 19.8 min, conversion yield 91.5%, ODS Condition 2).
ESI-MS (+): 7064.4 (LC/MS condition 2)

### Example 4-8:

The oligomers listed below were produced in a similar manner.

**[Table 6]**

| G 1 - s u c - P MO-OFF | Phosphonate | Activating Agent | Base | Conversion Yield (%) |
|---|---|---|---|---|
| C^{Bz} | T-T | diphenyl chlorophosphate | N-methyl imidazole | 89.7 |
| T | C^{Bz}-A | diphenyl chlorophosphate | N-methyl imidazole | 98.5 |
| T | C^{Bz}-A^{Bz}-T-C^{Bz} -T | diphenyl chlorophosphate | N-methyl imidazole | 95.0 |
| T-T | T-T | diphenyl chlorophosphate | pyridine | 94.8 |
| A^{Bz}-G^{CE,Pac} | T-T | diphenyl chlorophosphate | pyridine | 94.4 |
| C^{Bz}-C^{Bz}-T -C^{Bz}-C^{Bz} | T-T | bromotri pyrrolidino phosphonium= hexafluoro phosphate | N-methyl imidazole | 92.8 |
| T-G^{CE,Pac}-G^{CE,Pac}-G^{CE} ^{,Pac}-A^{Bz} | C^{Bz}-G^{CE,Pac}-A^{B z}-A^{Bz}-G^{CE,Pac} | diphenyl chlorophosphate | N-methyl imidazole | 93.9 |

Example 5: Preparation of G1-suc-PMO [C^{Bz}-C^{Bz}-T-C^{Bz}-C^{Bz}-G^{CE,Pac}-G^{CE,Pac}-T-T-C^{Bz}-T-G^{CE,Pac}-A^{Bz}-A^{Bz}-G^{CE,Pac}-G^{CE,Pac}-T-G^{CE,Pac}-T-T]-ON

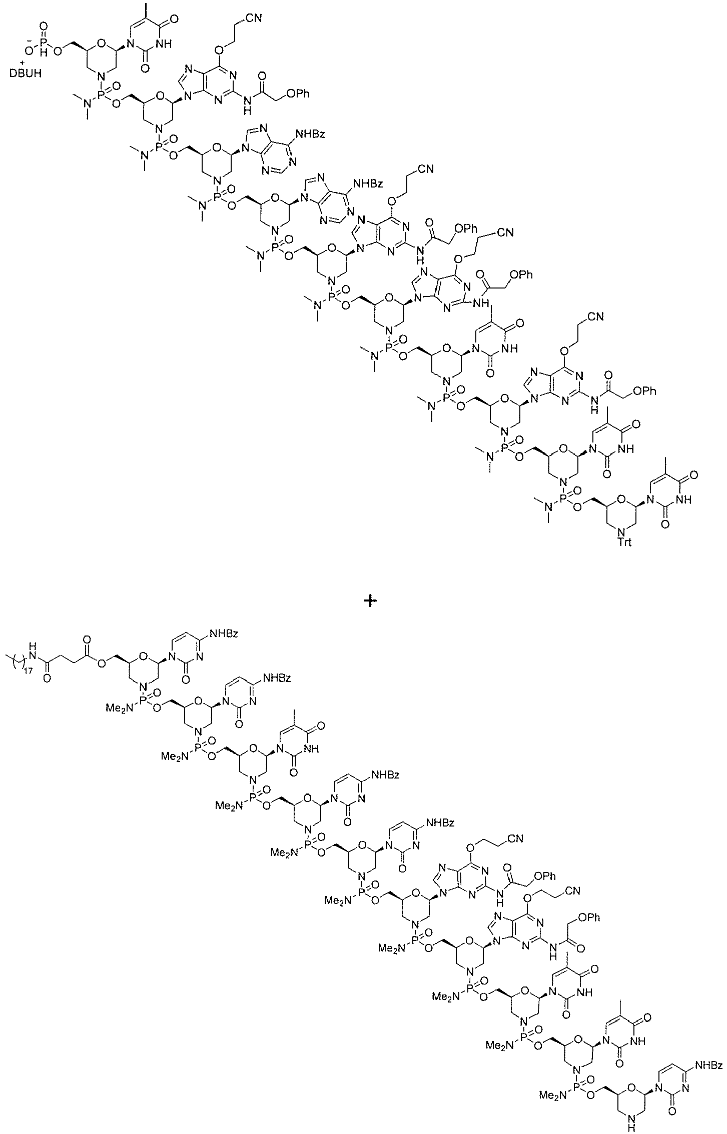

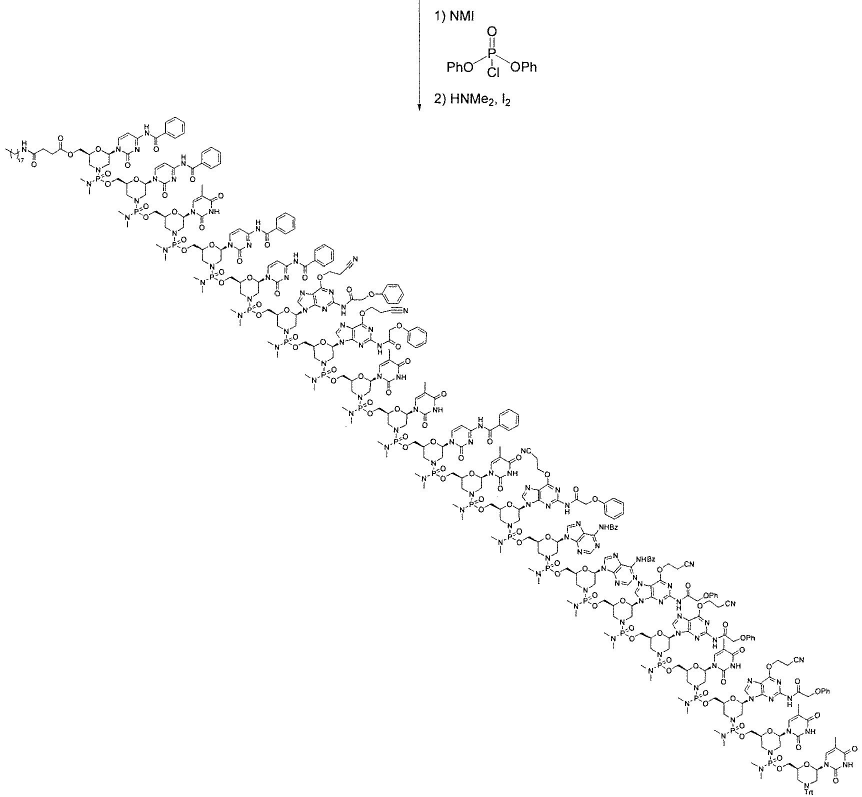

G1-suc-PMO[C^{Bz}-C^{Bz}-T-C^{Bz}-C^{Bz}-G^{CE,Pac}-G^{CE,Pac}-T-T-C^{Bz}]-OFF (28 mg, 0.00631 mmol) and H-phosphonate-PMO[T-G^{CE,Pac}-A^{Bz}-A^{Bz}-G^{CE,Pac}-G^{CE,Pac}-T-G^{CE,Pac}-T-T]-ON (DBU salt) (43 mg, 0.00947 mmol) and 16 µL (0.126 mmol) of N-methylimidazole were dissolved in 250 µL of dichloromethane. To this solution was added 13 µL (0.0631 mmol) of diphenyl chlorophosphate, followed by stirring at 40°C. After 10 minutes, 100 µL of the solution was mixed with 100 µL of an oxidizing solution (0.1 M iodine, 2 M dimethylamine/THF solution) and stirred for 5 minutes at room temperature. After completion of the reaction, a solution of 10% sodium thiosulfate in water was added to the reaction mixture, and the organic and aqueous layers were separated. The organic layer was diluted 20-fold with a solution of 80% acetonitrile in water and analyzed by HPLC (starting material Rt: 15.2 min, object product Rt: 17.6 min, conversion yield 91.0%, ODS Condition 2). To 10 µL of the reaction mixture, 190 µL of a mixture of ammonia water: ethanol = 3:1 was added, and the mixture was stirred at 55°C for 18 hours. The reaction mixture was filtered, and the filtrate was diluted 50-fold with a solution of 20% acetonitrile in water. MS measurement was performed and confirmed that the object product was formed.
ESI-MS (+): 6848.4 (LC/MS condition 1)

## Claims

1. A method for producing a compound of the formula [C-1] comprising that a compound of the formula [A-1]: 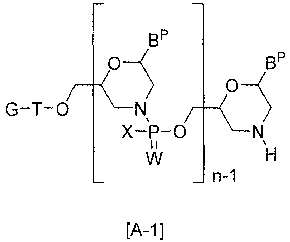 wherein
B^{P} is each the same or different and is each a nucleobase optionally protected;
n is an integer from 1 to 50;
W is each the same or different and is each an oxygen atom or a sulfur atom;
X is each the same or different and is each a hydroxyl group substituted with a group removable under neutral condition, 1,1,3,3-tetra(C₁₋₆ alkyl) guanidyl, C₁₋₆ alkoxy, di(C₁₋₆ alkyl)amino, mono(amino substituted with a group removable under basic condition-C₁₋₆ alkyl)amino, di(amino substituted with a group removable under basic condition-C₁₋₆ alkyl)amino, or a substituent group of the general formula [2] : 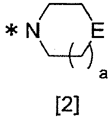
wherein
* is a binding position with P;
a is an integer from 0 to 2;
E is CH₂, CH-A¹ or N-A²;
A¹ is C₁₋₆ alkyl, mono(C₁₋₆ alkyl) amino-C₁₋₆ alkyl substituted with a group removable under basic condition, di (C₁₋₆ alkyl) amino-C₁₋₆ alkyl, tri (C₁₋₆ alkyl) ammonio-C₁₋₆ alkyl, amino substituted with a group removable under basic condition, mono(C₁₋₆ alkyl)amino substituted with a group removable under basic condition, di(C₁₋₆ alkyl)amino, tri(C₁₋₆ alkyl)ammonio, amino substituted with amidino substituted with a group removable under basic condition, or a substituent group of the general formula [3]: 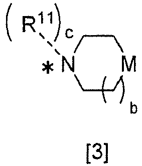
wherein
* is a binding position with E;
b is an integer from 0 to 2;
c is 0 or 1;
R¹¹ is C₁₋₆ alkyl; and
M is CH₂, oxygen atom, sulfur atom or N-(a group removable under basic condition); and
A² is C₁₋₆ alkyl, mono(C₁₋₆ alkyl) amino-C₁₋₆ alkyl substituted with a group removable under basic condition, di (C₁₋₆ alkyl) amino-C₁₋₆ alkyl, tri(C₁₋₆ alkyl) ammonio-C₁₋₆ alkyl, a group removable under basic condition, aryl or heteroaryl;
G is
(1) a silicon substituent group,
(2) C₁₋₁₈ alkyl-carbonyl optionally substituted, C₁₋₁₈ alkoxy-carbonyl optionally substituted, a long-chain alkyl-carbonyl, or a long-chain alkoxy-carbonyl,
(3) benzoyl substituted with one to five long-chain alkyloxy and/or long-chain alkenyloxy, or
(4) a substituent group represented by the general formula [7] : 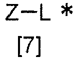
wherein
* is a binding position with T;
Z is
(1) (a soluble polymer soluble in an organic solvent)-oxy,
(2) (a soluble polymer soluble in an organic solvent)-amino,
(3) a long-chain alkyloxy, benzoyl substituted with one to five long-chain alkyloxy and/or long-chain alkenyloxy, or benzyl substituted with one to five long-chain alkyloxy and/or long-chain alkenyloxy,
(4) a solid-phase support, or
(5) a substituent group represented by the following general formula [8A] to [8N]: 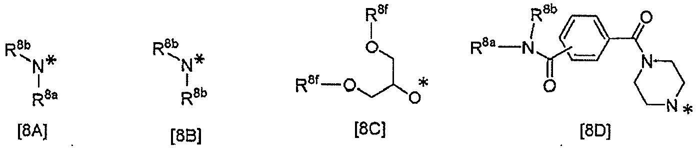 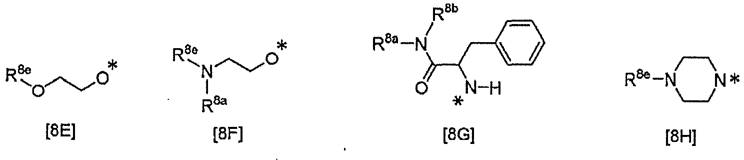 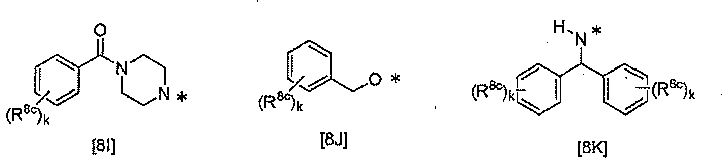 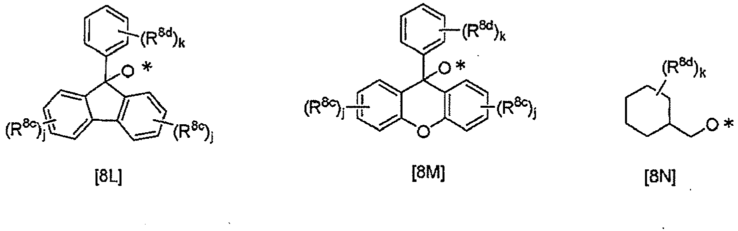
wherein
* is a binding position with L;
j is an integer from 0 to 4;
k is an integer from 0 to 5;
R^{8a} is a hydrogen atom or C₁₋₆ alkyl;
R^{8b} is each the same or different and is each a long-chain alkyl;
R^{8c} is each the same or different and is each a substituent group of the general formulae selected from [9A] to [9E] 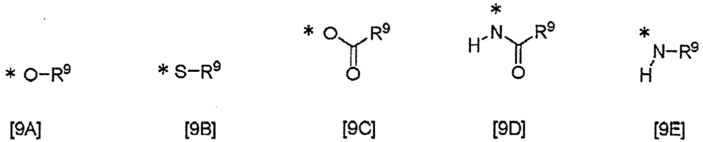
wherein
* is a binding position;
R⁹ is a long-chain alkyl and/or a long-chain alkenyl;
R^{8d} is each the same or different and is each a hydrogen atom, a halogen, a long-chain alkyl optionally substituted with 1 to 13 halogen atoms or a long-chain alkyloxy optionally substituted with 1 to 13 halogen atoms;
R^{8e} is
(1) a long-chain alkyl,
(2) a long-chain alkyl-carbonyl, or
(3) benzoyl substituted with one to five long-chain alkyloxy and/or long-chain alkenyloxy; and
R^{8f} is
(1) a long-chain alkyl,
(2) a long-chain alkyl-carbonyl, or
(3) a long-chain alkenyl-carbonyl; and
L is a group of the general formula [10]: 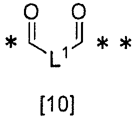 wherein
* is a binding position with Z;
** is a binding position with T; and
L¹ is C₂₋₁₀ alkylene optionally substituted or C₆₋₁₀ arylene optionally substituted; or
a group of the general formula [10-1]: 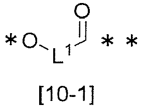 wherein
* is a binding position with Z;
** is a binding position with T; and
L¹ is C₁₋₁₀ alkylene optionally substituted or C₆₋₁₀ arylene optionally substituted; and
T is a single bond or a substituent group of the general formula [11], provided that T is a single bond when G is a silicon substituent group: 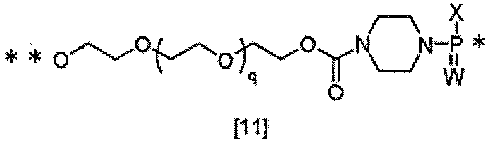 wherein
X and W are as defined above;
* is a binding position with **O, *O or *N of the above formulae [4a] to [4d] ;
** is a binding position with G; and
q is an integer from 0 to 10;
and a compound of the formula [B-1]: 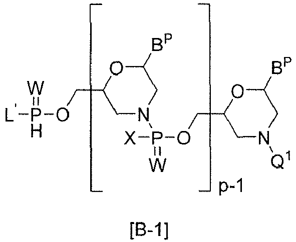 wherein
B^{P}, W and X are as defined above; and
p is an integer of 1 to 50;
L' is OH, -O⁻N+H(aliphatic amine), -O⁻N+H(cyclic amine), or -O⁻N⁺H(aromatic amine); and
Q¹ is a group removable under acidic condition,
are subjected to
(1) a treatment with at least one condensation agent selected from the group consisting of Phosphorus Reagent 1, Phosphorus Reagent 2 and onium reagent in the presence of a base, and then
(2) a treatment with an oxidizing agent and an organic amine to obtain the compound of the formula [C-1]: 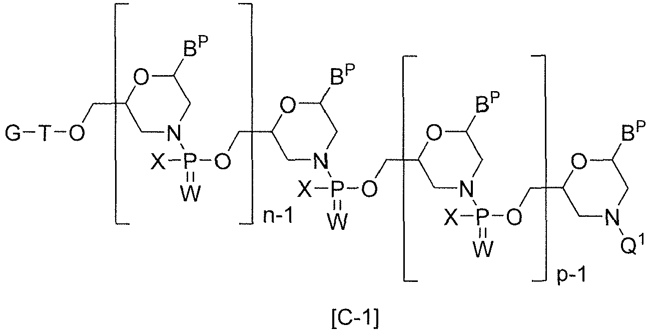
wherein
B^{P}, Q¹, W, X, G, T, n and p are as defined above.

2. The method according to claim 1 comprising further that the compound of the formula [C-1]: 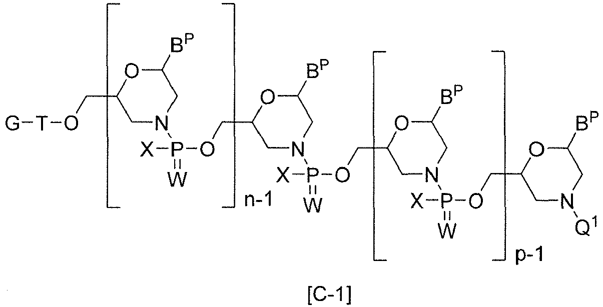 wherein
B^{P}, Q¹, W, X, G, T, n and p are as defined above,
is treated with an acid or with an acid and a scavenger to obtain a compound of the formula [C-0-1]: 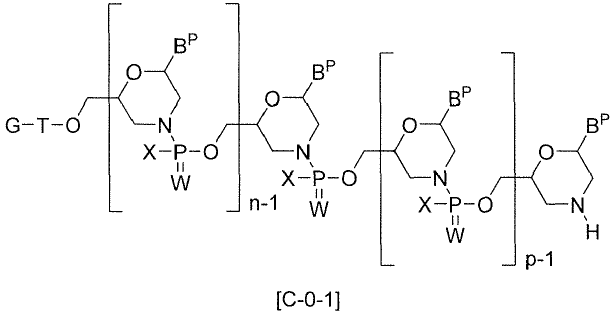 wherein
B^{P}, Q¹, W, X, G, T, n and p are as defined above;
and then, said compound of the formula [C-0-1] and a compound of the formula [B-0-1]: 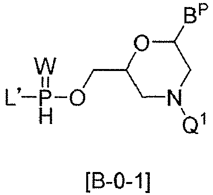 wherein
B^{P}, Q¹, W and L' are as defined above, are subjected to
(1) a treatment with at least one condensation agent selected from the group consisting of Phosphorus Reagent 1, Phosphorus Reagent 2 and onium reagent in the presence of a base, and then
(2) a treatment with an oxidizing agent and an organic amine to obtain a compound of the formula [C-1-1]: 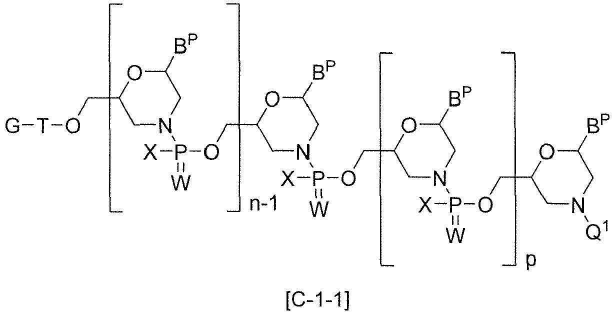
wherein
B^{P}, Q¹, W, X, G, T, n and p are as defined above.

3. The method according to claim 1 comprising further that the compound of the formula [C-1]: 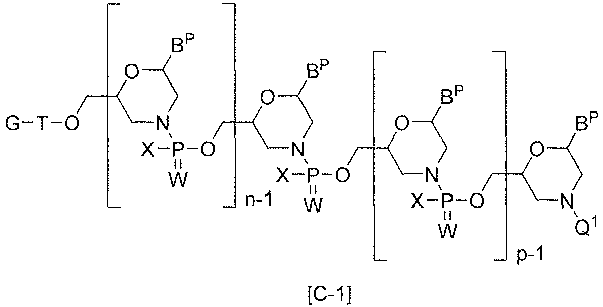 wherein
B^{P}, Q¹, W, X, G, T, n and p are as defined above,
is treated under a condition for removing the hydroxyl-protecting group to obtain a compound of the formula [C-0-2] : 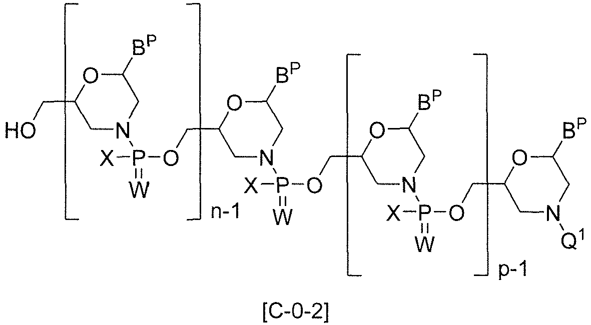 wherein
B^{P}, Q¹, W, X, n and p are as defined above;
and then said compound of the formula [C-0-2] is subjected to
(1) a treatment with a compound of the formula [P-1]: 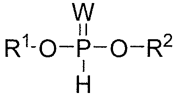 wherein
W is an oxygen atom or a sulfur atom, and
R¹ and R² are each the same or different and are each selected from the group consisting of H, C₁₋₆ alkyl optionally substituted, phenyl optionally substituted and PH(=O)-OH;
and a base and then
(2) a treatment with a hydrolyzing solution to obtain a compound of the formula [C-0-2-1]: 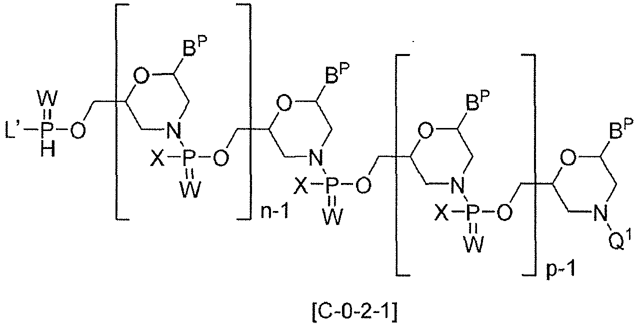
wherein B^{P}, Q¹, W, X, L', n and p are as defined above.

4. The method according to claim 3 comprising further that the compound of the formula [C-0-2-1]: 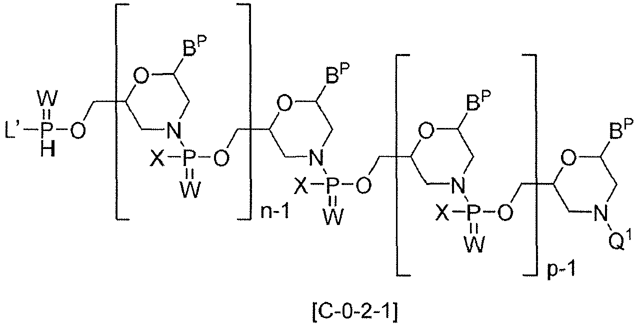 wherein
B^{P}, Q¹, W, X, L', n and p are as defined above,
and a compound of the formula [A-0-1]: 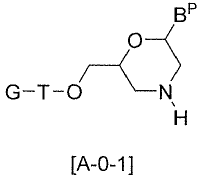 wherein
B^{P}, G and T are as defined above,
are subjected to
(1) a treatment with at least one condensation agent selected from the group consisting of Phosphorus Reagent 1, Phosphorus Reagent 2 and onium reagent in the presence of a base, and then
(2) a treatment with an oxidizing agent and an organic amine to obtain a compound of the formula [C-1-2]: 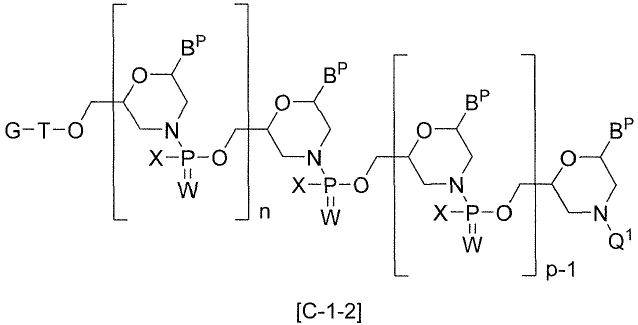
wherein
B^{P}, Q¹, W, X, G, T, n and p are as defined above.

5. The method according to claim 1 comprising further that a compound of the formula [B-1-0]: 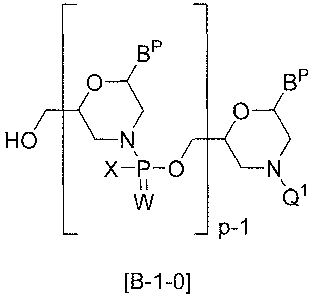 wherein
B^{P}, Q¹, W, X and p are as defined above,
is subjected to
(1) a treatment with a compound of the formula [P-1]: 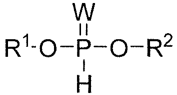 wherein
W, R¹ and R² are as defined above;
and a base and then,
(2) a treatment with a hydrolyzing solution to obtain a compound of the formula [B-1]: 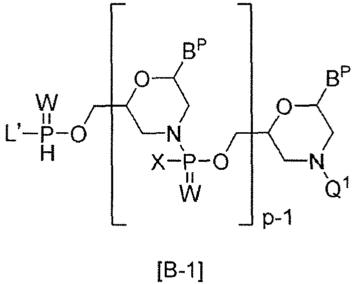 wherein
B^{P}, Q¹, W, X, L' and p are as defined above.

6. The method according to claim 1 comprising further that a compound of the formula [A-0]: 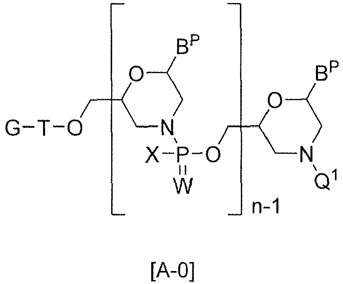 wherein
B^{P}, Q¹, W, X, G, T and n are as defined above,
is treated with an acid or with an acid and a scavenger to obtain a compound of the formula [A-1]: 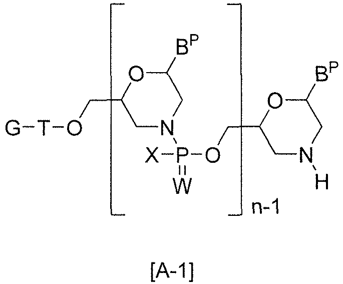 wherein
B^{P}, W, X, G, T and n are as defined above.

7. The method according to any one of claims 1 to 6, wherein
G is selected from the group consisting of: 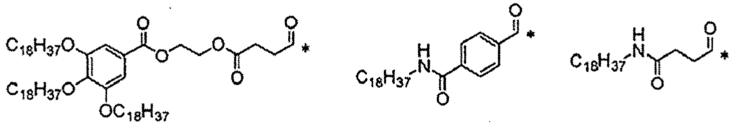 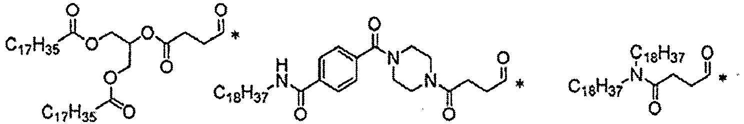 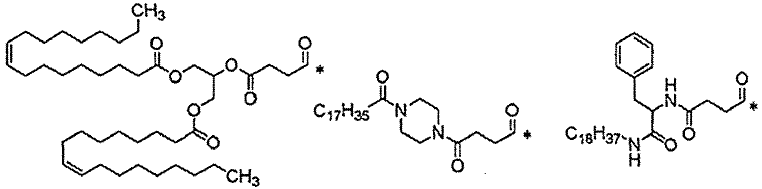 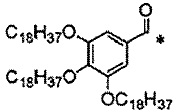
wherein
* is a binding position with T, and
T is a single bond.

8. A compound of the formula [B-1]: 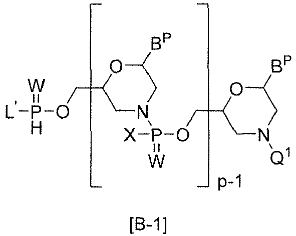 wherein
B^{P} is each the same or different and is each a nucleobase optionally protected;
Q¹ is trityl or dimethoxytrityl;
L' is -O⁻N⁺H(CH₂CH₃)₃ or -O-(HDBU)⁺;
W is O; and
p is an integer from 2 to 20.

9. The compound of the formula [B-1] according to claim 8 selected from the group consisting of:
((2S,6R)-6-(4-benzamido-2-oxopyrimidin-1(2H)-yl)-4-((((2S,6R)-6-(6-benzamido-9H-purin-9-yl)-4-tritylmorpholin-2-yl)methoxy) (dimethylamino)phosphoryl)morpholin-2-yl)methylphosphonate;
triethylammonium ((2S,6R)-6-(4-benzamido-2-oxopyrimidin-1(2H)-yl)-4-((((2S,6R)-6-(6-benzamido-9H-purin-9-yl)-4-tritylmorpholin-2-yl)methoxy)(dimethylamino)phosphoryl)morpholin-2-yl)methylphosphonate;
((2S,6R)-6-(4-benzamido-2-oxopyrimidin-1(2H)-yl)-4-((((2S,6R)-4-((((2S,6R)-6-(4-benzamido-2-oxopyrimidin-1(2H)-yl)-4-((dimethylamino)(((2S,6R)-6-(5-methyl-2,4-dioxo-3,4-dihydropyrimidin-1(2H)-yl)-4-trimethylmorpholin-2-yl)methoxy)phosphoryl)morpholin-2-yl) (dimethylamino)phosphoryl)-6-(5-methyl-2,4-dioxo-3,4-dihydropyrimidin-1(2H)-yl)morpholin-2-yl)methoxy)phosphoryl)-6-(6-benzamido-9H-purin-9-yl)morpholin-2-yl)methoxy)(dimethylamino)phosphoryl)morpholin-2-yl)methylphosphonate;
triethylammonium ((2S,6R)-6-(4-benzamido-2-oxopyrimidin-1(2H)-yl)-4-((((2S,6R)-4-((((2S,6R)-6-(4-benzamido-2-oxopyrimidin-1(2H)-yl)-4-((dimethylamino)(((2S,6R)-6-(5-methyl-2,4-dioxo-3,4-dihydropyrimidin-1(2H)-yl)-4-tritylmorpholin-2-yl)methoxy)phosphoryl)morpholin-2-yl)(dimethylamino)phosphoryl)-6-(5-methyl-2,4-dioxo-3,4-dihydropyrimidin-1(2H)-yl)morpholin-2-yl)methoxy)phosphoryl)-6-(6-benzamido-9H-purin-9-yl)morpholin-2-yl)methoxy)(dimethylamino)phosphoryl)morpholin-2-yl)methyl phosphonate;
((2S,6R)-6-(4-benzamido-2-oxopyrimidin-1(2H)-yl)-4-((((2S,6R)-4-((((2S,6R)-6-(6-benzamido-9H-purin-9-yl)-4-((((2S,6R)-6-(6-benzamido-9H-purin-9-yl)-4-((((2S,6R)-6-((2-cyanoethoxy)-2-(2-phenoxyacetamide)-9H-purin-9-yl)-4-tritylmorpholin-2-yl)methoxy)(dimethylamino)phosphoryl)morpholin-2-yl)methoxy)(dimethylamino)phosphoryl)morpholin-2-yl)methoxy)(dimethylamino)phosphoryl)-6-((2-cyanoethoxy)-2-(2-phenoxyacetamide)-9H-purin-9-yl)morpholin-2-yl)methoxy)(dimethylamino)phosphoryl)morpholin-2-yl)methylphosphonate;
triethylammonium ((2S,6R)-6-(4-benzamido-2-oxopyrimidin-1(2H)-yl)-4-((((2S,6R)-4-((((2S,6R)-6-(6-benzamido-9H-purin-9-yl)-4-((((2S,6R)-6-(6-benzamido-9H-purin-9-yl)-4-((((2S,6R)-6-((2-cyanoethoxy)-2-(2-phenoxyacetamide)-9H-purin-9-yl)-4-tritylmorpholin-2-yl)methoxy)(dimethylamino)phosphoryl)morpholin-2-yl)methoxy)(dimethylamino)phosphoryl)morpholin-2-yl)methoxy)(dimethylamino)phosphoryl)-6-((2-cyanoethoxy-2-(2-phenoxyacetamide)-9H-purin-9-yl)morpholin-2-yl)methoxy)(dimethylamino)phosphoryl)morpholin-2-yl)methylphosphonate;
((2S,6R)-4-((((2S,6R)-4-((((2S,6R)-4-((((2S,6R)-6-(4-benzamido-2-oxopyrimidin-1(2H)-yl)-4-tritylmorpholin-2-yl)methoxy)(dimethylamino)phosphoryl)-6-(5-methyl-2,4-dioxo-3,4-dihydropyrimidin-1(2H)-yl)morpholin-2-yl)methoxy)(dimethylamino)phosphoryl)-6-(5-methyl-2,4-dioxo-3,4-dihydropyrimidin-1(2H)-yl)morpholin-2-yl)methoxy)(dimethylamino)phosphoryl)-6-((2-cyanoethoxy)-2-(2-phenoxyacetamide)-9H-purin-9-yl)morpholin-2-yl)methoxy)(dimethylamino)phosphoryl)-6-((2-cyanoethoxy)-2-(2-phenoxyacetamide)-9H-purin-9-yl)morpholin-2-yl)methylphosphonate;
triethylammonium ((2S,6R)-4-((((2S,6R)-4-((((2S,6R)-4-((((2S,6R)-6-(4-benzamido-2-oxopyrimidin-1(2H)-yl)-4-tritylmorpholin-2-yl)methoxy)(dimethylamino)phosphoryl)-6-(5-methyl-2,4-dioxo-3,4-dihydropyrimidin-1(2H)-yl)morpholin-2-yl)methoxy)(dimethylamino)phosphoryl)-6-(5-methyl-2,4-dioxo-3,4-dihydropyrimidin-1(2H)-yl)morpholin-2-yl)methoxy)(dimethylamino)phosphoryl)-6-((2-cyanoethoxy)-2-(2-phenoxyacetamide)-9H-purin-9-yl)morpholin-2-yl)methoxy)(dimethylamino)phosphoryl)-6-((2-cyanoethoxy)-2-(2-phenoxyacetamide)-9H-purin-9-yl)morpholin-2-yl)methylphosphonate;
((2S,6R)-6-(4-benzamido-2-oxopyrimidin-1(2H)-yl)-4-((((2S,6R)-6-(4-benzamido-2-oxopyrimidin-1(2H)-yl)-4-tritylmorpholin-2-yl)methoxy)(dimethylamino)phosphoryl)morpholin-2-yl)methylphosphonate;
triethylammonium ((2S,6R)-6-(4-benzamido-2-oxopyrimidin-1(2H)-yl)-4-((((2S,6R)-6-(4-benzamido-2-oxopyrimidin-1(2H)-yl)-4-tritylmorpholin-2-yl)methoxy)(dimethylamino)phosphoryl)morpholin-2-yl)methylphosphonate;
((2S,6R)-4-((dimethylamino)(((2S,6R)-6-(5-methyl-2,4-dioxo-3,4-dihydropyrimidin-1(2H)-yl)-4-tritylmorpholin-2-yl)methoxy)phosphoryl)-6-(5-methyl-2,4-dioxo-3,4-dihydropyrimidin-1(2H)-yl)morpholin-2-yl)methylphosphonate;
triethylammonium (2S,6R)-4-((dimethylamino)(((2S,6R)-6-(5-methyl-2,4-dioxo-3,4-dihydropyrimidin-1(2H)-yl)-4-tritylmorpholin-2-yl)methoxy)phosphoryl)-6-(5-methyl-2,4-dioxo-3,4-dihydropyrimidin-1(2H)-yl)morpholin-2-yl)methylphosphonate;
((2S,6R)-4-((dimethylamino)(((2S,6R)-4-((dimethylamino)(((2S,6R)-6-(5-methyl-2,4-dioxo-3,4-dihydropyrimidin-1(2H)-yl)-4-tritylmorpholin-2-yl)methoxy)phosphoryl)-6-(5-methyl-2,4-dioxo-3,4-dihydropyrimidin-1(2H)-yl)methoxy)phosphoryl)-6-(5-methyl-2,4-dioxo-3,4-dihydropyrimidin-1(2H)-yl)morpholin-2-yl)methylphosphonate;
triethylammonium ((2S,6R)-4-((dimethylamino)(((2S,6R)-4-((dimethylamino)(((2S,6R)-6-(5-methyl-2,4-dioxo-3,4-dihydropyrimidin-1(2H)-yl)-4-tritylmorpholin-2-yl)methoxy)phosphoryl)-6-(5-methyl-2,4-dioxo-3,4-dihydropyrimidin-1(2H)-yl)methoxy)phosphoryl)-6-(5-methyl-2,4-dioxo-3,4-dihydropyrimidin-1(2H)-yl)morpholin-2-yl)methylphosphonate;
((2S,6R)-4-((dimethylamino)(((2S,6R)-4-((dimethylamino)(((2S,6R)-4-((dimethylamino)((((2S,6R)-6-(5-methyl-2,4-dioxo-3,4-dihydropyrimidin-1(2H)-yl)-4-tritylmorpholin-2-yl)methoxy)phosphoryl-6-(5-methyl-2,4-dioxo-3,4-dihydropyrimidin-1(2H)-yl)methoxy)phosphoryl-6-(5-methyl-2,4-dioxo-3,4-dihydropyrimidin-1(2H)-yl)methoxy)phosphoryl)-6-(5-methyl-2,4-dioxo-3,4-dihydropyrimidin-1(2H)-yl)morpholin-2-yl)methylphosphonate;
triethylammonium ((2S,6R)-4-((dimethylamino)(((2S,6R)-4-((dimethylamino)(((2S,6R)-4-((dimethylamino)((((2S,6R)-6-(5-methyl-2,4-dioxo-3,4-dihydropyrimidin-1(2H)-yl)-4-tritylmorpholin-2-yl)methoxy)phosphoryl)-6-(5-methyl-2,4-dioxo-3,4-dihydropyrimidin-1(2H)-yl)methoxy)phosphoryl)-6-(5-methyl-2,4-dioxo-3,4-dihydropyrimidin-1(2H)-yl)methoxy)phosphoryl)-6-(5-methyl-2,4-dioxo-3,4-dihydropyrimidin-1(2H)-yl)morpholin-2-yl)methylphosphonate;
1,8-diazabicyclo(5.4.0)-7-undecenium ((2S,6R)-6-(4-benzamido-2-oxopyrimidin-1(2H)-yl)-4-((((2S,6R)-4-((((2S,6R)-6-(4-benzamido-2-oxopyrimidin-1(2H)-yl)-4-((dimethylamino)(((2S,6R)-6-(5-methyl-2,4-dioxo-3,4-dihydropyrimidin-1(2H)-yl)-4-tritylmorpholin-2-yl)methoxy)phosphoryl)morpholin-2-yl)(dimethylamino)phosphoryl)-6-(5-methyl-2,4-dioxo-3,4-dihydropyrimidin-1(2H)-yl)morpholin-2-yl)methoxy)phosphoryl)-6-(6-benzamido-9H-purin-9-yl) morpholin-2-yl)methoxy)(dimethylamino)phosphoryl)morpholin-2-yl)methylphosphonate; and
1,8-diazabicyclo(5.4.0)-7-undecenium ((2S,6R)-4-(((2S,6R)-4-(((25,6R)-6-(6-benzamidopurin-9-yl)-4-(((2S,6R)-6-(6-benzamidopurin-9-yl)-4-(((2S,6R)-4-(((2S,6R)-6-(6-(2-cyanoethoxy)-2-((2-phenoxyacetyl)amino)purin-9-yl)-4-(((2S,6R)-4-(((2S,6R)-6-(6-(2-cyanoethoxy)-2-((2-phenoxyacetyl)amino)purin-9-yl-4-(dimethylamino-(((2S,6R)-4-(dimethylamino-(((2S,6R)-6-(5-methyl-2,4-dioxo-pyrimidin-1-yl)-4-trityl-morpholin-2-yl)methoxy)phosphoryl)-6-(5-methyl-2,4-dioxo-pyrimidin-1-yl)morpholin-2-yl)methoxy)phosphoryl)morpholin-2-yl)methoxy-(dimethylamino)phosphoryl-6-(5-methyl-2,4-dioxo-pyrimidin-1-yl)morpholin-2-yl)methoxy-(dimethylamino)phosphoryl)morpholin-2-yl)methoxy-(dimethylamino)phosphoryl)-6-(6-(3-cyanopropoxy-2-((2-phenoxyacetyl)amino)purin-9-yl)morpholin-2-yl)methoxy-(dimethylamino)phosphoryl)morpholin-2-yl)methoxy-(dimethylamino)phosphoryl)morpholin-2-yl)methoxy-(dimethylamino)phosphoryl)-6-(6-(2-cyanoethoxy)-2-((2-phenoxyacetyl)amino)purin-9-yl)morpholin-2-yl)methoxy-(dimethylamino)phosphoryl)-6-(5-methyl-2,4-dioxo-pyrimidin-1-yl)morpholin-2-yl)methylphosphinate.

10. A compound of the formula [B-0-1]: 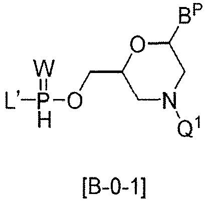 wherein
B^{P} is a nucleobase optionally protected;
Q¹ is trityl or dimethoxytrityl;
L' is O⁻(HDBU)⁺; and
W is O.

11. The compound of the formula [B-0-1] according to claim 10 selected from the group consisting of:
1,8-diazabicyclo(5.4.0)-7-undecenium ((2S,6R)-6-(6-benzamido-9H-purin-9-yl)-4-tritylmorpholin-2-yl)methylphosphonate;
1,8-diazabicyclo(5.4.0)-7-undecenium ((2S,6R)-6-(4-benzamido-2-oxopyrimidin-1(2H)-yl)-4-tritylmorpholin-2-yl)methylphosphonate;
1,8-diazabicyclo(5.4.0)-7-undecenium ((2S,6R)-6-(5-methyl-2,4-dioxo-3,4-dihydropyrimidin-1(2H)-yl)-4-tritylmorpholin-2-yl)methylphosphonate;
1,8-diazabicyclo(5.4.0)-7-undecenium ((2S,6R)-6-((2-cyanoethoxy)-2-(2-phenoxyacetamido)-9H-purin-9-yl)-4-tritylmorpholin-2-yl)methylphosphonate; and
1,8-diazabicyclo(5.4.0)-7-undecenium ((2S,6R)-6-(2-(2-phenylacetamide)-6-((4-(pivaloyloxy)benzyl)oxy)-9H-purin-9-yl)-4-tritylmorpholin-2-yl)methylphosphonate.
